# EUROPÄISCHE PATENTSCHRIFT

(11) **EP 0 527 378 B1**
(45) Veröffentlichungstag und Bekanntmachung des Hinweises auf die Patenterteilung: **28.02.1996**
(21) Anmeldenummer: 92112772.6
(22) Anmeldetag: 27.07.1992
(51) Int. Cl.: C07D 239/60, C07D 403/12, C07D 409/12, C07D 251/30, A01N 43/54

(54) **Salicyl(thio)etherderivate, Verfahren und Zwischenprodukte zu ihrer Herstellung**
Salicyl(thio)ethers derivatives, process and intermediates for their preparation
Dérivés de salicyl(thio)éthers, procédé et intermédiaires pour leur production

(30) Priorität: 10.08.1991 DE 4126937
(43) Veröffentlichungstag der Anmeldung: 17.02.1993
(73) Patentinhaber: BASF Aktiengesellschaft, D-67063 Ludwigshafen (DE)
(72) Erfinder: Vogelbacher, Uwe Josef, Dr., W-6700 Ludwigshafen (DE); Rheinheimer, Joachim Dr., W-6700 Ludwigshafen (DE); Saupe, Thomas, Dr., W-6902 Sandhausen (DE); Meyer, Norbert, Dr., W-6802 Ladenburg (DE); Gerber, Matthias, Dr., W-6704 Mutterstadt (DE); Westphalen, Karl-Otto, Dr., W-6720 Speyer (DE); Walter, Helmut, Dr., W-6719 Obrigheim (DE)

(56) Entgegenhaltungen:
- EP-A- 0 402 751

## Beschreibung

Die vorliegende Erfindung beschreibt Salicyl(thio)etherderivate der Formel I, 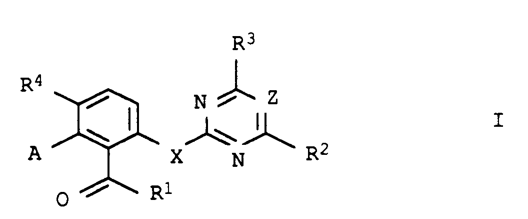
in der die Substituenten folgende Bedeutung haben:
- R¹: ein Rest 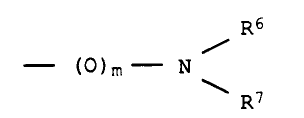 in dem m für 0 oder 1 steht und R⁶ und R⁷, die folgende Bedeutung haben:
Wasserstoff;
C₁-C₆-Alkyl, C₃-C₆-Alkenyl, C₃-C₆-Alkinyl, wobei diese Reste jeweils ein bis fünf Halogenatome und/oder ein bis zwei der folgenden Gruppen tragen können: C₁-C₆-Alkoxy, C₃-C₆-Alkenyloxy, C₃-C₆-Alkinyloxy, C₁-C₆-Alkylthio, C₃-C₆-Alkenylthio, C₃-C₆-Alkinylthio, C₁-C₆-Halogenalkoxy, Cyano, C₁-C₆-Alkylcarbonyl, C₃-C₆-Alkenylcarbonyl, C₃-C₆-Alkinylcarbonyl, C₁-C₆-Alkoxycarbonyl, C₃-C₆-Alkenyloxycarbonyl, C₃-C₆-Alkinyloxycarbonyl, -C₁-C₆-Dialkylamino, C₁-C₆-Cycloalkyl;
ggf. substituiertes C₁-C₆-Cycloalkyl;
ggf. substituiertes Phenyl;
R⁶ zusammen mit R⁷ eine ggf. substituierte C₄-C₇-Alkylenkette, in der eine CH₂-Gruppe durch Sauerstoff, Schwefel oder -NH ersetzt sein kann;
eine Gruppe 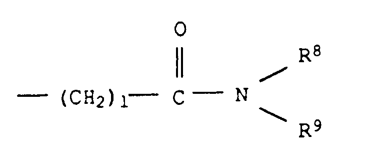 in der R⁸ und R⁹ für Wasserstoff, C₁-C₆-Alkyl, C₃-C₆-Alkenyl oder C₃-C₆-Alkinyl stehen und l die Werte 1, 2, 3 oder 4 annimmt;
oder eine Gruppe

― (CH₂)ₗ ― S(=O)ₖ-R¹⁰

in der R¹⁰ für C₁-C₆-Alkyl, C₃-C₆-Alkenyl oder C₃-C₆-Alkinyl steht, l für 1, 2, 3 oder 4 und k für 0, 1 oder 2 stehen;
ein Rest OR⁵, worin
- R⁵: ein über ein Stickstoffatom gebundener ggf. substituierter 5-gliedriger aromatischer Heterocyclus mit ein bis vier Stickstoffatomen im Ring
oder einen Rest 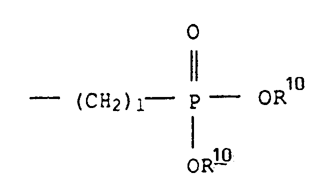 bedeutet, in dem R¹⁰ und l die oben genannte Bedeutung haben,
oder ein Rest

-HN-SO₂-R¹²

in dem R¹² für die Reste C₁-C₆-Alkyl, C₁-C₆-Halogenalkyl oder Phenyl steht, die ihrerseits ein bis vier der folgenden Substituenten tragen können: Halogen, Nitro, Cyano, C₁-C₆-Alkyl;
- R²,R³: C₁-C₄-Alkyl, C₁-C₄-Halogenalkyl, C₁-C₄-Alkoxy, C₁-C₄-Halogenalkoxy und/oder C₁-C₄-Alkylthio;
- X: ein Sauerstoff- oder Schwefelatom;
- Z: Stickstoff oder die Methingruppe;
- R⁴: Wasserstoff, Halogen, Nitro, C₁-C₄-Alkyl, Cyano oder C₁-C₄-Halogenalkyl;
- A: ein 5-gliedriger Heteroaromat mit ein bis vier Stickstoffatomen oder ein bis drei Stickstoffatomen sowie zusätzlich einem Schwefel- oder Sauerstoffatom im Ring, welcher ein bis drei Halogenatome und/oder einen bis drei der folgenden Reste tragen kann: Nitro, Cyano, C₁-C₄-Alkyl, C₁-C₄-Alkylthio, C₁-C₄-Halogenalkyl oder Phenyl, das unsubstituiert oder durch ein bis drei Halogenatome und/oder ein bis drei Methylgruppen substituiert ist;
ein benzokondensierter 5-gliedriger Heteroaromat, der ein bis drei Stickstoffatome oder ein Stickstoffatom und zusätzlich ein Sauerstoff- oder Schwefelatom enthalten und einen der folgenden Reste tragen kann: C₁-C₄-Alkyl, Halogen, Cyano;
ein Thienyl- oder Furylrest, der ein bis drei Halogenatome und/oder einen bis drei der folgenden Reste tragen kann: C₁-C₄-Alkyl, C₁-C₂-Halogenalkyl, ggf. substituiertes Phenyl oder Nitro;
ein Benzthienyl- oder Benzofurylrest, der ein Halogenatom und/oder einen der folgenden Reste tragen kann: C₁-C₄-Alkyl, C₁-C₂-Halogenalkyl oder Nitro;
ein Pyridylrest, der ein bis drei Halogenatome und/oder einen bis drei der folgenden Reste tragen kann: C₁-C₄-Alkyl, C₁-C₂-Halogenalkyl, ggf. substituiertes Phenyl oder Nitro;
einen Naphthyl- oder Chinolinylrest welcher jeweils bis zu drei Halogenatome und/oder bis zu drei der folgenden Reste tragen kann: C₁-C₄-Alkyl oder C₁-C₂-Halogenalkyl,
wobei der Ausdruck ggf. substituiert in den oben genannten Fällen jeweils bedeutet, daß die entsprechenden Gruppen einen oder mehrere der folgenden Substituenten tragen können: Halogen, Nitro, Cyano, C₁-C₆-Alkyl, C₁-C₆-Halogenalkyl, C₁-C₆-Alkoxy, C₁-C₆-Alkylthio.

In der Literatur (EP-A 223 406, EP-A 249 708, EP-A 287 072, EP-A 426 476, EP-A 287 079, EP-A 346 789 und EP-A 402 751)) sind herbizid wirksame, substituierte Salicylsäuren und deren Schwefelanaloge beschrieben. Ihre Wirkung ist jedoch nicht immer befriedigend.

Aufgabe der vorliegenden Erfindung waren daher neue Salicylsäurederivate bzw. deren Schwefelanaloge mit verbesserten herbiziden Eigenschaften oder weiteren im Pflanzenschutz erwünschten Eigenschaften wie wachstumsregulierender, fungizider oder nitrifikationshemmender Wirkung.

Entsprechend dieser Aufgabe wurden die eingangs definierten Verbindungen der Formel I gefunden. Außerdem wurden Verfahren zur Herstellung der Verbindungen I und Verfahren zur Bekämpfung unerwünschten Pflanzenwuchses mit den Verbindungen I gefunden. Es wurde außerdem gefunden, daß Salicylsäurederivate der vorstehend definierten allgemeinen Formel I ausgezeichnete pflanzenwachstumsregulierende Eigenschaften besitzen und darüber hinaus fungizid wirksam sind und nitrifikationshemmend wirken. Als Zwischenprodukte zur Herstellung der Verbindungen I wurden die neuen Salicylsäurederivate II' gefunden.

Verbindungen der Formel I erhält man beispielsweise, indem man ein entsprechend substituiertes Salicylsäurederivat der Formel II, das in Einzelfällen bekannt ist oder nach üblichen Methoden, ausgehend von bekannten Vorprodukten, hergestellt werden kann, mit einer entsprechenden Verbindung der Formel III in Gegenwart einer Base umsetzt. 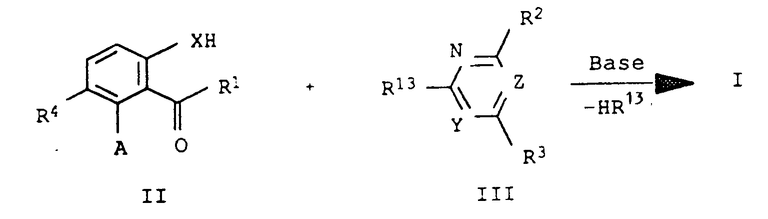
R¹³ in Formel III bedeutet eine übliche nucleofuge Abgangsgruppe, beispielsweise Halogen wie Chlor, Brom, Iod, Aryl- oder Alkylsulfonyl wie z.B. Toluolsulfonyl und Methylsulfonyl oder eine andere äquivalente Abgangsgruppe. Verbindungen der Formel III mit einem reaktionsfähigen Substituenten R¹³ sind bekannt oder mit dem allgemeinen Fachwissen leicht zu erhalten. Als Base können Alkali- oder Erdalkalimetallhydride wie NaH oder CaH₂, Alkalimetallhydroxyde wie NaOH oder KOH, Alkalimetallalkoholate wie Kalium-tert.-butylat, Alkalimetallcarbonate wie Na₂CO₃ oder K₂CO₃, Alkalimetallamide wie NaNH₂ oder Lithiumdiisopropylamid oder tertiäre Amine Verwendung finden. Bei Einsatz einer anorganischen Base kann man einen Phasentransferkatalysator zusetzen, wenn dies den Umsatz fördert.

Verbindungen der Formel I können auch dadurch hergestellt werden, daß man von den entsprechenden Carbonsäuren, d.h. Verbindungen der Formel I, in denen R¹ Hydroxyl bedeutet, ausgeht und diese zunächst auf übliche Weise in eine aktivierte Form wie ein Halogenid oder Imidazolid überführt und dieses dann mit der entsprechenden Hydroxylverbindung umsetzt. Diese beiden Schritte lassen sich beispielsweise auch dadurch vereinfachen, daß man die Carbonsäure in Gegenwart eines wasserabspaltenden Mittels wie eines Carbodiimids auf die Hydroxylverbindung einwirken läßt.

Die Zwischenprodukte der Formel II lassen sich, wenn X für ein Sauerstoffatom und A für einen über ein Kohlenstoffatom gebundenen Heteroaromaten steht, gemäß dem untenstehenden Schema aus einer 1,3-Dicarbonylverbindung IV (mit R⁵ = ggf. phenylsubstituiertes C₁-C₁₀-Alkyl, insbesondere C₁-C₄-Alkyl) und einem α, β-ungesättigten Keton V aufbauen: 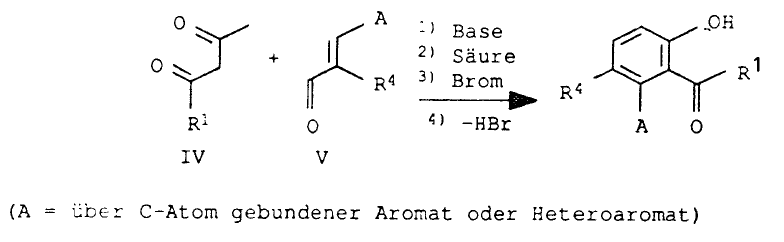
Dabei sind die Verbindungen IV und V im allgemeinen bekannt oder nach den üblichen Verfahren leicht herstellbar. Als Base kommen die obengenannten Verbindungen in Frage. Als Säure eignen sich starke Säuren, beispielsweise Chlorwasserstoff, Bromwasserstoff, Tetrafluoroborsäure, Toluolsulfonsäure oder Trifluoressigsäure. Die Bromwasserstoffabspaltung kann thermisch oder in Gegenwart einer Base, beispielsweise eines organischen Amins, durchgeführt werden.

Steht A in Formel II für einen über ein Stickstoffatom gebundenen Heteroaromaten und X für ein Sauerstoffatom, so kann man dieses Zwischenprodukt gemäß dem folgenden Schema aufbauen: 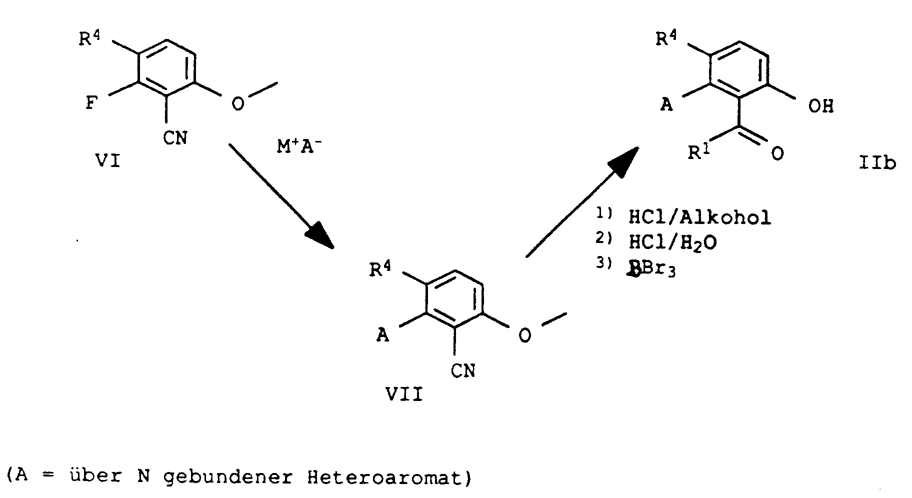
Dabei steht M⁺A⁻ für das jeweilige Alkalimetallazolid. Als Alkohole kommen bei der Spaltung des Nitrils VII insbesondere C₁-C₄-Alkylalkohole in Frage.

Die wie oben beschrieben hergestellten Zwischenprodukte der Formel II fallen üblicherweise als Alkylester an. Diese lassen sich nach den bekannten Verfahren zu den Carbonsäuren hydrolysieren. Man kann letztere nun nach literaturbekannten Verfahren in verschiedene Ester überführen, die man zur Darstellung von Wirkstoffen der Formel I gemäß Anspruch 1 benötigt.

Im Hinblick auf die herbizide und pflanzenwachstumsregulierende Wirksamkeit sind Verbindungen I bevorzugt, in denen die Substituenten folgende Bedeutung haben:
- R¹: ein Rest 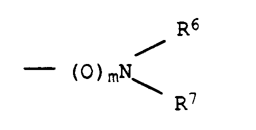 worin m die Werte 0 oder 1 annimmt und R⁶ und R⁷
Wasserstoff;
Alkyl wie insbesondere Methyl, Ethyl, Propyl, 1-Methylethyl, Butyl, 1-Methylpropyl, 2-Methylpropyl, 1,1-Dimethylethyl, n-Pentyl, 1-Methylbutyl, 2-Methylbutyl, 3-Methylbutyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, n-Heptyl, 1-Methylhexyl, 2-Methylhexyl, 3-Methylhexyl, 4-Methylhexyl, 5-Methylhexyl, 1-Ethylpentyl, 2-Ethylpentyl,1-Propylbutyl und Octyl;
Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl,1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dim-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 2-Ethyl-2-butenyl, 1-Ethyl-3-butenyl, 2-Ethyl-3-butenyl, 1,1,2-Trimethyl-2-propenyl, 1-Ethyl-1-methyl-2-propenyl, 1-Ethyl-2-methyl-2-propenyl, insbesondere 2-Propenyl, 2-Butenyl, 3-Methyl-2-butenyl und 3-Methyl-2-pentenyl;
Alkinyl wie 2-Propinyl, 2-Butinyl, 3-Butinyl-, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl2-propinyl, 1-Ethyl-2-propinyl, 2-Hexinyl, 3-Hexinyl, 4-Hexinyl, 5-Hexinyl, 1-Methyl2-pentinyl, 4-Methyl-2-pentinyl, 1-Methyl-3-pentinyl, 2-Methyl-3-pentinyl, 1-Methyl4-pentinyl, 2-Methyl-4-pentinyl, 3-Methyl-4-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl, 1-Ethyl-1-methyl-2-propinyl, vorzugsweise 2-Propinyl, 2-Butinyl, 1-Methyl-2-propinyl und 1-Methyl-2-butinyl, insbesondere 2-Propinyl und 1-Methyl-2-propinyl.
Die vorstehend genannten Alkyl-, Alkenyl- und Alkinylgruppen können jeweils ein bis fünf Halogenatome, vorzugsweise Fluor und Chlor, und/oder ein bis zwei der folgenden Reste tragen:
Cyano;
Alkoxy mit ein bis vier Kohlenstoffatomen, insbesondere Alkoxy, wie z.B. Methoxy, Ethoxy, Propoxy,1-Methylethoxy, Butoxy, 1-Methylpropoxy, 2-Methylpropoxy, 1,1-Dimethylethoxy, vorzugsweise Methoxy, Ethoxy und 1-Methylethoxy;
Alkenyloxy mit ein bis vier Kohlenstoffatomen, insbesondere Alkenyloxy, wie z.B. Ethenyloxy, Propenyloxy, 1-Methylethenyloxy, Butenyloxy, 1-Methylpropenyloxy, 2-Methylpropenyloxy, 1,1-Dimethylethenyloxy, vorzugsweise Ethenyloxy und1-Methylethenyloxy;
Alkinyloxy mit ein bis vier Kohlenstoffatomen, insbesondere Alkinyloxy, wie z.B. Ethinyloxy, Propinyloxy, 1-Methylethinyloxy, Butinyloxy, 1-Methylpropinyloxy, 2-Methylpropinyloxy, 1,1-Dimethylethinyloxy, vorzugsweise Ethinyloxy und 1-Methylethinyloxy;
Alkylthio mit ein bis vier Kohlenstoffatomen, insbesondere Alkylthio, wie z.B. Methylthio, Ethylthio, Propylthio, 1-Methylethylthio, Butylthio, 1-Methylpropylthio, 2-Methylpropylthio, 1,1-Dimethylethylthio, vorzugsweise Methylthio, Ethylthio und 1-Methylethylthio;
Alkenylthio mit ein bis vier Kohlenstoffatomen, insbesondere Alkenylthio, wie z.B. Ethenylthio, Propenylthio, 1-Methylethenylthio, Butenylthio, 1-Methylpropenylthio, 2-Methylpropenylthio, 1,1-Dimethylethenylthio, vorzugsweise Ethenylthio und 1-Methylethenylthio;
Alkinylthio mit ein bis vier Kohlenstoffatomen, insbesondere Alkinylthio, wie z.B. Ethinylthio, Propinylthio, 1-Methylethinylthio, Butinylthio, 1-Methylpropinylthio, 2-Methylpropinylthio, 1,1-Dimethylethinylthio, vorzugsweise Ethinylthio und 1-Methylethinylthio;
Halogenalkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy, Pentafluorethoxy, insbesondere Difluormethoxy und Trifluormethoxy;
Alkylcarbonyl wie insbesondere Methylcarbonyl, Ethylcarbonyl, Propylcarbonyl, 1-Methylethylcarbonyl, Butylcarbonyl, 1-Methylpropylcarbonyl, 1,1-Dimethylethylcarbonyl, Pentylcarbonyl, 2-Methylbutylcarbonyl, 1,1-Dimethylpropylcarbonyl, 1,2-Dimethylpropylcarbonyl,2,2-Dimethylpropylcarbonyl, 1-Ethylpropylcarbonyl, Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropylcarbonyl;
Alkoxycarbonyl wie Methoxycarbonyl, Ethoxycarbonyl, Propyloxycarbonyl, 1-Methylethoxycarbonyl, Butyloxycarbonyl, 1-Methylpropyloxycarbonyl, 2-Methyl propyloxycarbonyl, 1,1-Dimethylpropyloxycarbonyl, n-Pentyloxycarbonyl, 1-Methylbutyloxycarbonyl, 2-Methylbutyloxycarbonyl, 3-Methylbutyloxycarbonyl, 1,2-Dimethylpropyloxycarbonyl, 1,1-Dimethylpropyloxycarbonyl, 2,2-Dimethylpropyl oxycarbonyl, 1-Ethylpropyloxycarbonyl, n-Hexyloxycarbonyl, 1-Methylpentenyloxycarbonyl, 2-Methylpentenyloxycarbonyl, 3-Methylpentenyloxycarbonyl, 4-Methylpentenyloxycarbonyl, 1,2-Dimethylbutyloxycarbonyl, 1,3-Dimethylbutyloxycarbonyl, 2,3-Dimethylbutyloxycarbonyl, 1,1-Dimethylbutyloxycarbonyl, 2,2-Dimethylbutyloxycarbonyl, 3,3-Dimethylbutyloxycarbonyl, 1,1,2-Trimethylpropyloxycarbonyl, 1,2,2-Trimethylpropyloxycarbonyl, 1-Ethylbutyloxycarbonyl, 2-Ethylbutyloxycarbonyl, 1-Ethyl-2-methylpropyloxycarbonyl, n-Heptyloxycarbonyl, 1-Methylhexyloxycarbonyl, 2-Methylhexyloxycarbonyl, 3-Methylhexyloxycarbonyl, 4-Methylhexyloxycarbonyl, 5-Methylhexyloxycarbonyl, 1-Ethylpentenyloxycarbonyl, 1-Propylbutyloxycarbonyl, Octyloxycarbonyl, insbesondere Methoxycarbonyl, Ethoxycarbonyl, 1-Methylethoxycarbonyl, 1-Methylpropyloxycarbonyl;
Cycloalkyl wie Cyclopropyl, Cyclobutyl, Cyclopentyl, Cyclohexyl, Cycloheptyl;
Dialkylamino wie Dimethylamino, Diethylamino, Dipropylamino, Di-1-methylethyl, Dibutylamino, Di-1-methylpropylamino, Di-2-methylpropylamino, Di-1,1-methylethylamino, Ethylmethylamino, Propylmethylamino, 1-Methylethylmethylamino, oder Butylmethylamino;
Phenyl, Phenoxy, oder Phenylcarbonyl, wobei diese aromatischen Reste ihrerseits ein bis fünf Halogenatome wie vorstehend genannt, insbesondere Fluor, Chlor und Brom und/oder ein bis drei der folgenden Reste tragen können: Alkyl, Halogenalkyl, Alkoxy, und/oder Alkylthio mit jeweils ein bis vier Kohlenstoffatomen wie im allgemeinen und im besonderen vorstehend genannt;
C₃-C₁₂-Cycloalkyl, insbesondere C₃-C₆-Cycloalkyl wie Cyclopropyl, Cyclobutyl, Cyclopentyl, Cyclohexyl, das ggf. durch ein bis drei C₁-C₄-Alkylreste substituiert ist;
Phenyl oder Phenyl ein- bis dreifach substituiert durch C₁-C₄-Alkyl oder -Alkoxy, wie Methyl, Ethyl, Propyl, Butyl, Methoxy, Ethoxy, oder Phenyl substituiert durch ein bis fünf Halogenatome, z.B. Chlor oder Fluor;
R⁶ und R⁷ gemeinsam eine zu einem Ring geschlossene Alkylengruppe oder gemeinsam eine zu einem Ring geschlossene Alkylengruppe mit einem Heteroatom bilden, wobei der Ring bevorzugt fünf bis sieben Glieder aufweist und das Heteroatom Sauerstoff, Stickstoff oder Schwefel ist, und der so gebildete Ring einen oder mehrere der unter "ggf. substituiert" genannten Reste trägt.

Für die Gruppierung -NR⁶R⁷ seien besonders folgende Reste genannt: Dimethylamino, tert.-Butylamino, Cyclohexylamino, 1-Cyano-1-cyclohexylamino, Isopropylamino, sek.-Butylamino, Methylamino, Diethylamino, 1-Pyrrolidinyl, 1-Piperidyl, 4-Morpholinyl, Phenylamino, Methyl-phenylamino, Methoxymethylamino, Bis-(methoxymethyl)-amino,Methylthiomethylamino;
ein Rest 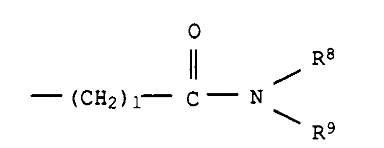
in dem l für eine Zahl von 1 bis 4, bevorzugt 1 und 2 steht und R⁸ und R⁹ Alkyl, Alkenyl und Alkinyl wie oben genannt darstellen;
ein Rest

-(CH₂)ₗ-S(=O)ₖ-R¹⁰

in dem l für eine Zahl von 1 bis 4, bevorzugt 1 und 2 steht und R¹⁰ Alkyl, Alkenyl und Alkinyl wie oben genannt darstellt;
ein Rest OR⁵, worin
- R⁵: 5-gliedriges Hetaryl mit ein bis vier Stickstoffatomen im Ring, das über ein Stickstoffatom gebunden ist und ein bis vier der unter "ggf. substituiert" genannten Reste tragen kann. Insbesondere seien genannt: 1-Pyrazolyl, 3-Methyl-1-pyrazolyl, 4-Methyl-1-pyrazolyl, 3,5-Dimethyl-1-pyrazolyl, 3-Phenyl-1-pyrazolyl, 4-Phenyl-1-pyrazolyl, 4-Chlor-1-pyrazolyl, 4-Brom-1-pyrazolyl, 4-Jod-1-pyrazolyl, 1-Imidazolyl, 1-Benzimidazolyl, 1,2,4-Triazol-1-yl, 3-Methyl-1,2,4-triazol-1-yl, 5-Methyl-1,2,4-triazol-1-yl, 1-Benztriazolyl
oder eine Gruppe 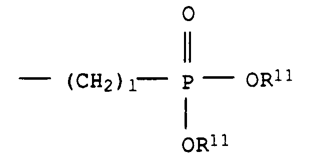 darstellt, in der l für eine Zahl von 1 bis 4, bevorzugt 1 und 2 steht und R¹¹ Alkyl, Alkenyl und Alkinyl wie für R⁶ und R⁷ genannt darstellt,
ein Rest

-HN-SO₂-R¹²,

in dem R¹² folgende Bedeutung hat:
C₁-C₆-Alkyl, das ein bis vier der folgenden Substituenten tragen kann: Halogen, Nitro, Cyano, C₁-C₆-Alkyl, insbesondere seien genannt: Methyl, Cyanomethyl, Ethyl, 2-Nitroethyl, Propyl, 1-Methylethyl, Butyl, 1-Methylpropyl, 2-Methylpropyl, 1,1-Dimethylethyl, n-Pentyl, 1-Methylbutyl, 2-Methylbutyl, 3-Methylbutyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 1-Ethyl-2-methylpropyl;
Phenyl, das ein bis vier der folgenden Substituenten tragen kann: Halogen, Nitro, Cyano, C₁-C₆-Alkyl; insbesondere seien genannt: 2-Fluorphenyl, 3-Fluorphenyl, 4-Fluorphenyl, 2,6-Difluorphenyl, 2,4-Difluorphenyl, 2,3-Difluorphenyl, 2-Fluor-4-trifluormethylphenyl, 2-Chlorphenyl, 3-Chlorphenyl, 2-Bromphenyl, 2-Iodphenyl, 2-Chlor-6-fluorphenyl, 2,4-Dichlorphenyl, 2,6-Dichlorphenyl, 3,5-Dichlorphenyl, 2-Chlor-4-fluorphenyl, 2-Chlor-6-methylphenyl, 2,3,5-Trichlorphenyl, 2-Methylphenyl, 3-Methylphenyl, 4-Methylphenyl, 2,6-Dimethylphenyl, 2,4-Dimethylphenyl, 3,5-Dimethylphenyl, 2,4,6-Trimethylphenyl, 2-Chlor-4-methylphenyl, 2-Trifluormethylphenyl, 3-Trifluormethylphenyl, 2-Cyanophenyl, 3-Cyanophenyl, 4-Cyanophenyl, 2-Nitrophenyl, 3-Nitrophenyl, 4-Nitrophenyl;
- R2, R³: im allgemeinen und im besonderen die bei R⁶ und R⁷ genannten Alkylgruppen, Halogenalkylgruppen, Alkoxygruppen, Halogenalkoxygruppen und/oder Alkylthiogruppen mit jeweils ein bis vier Kohlenstoffatomen, insbesondere Methyl, Trifluormethyl, Methoxy, Difluormethoxy, Methylthio;
- R⁴: Wasserstoff; Halogen, insbesondere Fluor und Chlor; Cyano; Alkyl mit ein bis vier Kohlenstoffatomen, insbesondere ein bis drei Kohlenstoffatomen, welches ein- bis fünffach durch Halogen, insbesondere Fluor und Chlor substituiert ist. Beispielsweise seien Methyl, Ethyl, n-Propyl, iso-Propyl, Trichlormethyl, Trifluormethyl genannt.
- A: ggf. substituiertes 5-gliedriges Hetaryl mit ein bis vier Stickstoffatomen wie Pyrrolyl, Pyrazolyl, Imidazolyl, 1,2,3-Triazolyl, 1,2,4-Triazolyl oder Tetrazolyl oder ein bis zwei Stickstoffatomen und zusätzlich einem Schwefel- oder Sauerstoffatom wie Isoxazolyl, Oxazolyl, Thiazolyl, Thiadiazolyl.
Beispielsweise seien folgende Hetarylreste genannt:
Pyrrol-1-yl, 2-Methyl-pyrrol-1-yl, 3-Methyl-pyrrol-1-yl, 1-Methyl-pyrrol-2-yl, 1-Methyl-pyrrol-3-yl, Pyrazol-1-yl, 4-Methylpyrazol-1-yl, 3,5-Dimethylpyrazol-1-yl, 3,4,5-Trimethylpyrazol-1-yl, 4-Chlorpyrazol-1-yl, 4-Phenylpyrazol-1-yl, 4-Nitropyrazol-1-yl, Imidazol-1yl, 4,5-Dimethylimidazol-1-yl, 2-Methyl-4,5-dichlorimidazol-1-yl, 4-Nitroimidazol-1-yl, 5-Nitroimidazol-1-yl, 1,2,4-Triazol-1-yl, 3(5)-Methyl-1,2,4-triazol-1-yl, 1,2,3-Triazol-1-yl, 4,5-Dimethyl-1,2,3-triazol-1-yl, 1,2,3,4-Tetrazol-1-yl, 1-Methylpyrazol-4-yl, 1-Phenylpyrazol-4-yl, 1,3,5-Trimethylpyrazol-4-yl, 1-Methylpyrazol-5-yl, 1-Phenylpyrazol-5-yl, 1-Methylpyrazol-3-yl, 1-Phenylpyrazol-3-yl, 1-Methylimidazol-2-yl, 1-Methylimidazol-5-yl, 1-Phenylimidazol-5-yl, 1-Phenyl-1,2,3-triazol-4-yl, Isoxyzol-5-yl, Isoxazol-4-yl, 3-Methylisoxazol5-yl, 3-Isopropylisoxazol-5-yl, 3-Phenylisoxazol-5-yl, Oxazol-2-yl, 2-Methyloxazol4-yl, Thiazol-4-yl, 4-Methylthiazol-2-yl, 4-Methylthiazol-5-yl, 2-Phenylthiazol-5-yl, 4-Phenylthiazol-2-yl.
Die genannten Reste können wie unten genannt, substituiert sein oder einen Phenylrest tragen, der durch Halogen oder ein bis drei Methylreste substituiert sein kann.
Ggf. substituiertes benzoanneliertes Hetaryl mit ein bis drei Stickstoffatomen oder einem Stickstoffatom und einem Sauerstoff- oder Schwefelatom im Ring wie z.B. Indolyl, Indazolyl, Benztriazolyl, Benzoxazolyl, Benzisoxazolyl, Benzthiazolyl; ggf. substituierte Thienylreste oder Benzthienylreste sind z.B.: 2-Thienyl, 3-Thienyl, 2-Chlor-5-thienyl, 4-Brom-2-thienyl, 2-Nitro-5-thienyl, 2-Benzthienyl, 3-Benzthienyl.
ggf. substituierte Naphthyl- oder Chinolinylreste sind z.B. 1-Naphthyl, 2-Naphthyl, 2-Chlor-1-naphthyl, 6-Chlor-1-naphthyl, 2,6-Dichlor-1-naphthyl, 2-Methyl-6-chlor-1-naphthyl, 2-Chinolinyl,4-Chinolinyl, 6-Chinolinyl, 8-Chinolinyl, 6-Methyl-2-chinolinyl.

Wird ein Rest als "ggf. substituiert" bezeichnet, so kann er einen oder mehrere insbesondere 1 bis 3 der folgenden Substituenten tragen: Halogen, insbesondere Chlor und Brom, Nitro, Cyano, sowie Alkyl, Halogenalkyl, Alkoxy und Alkylthio wie für R⁶ und R⁷ genannt.

Insbesondere bevorzugt sind Verbindungen der Formel I, in denen
- R², R³: Methoxy, Methyl, Difluormethoxy oder Chlor;
- R⁴: Wasserstoff oder Methyl;
- R⁶, R⁷: Wasserstoff, Methyl oder eine C₄- bzw. C₅-Alkylenkette
bedeuten und in denen X Sauerstoff, Y Stickstoff und Z eine Methingruppe darstellen und A die in Anspruch genannte Bedeutung hat.

Die in den nachfolgenden Tabellen wiedergegebenen Strukturen beschreiben besonders bevorzugte Wirkstoffe der Formel I.

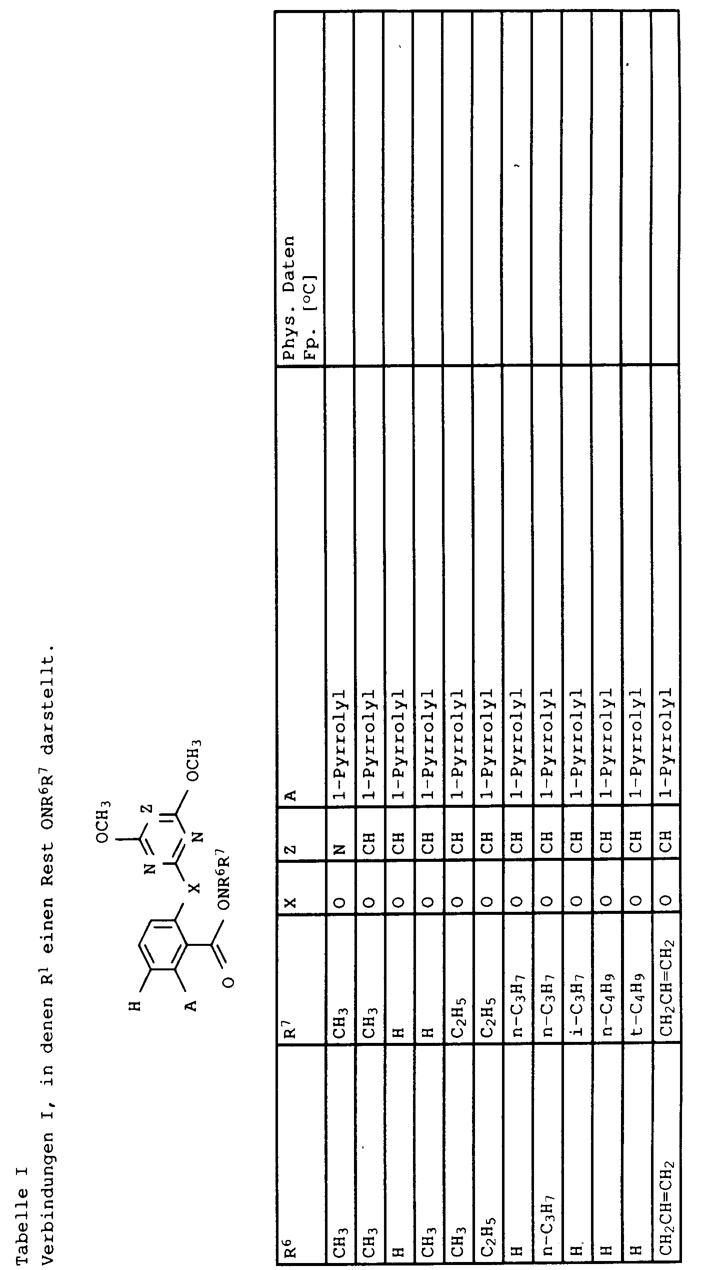

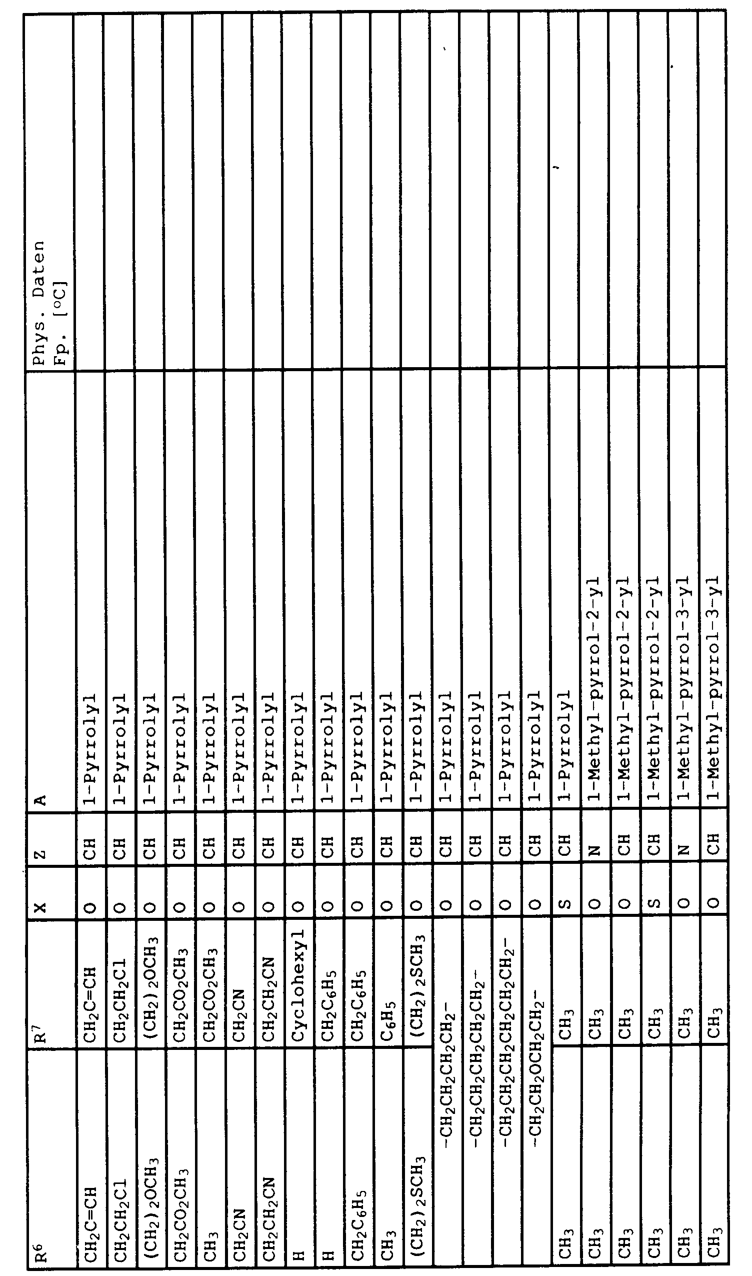

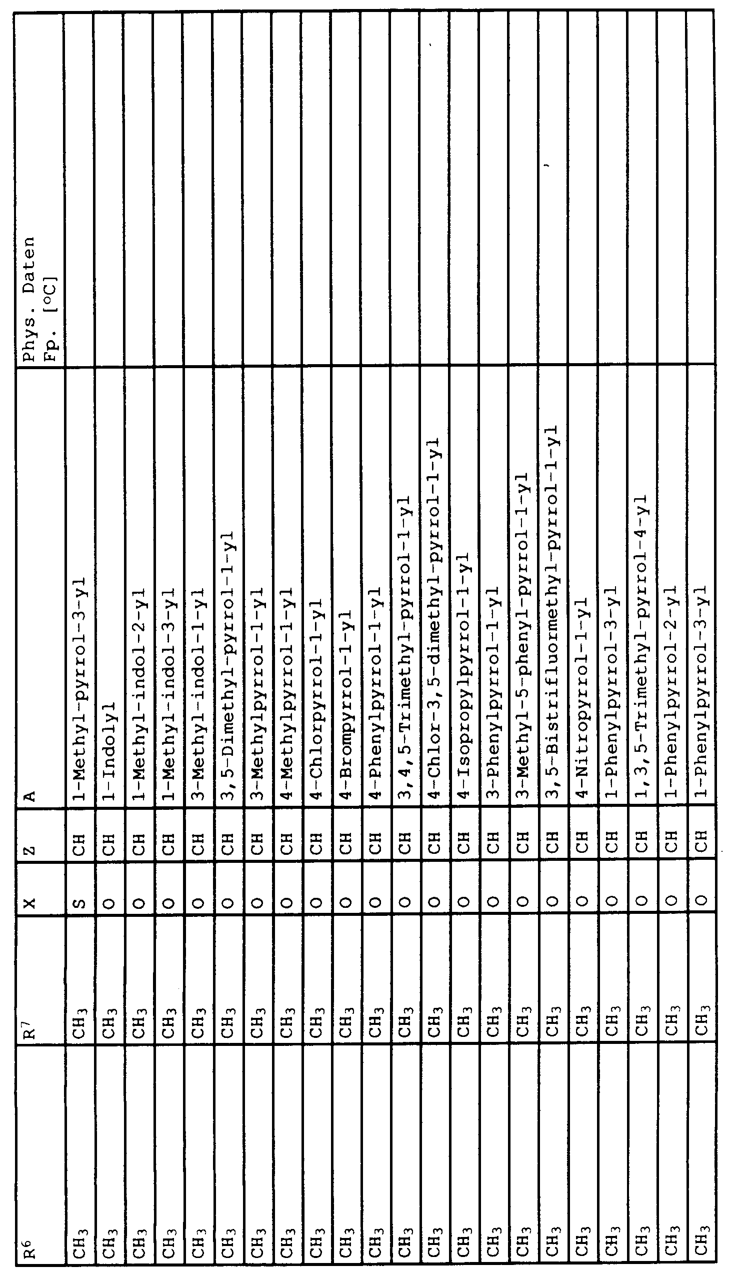

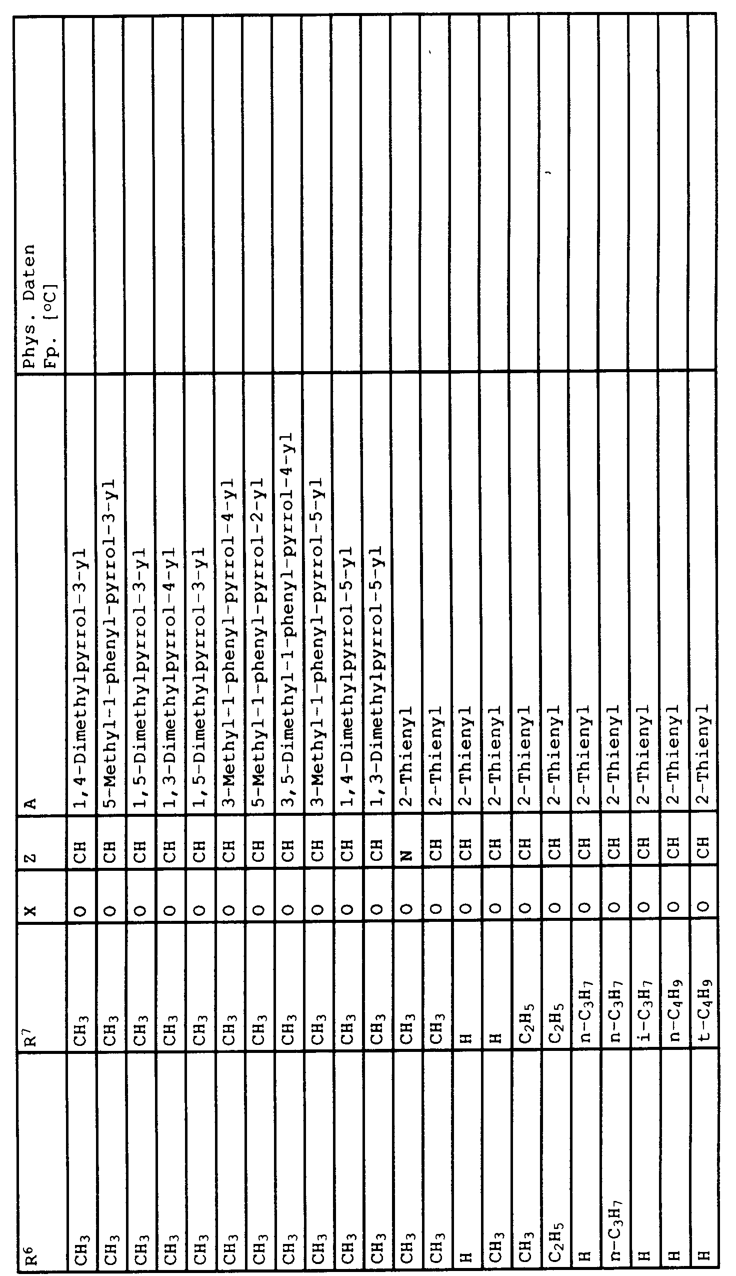

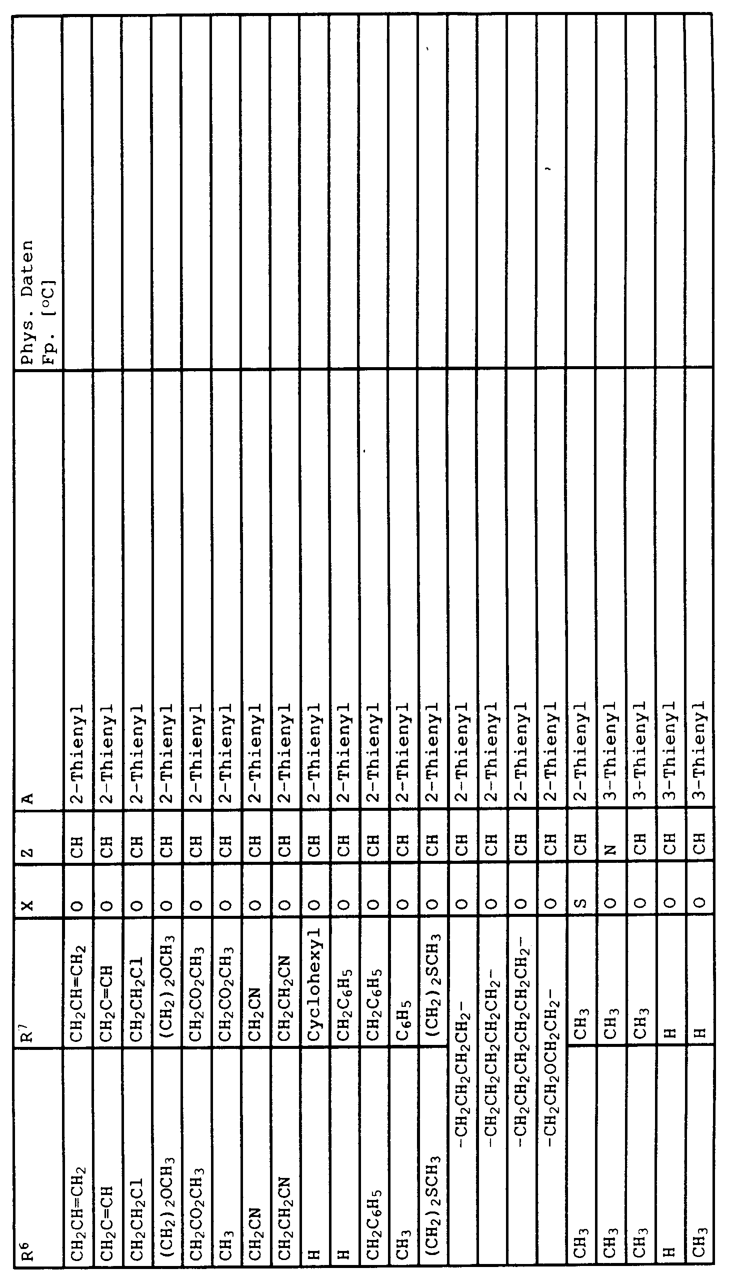

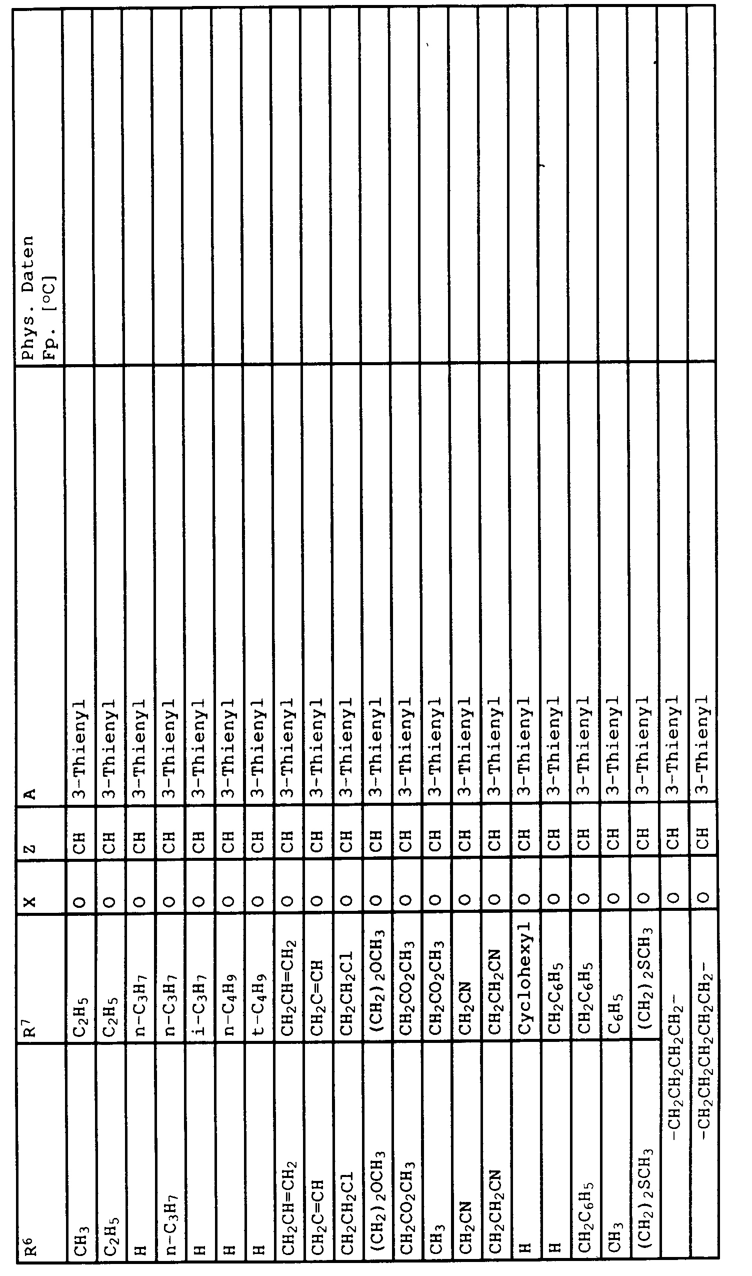

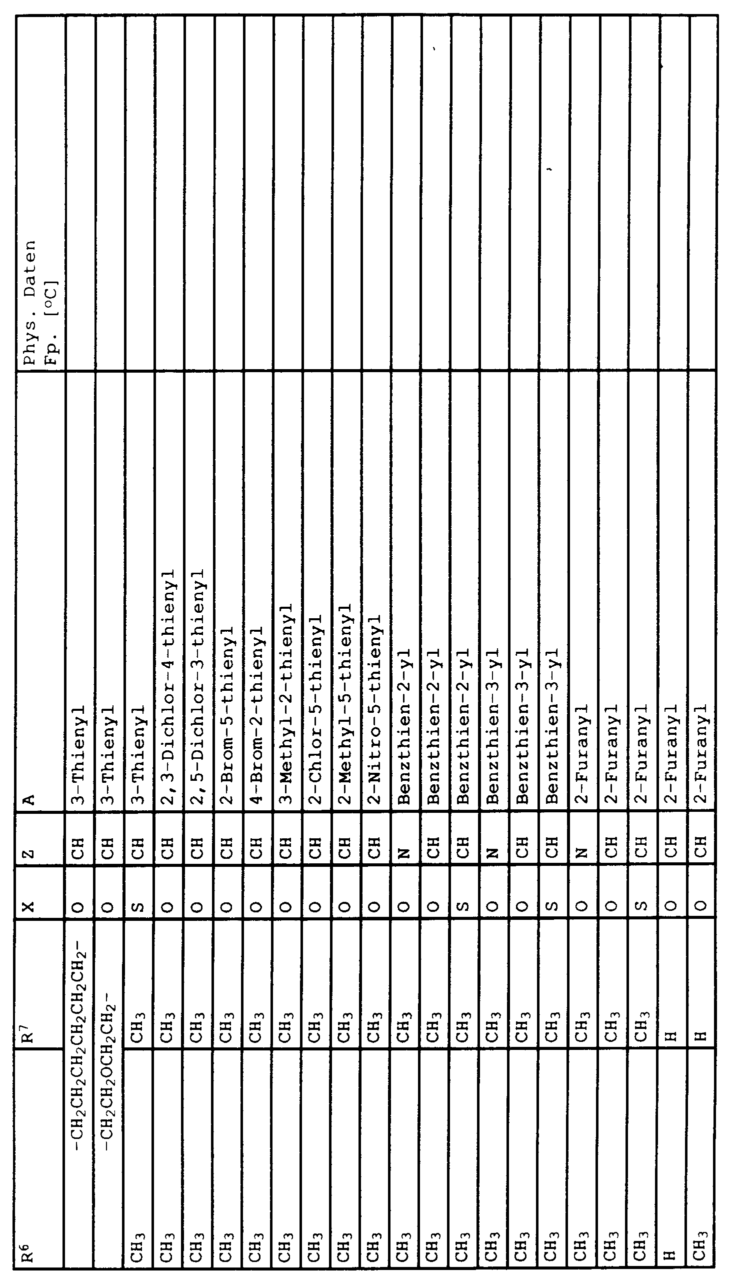

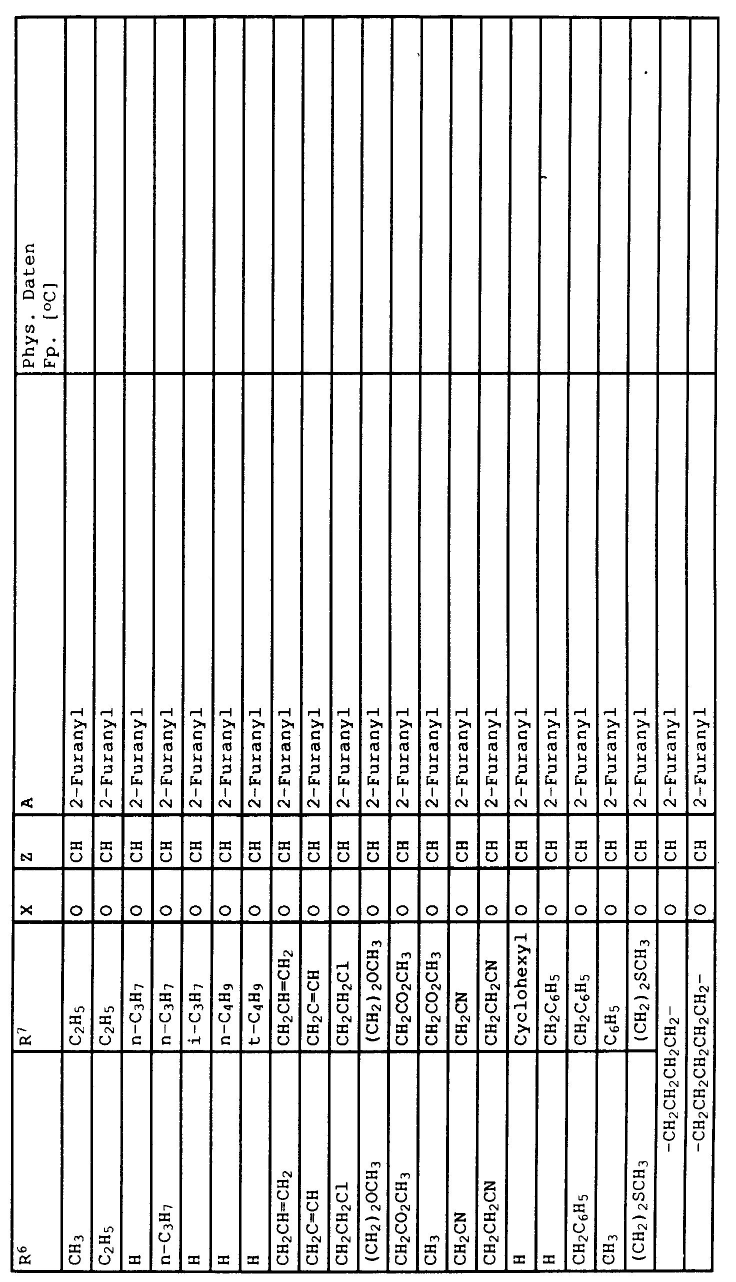

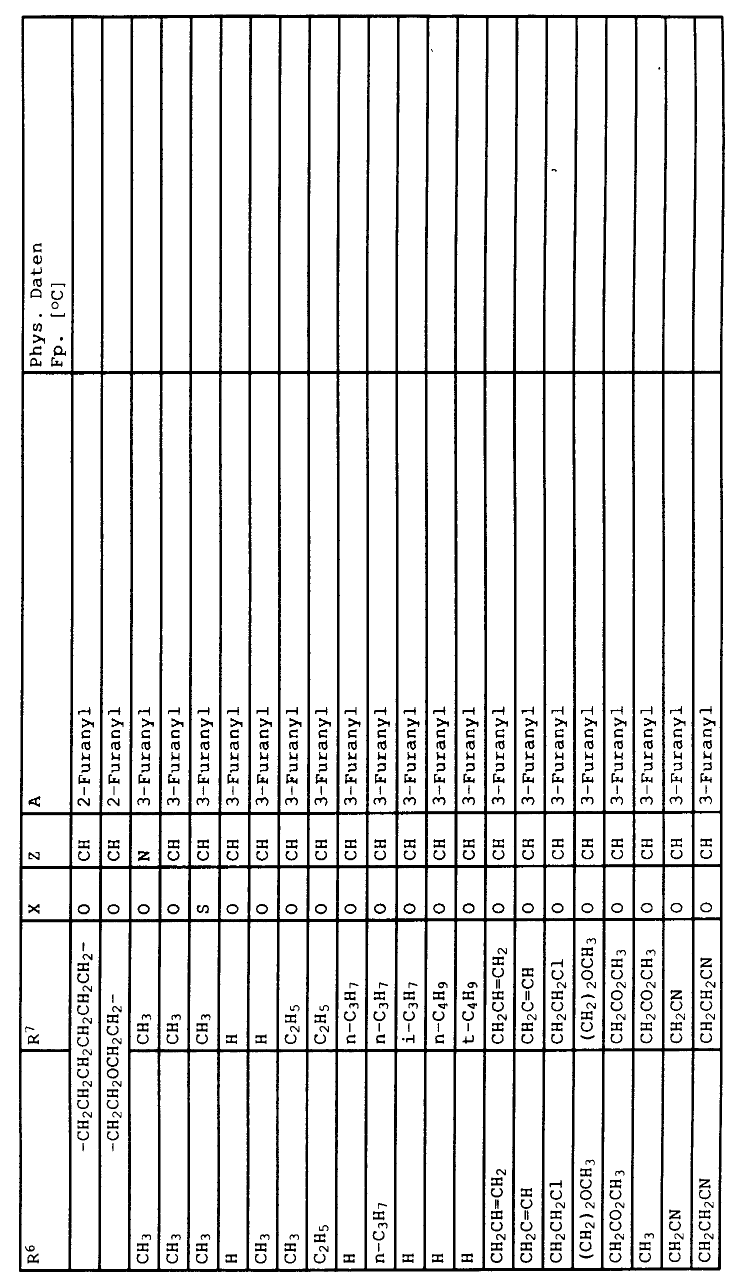

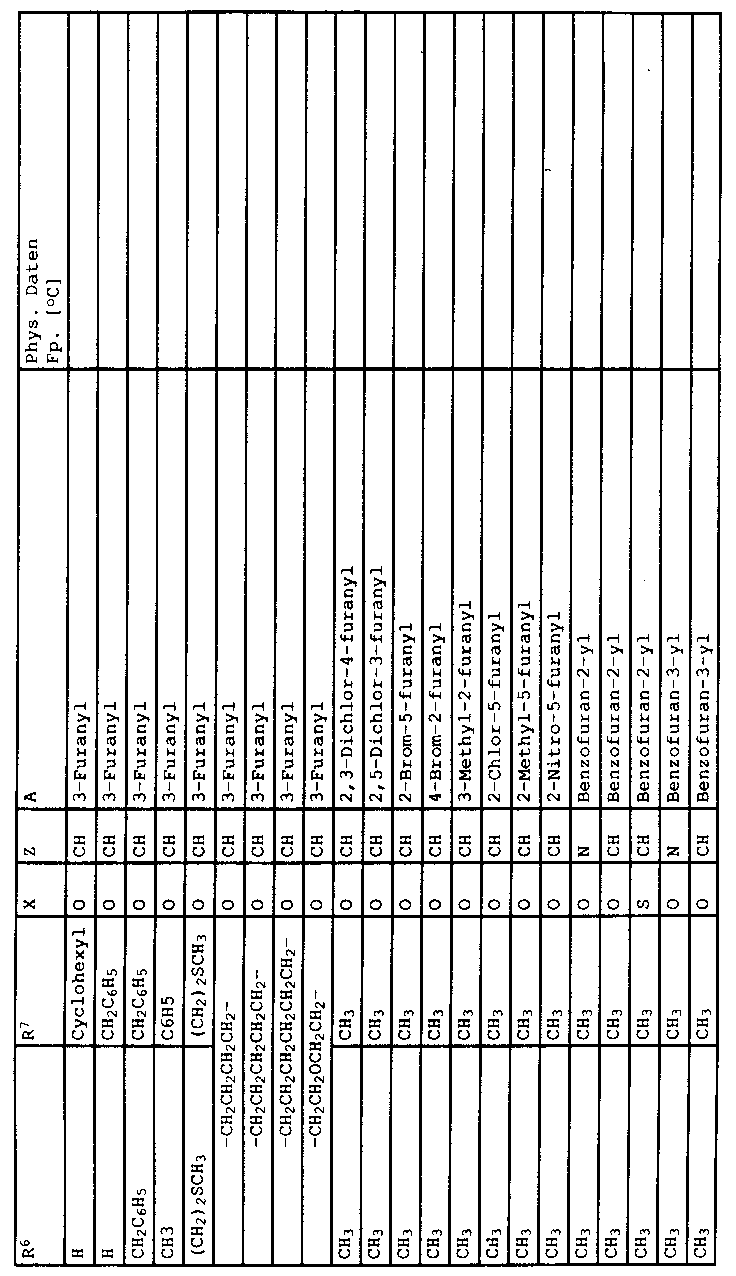

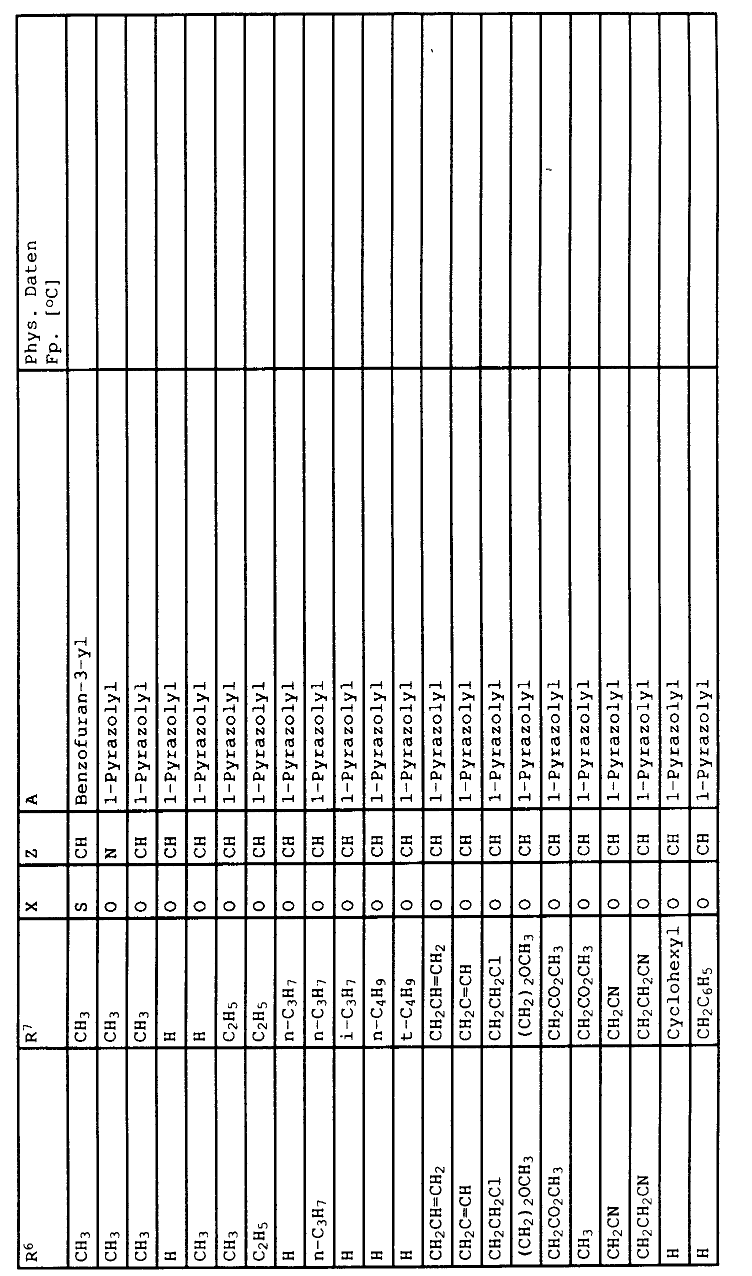

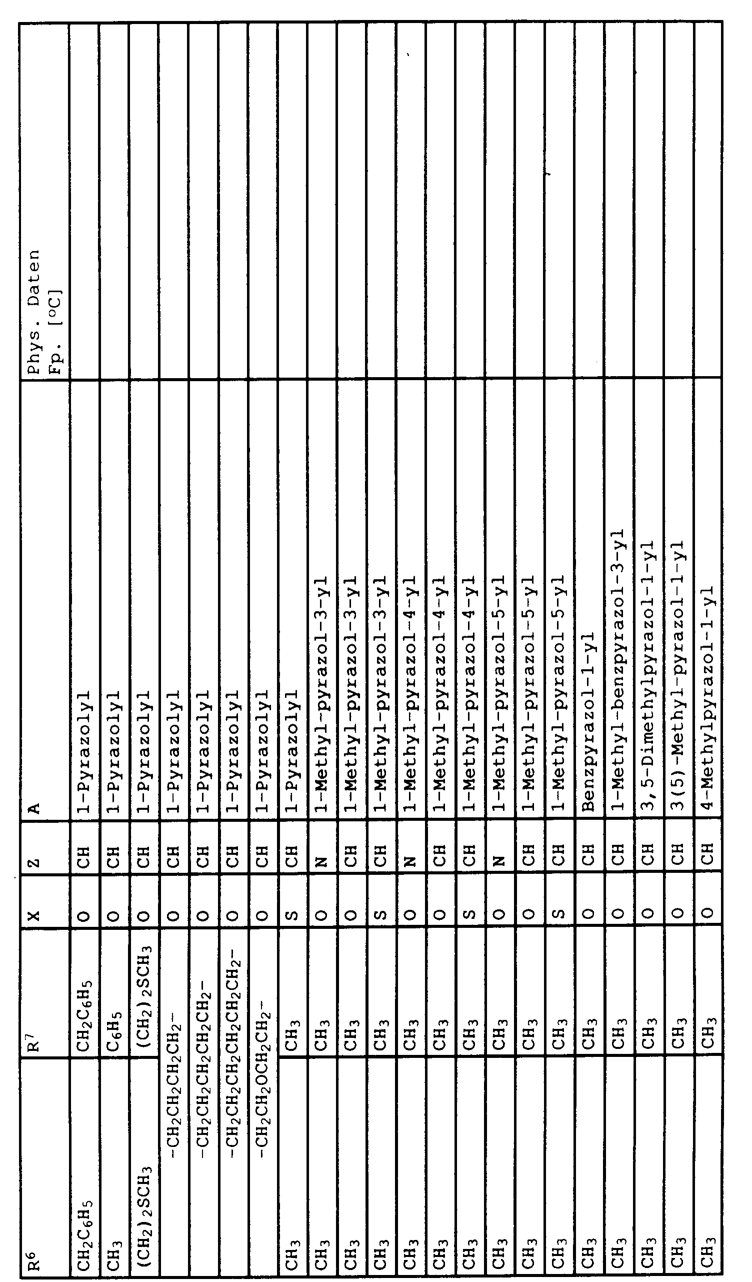

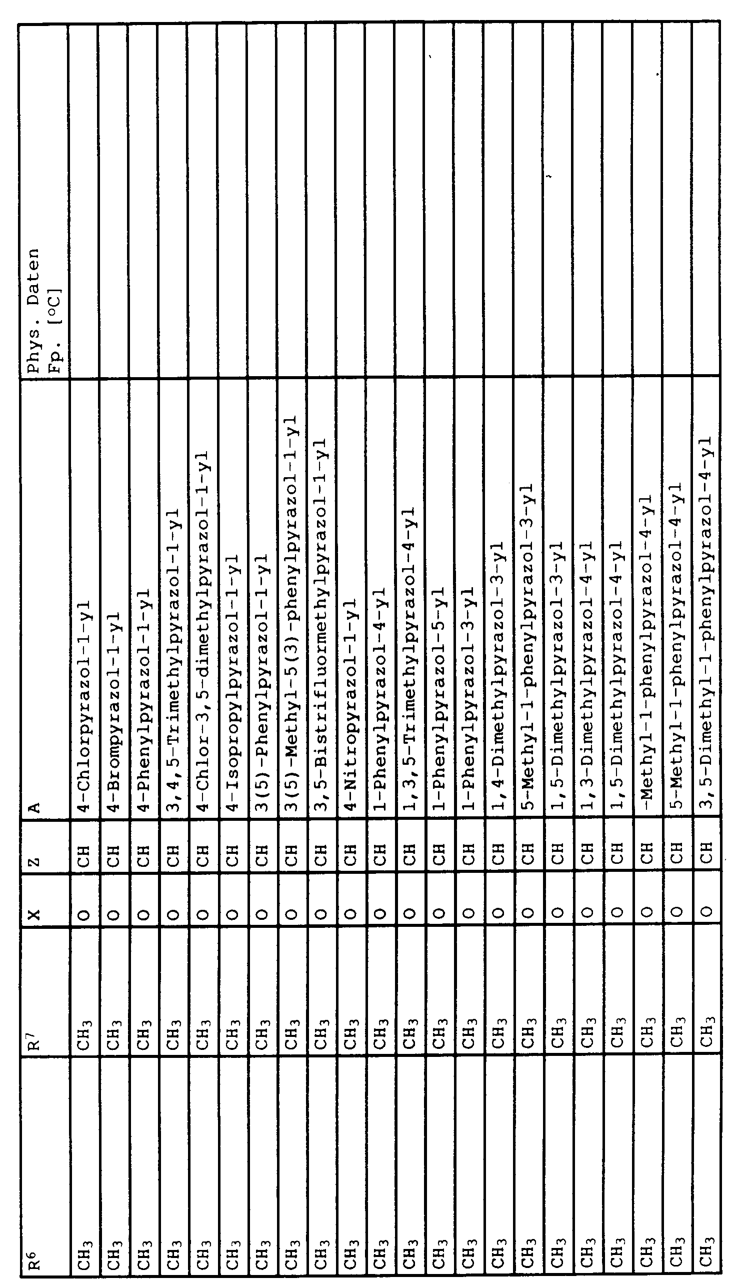

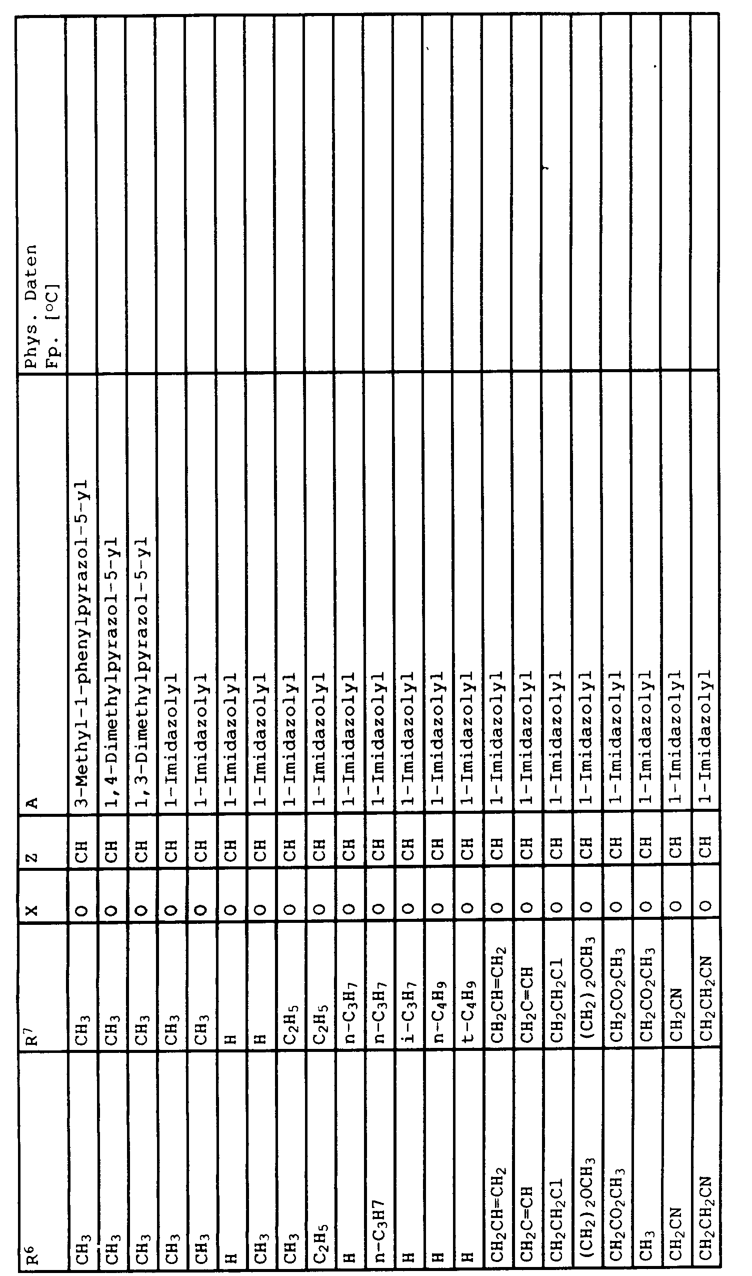

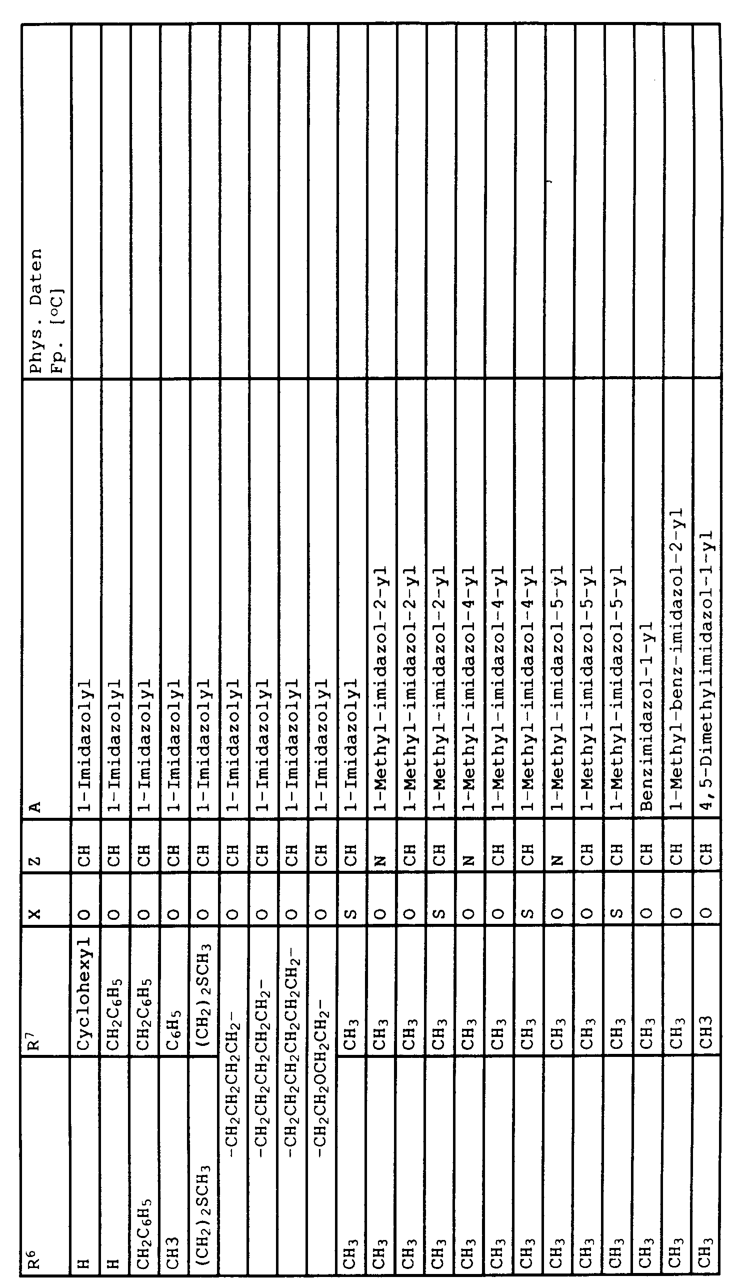

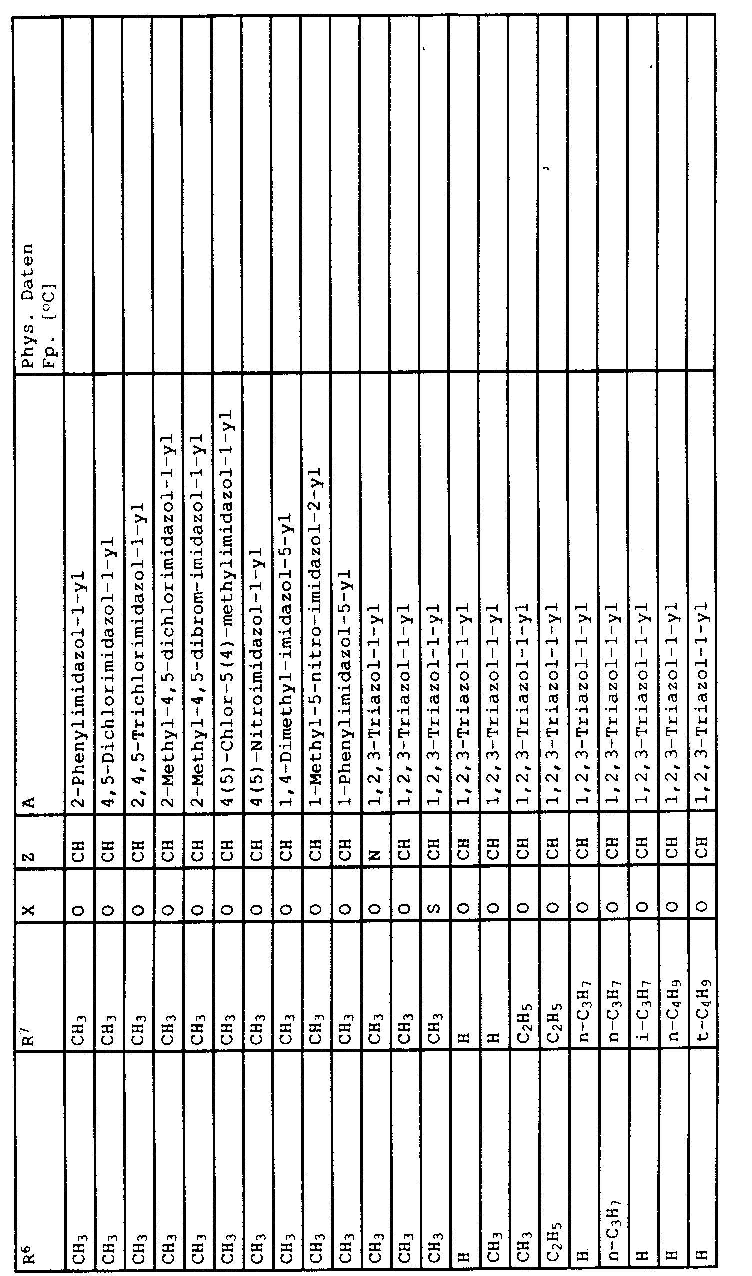

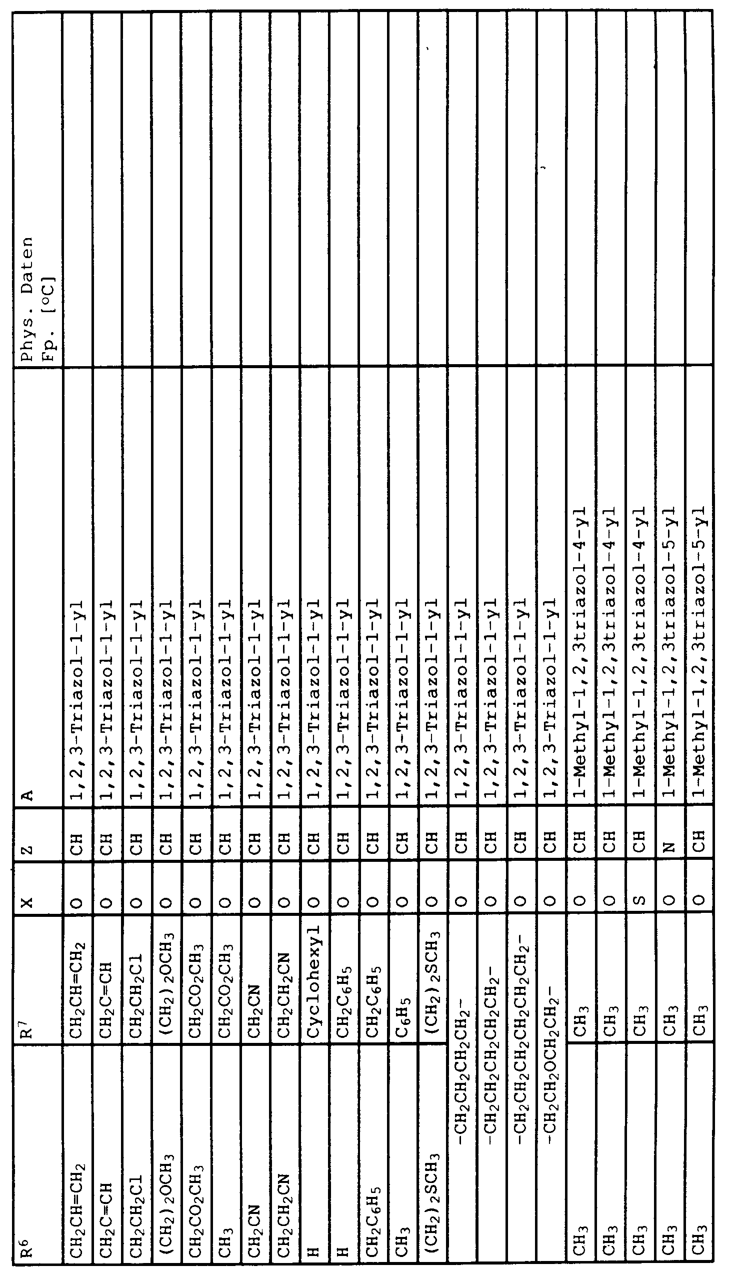

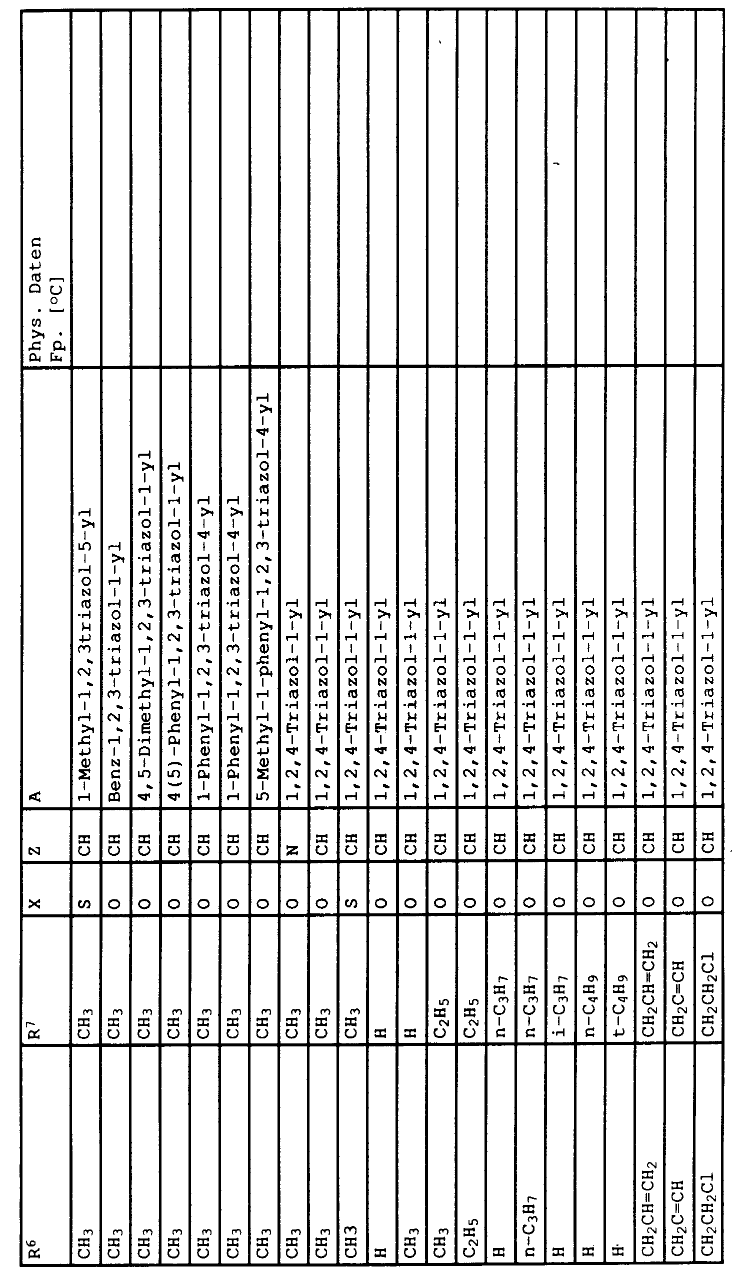

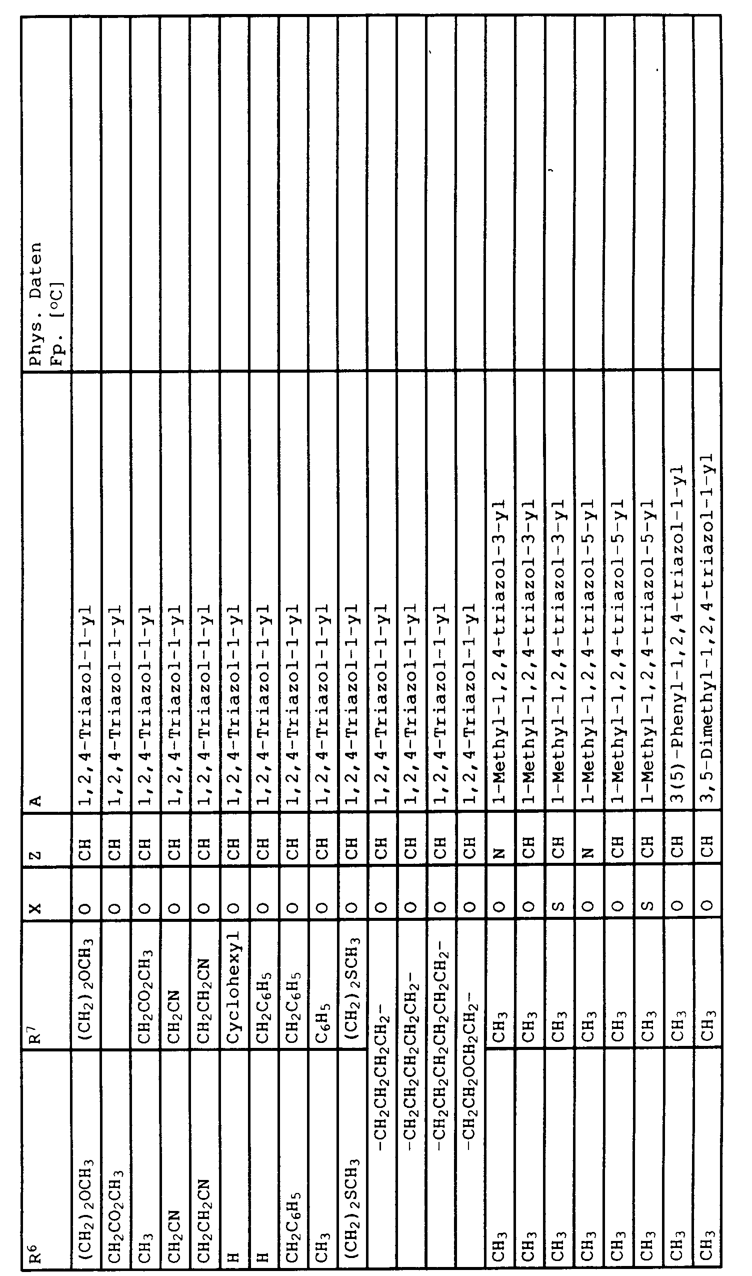

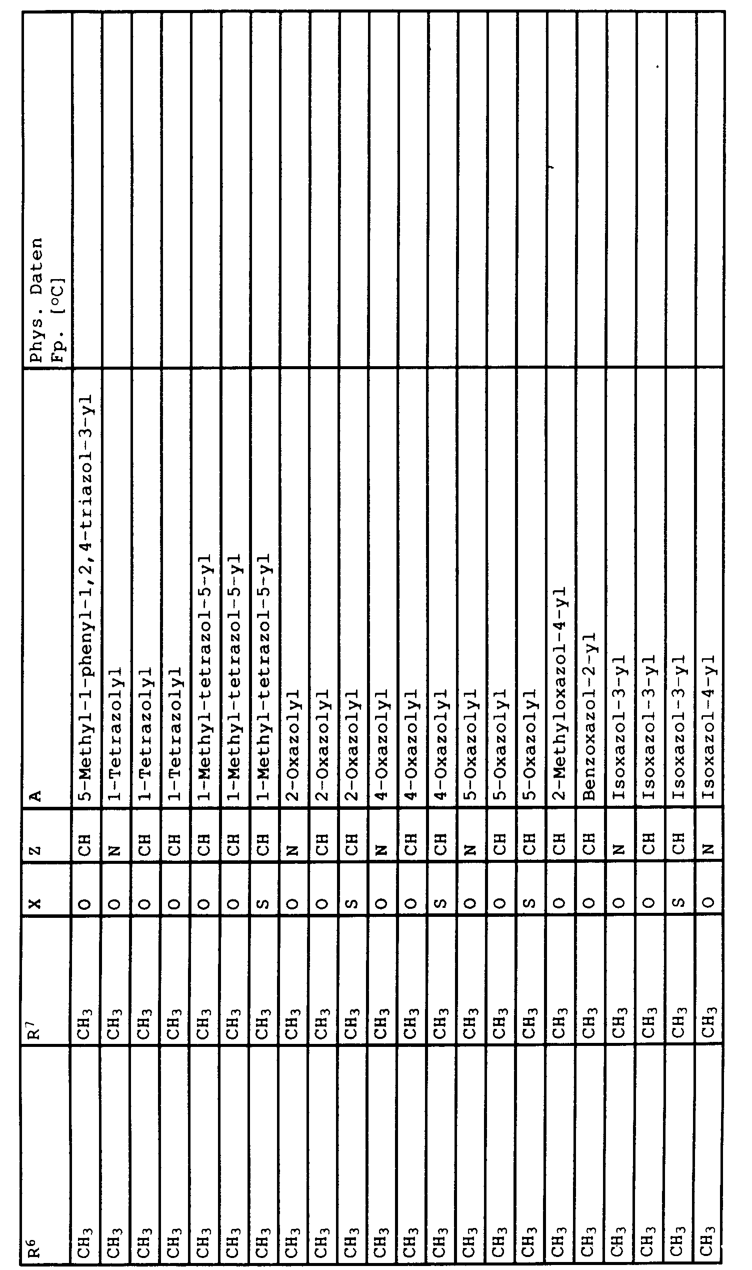

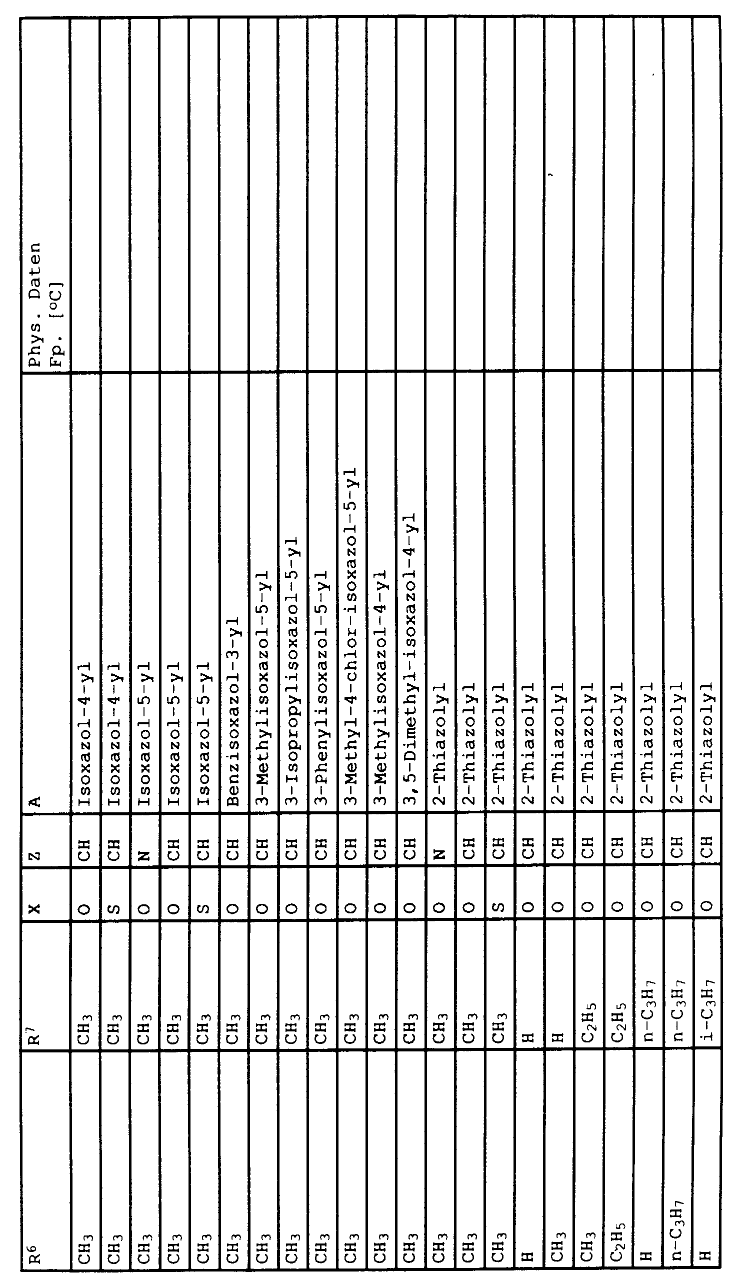

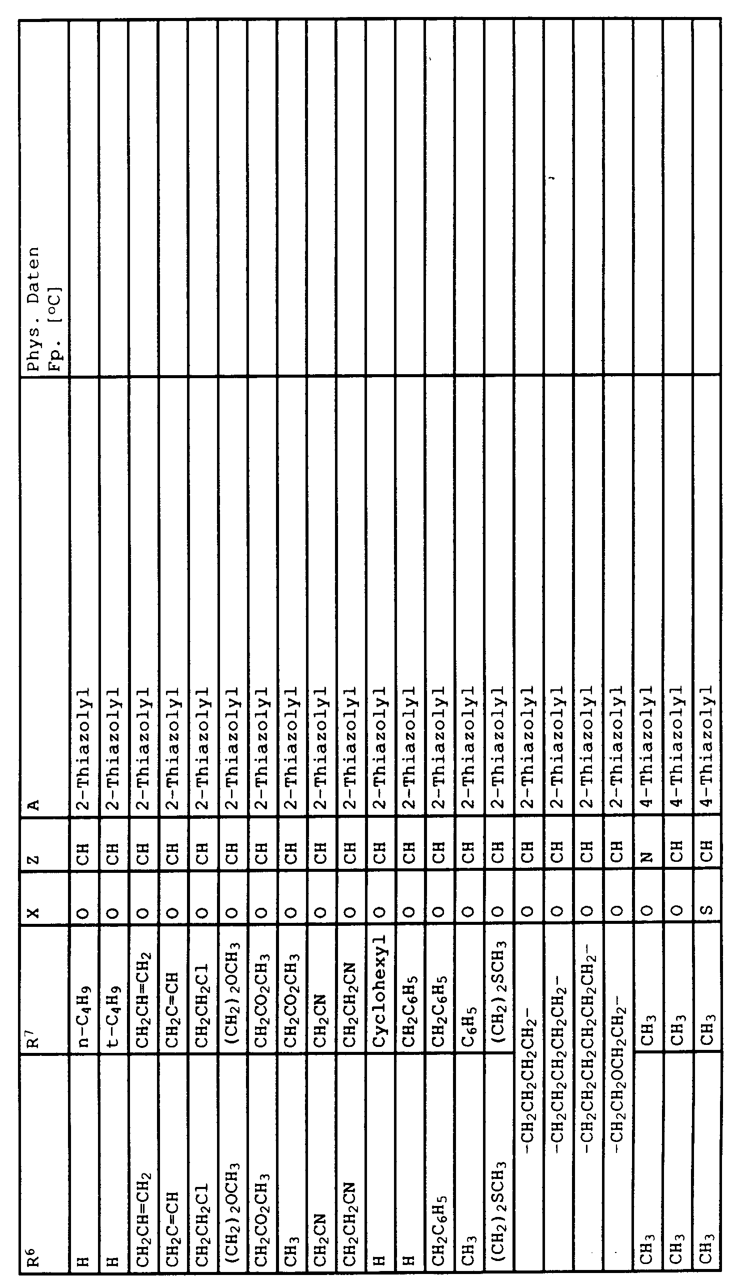

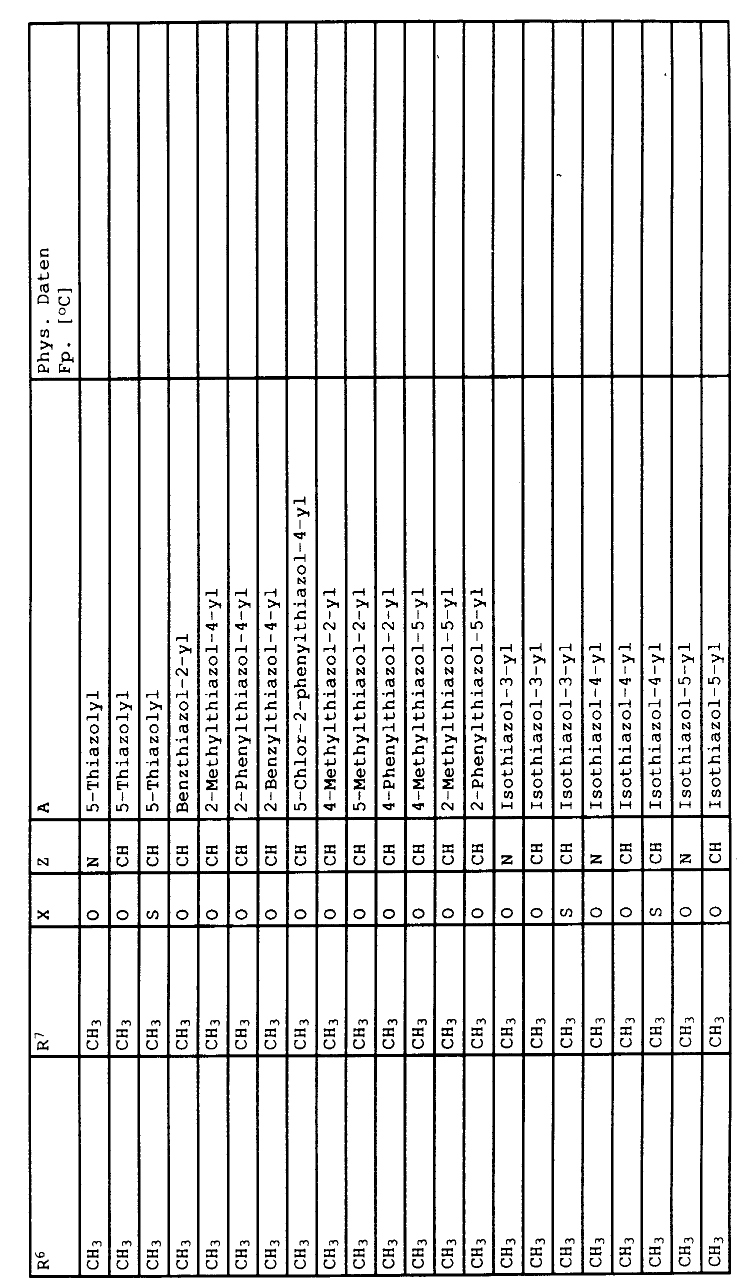

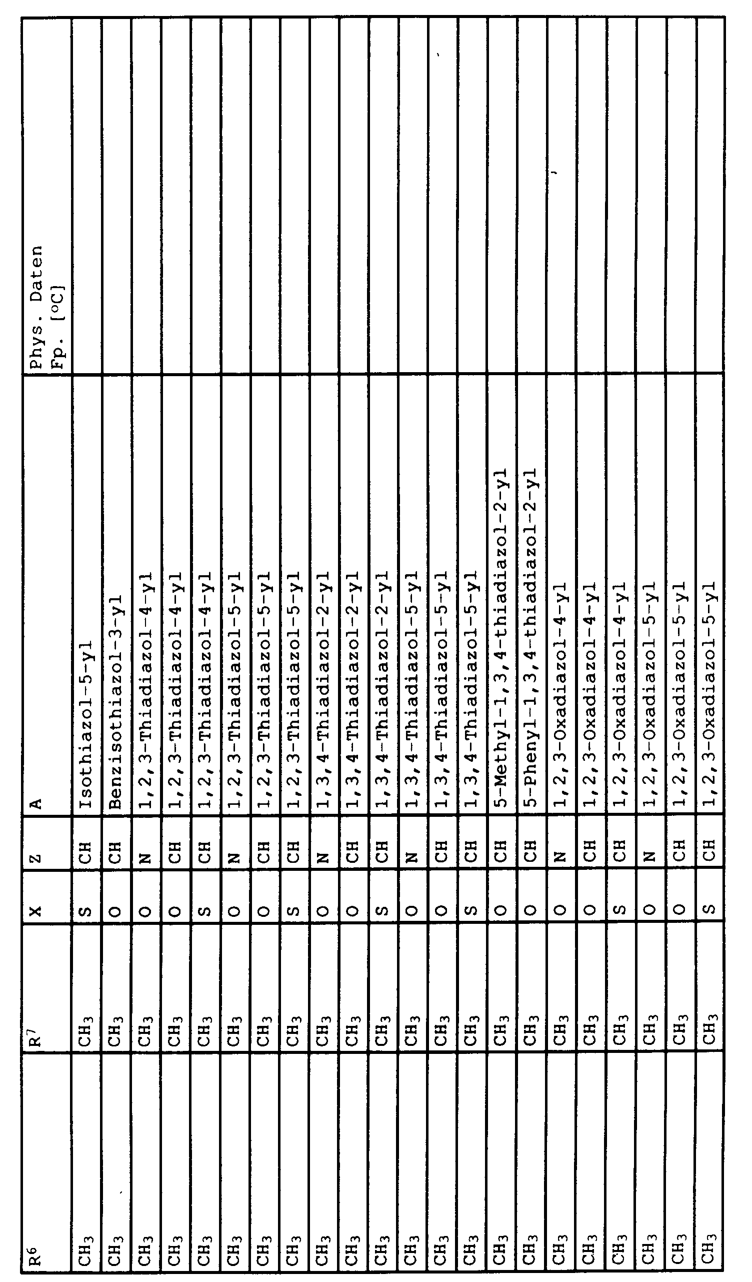

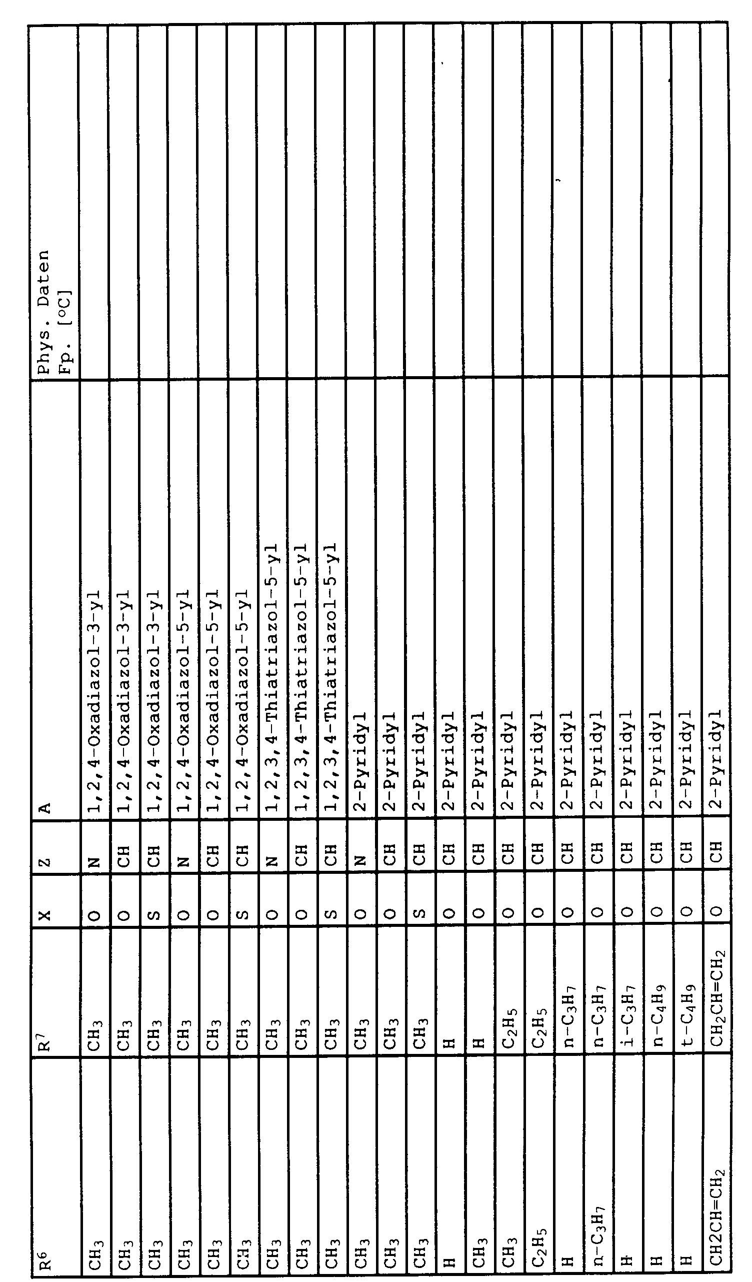

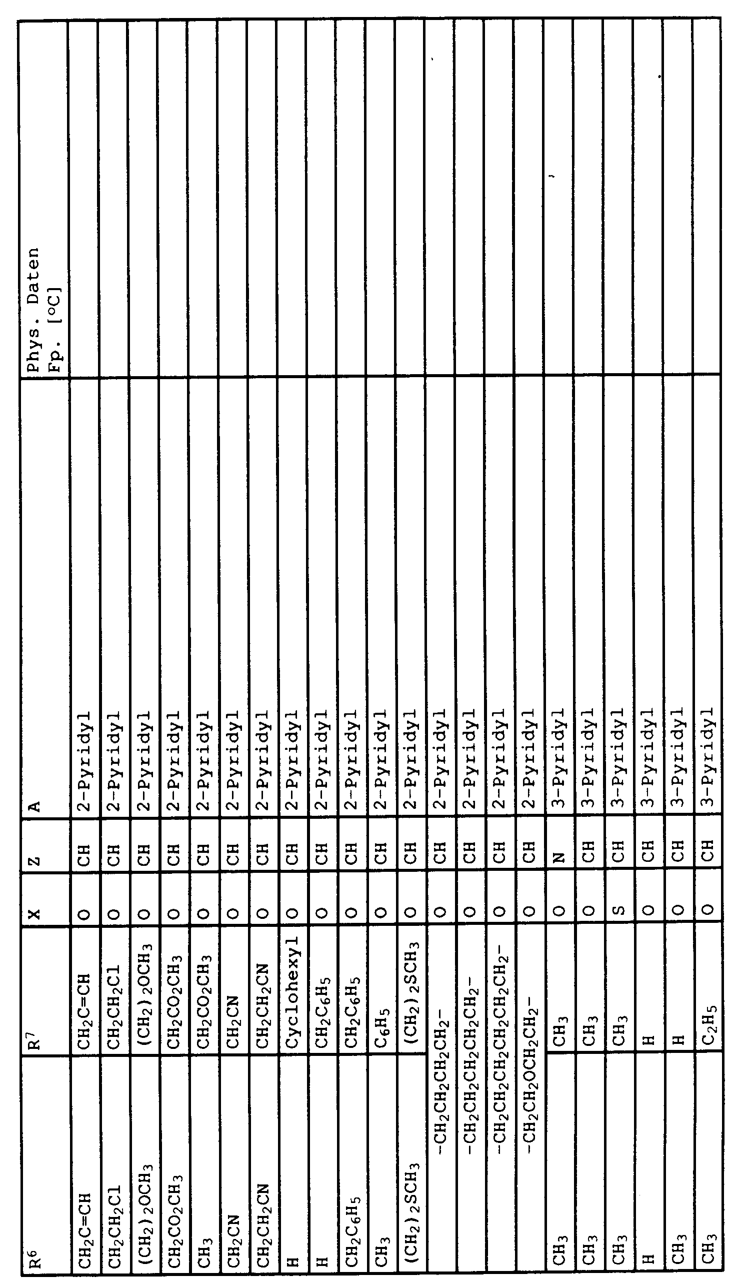

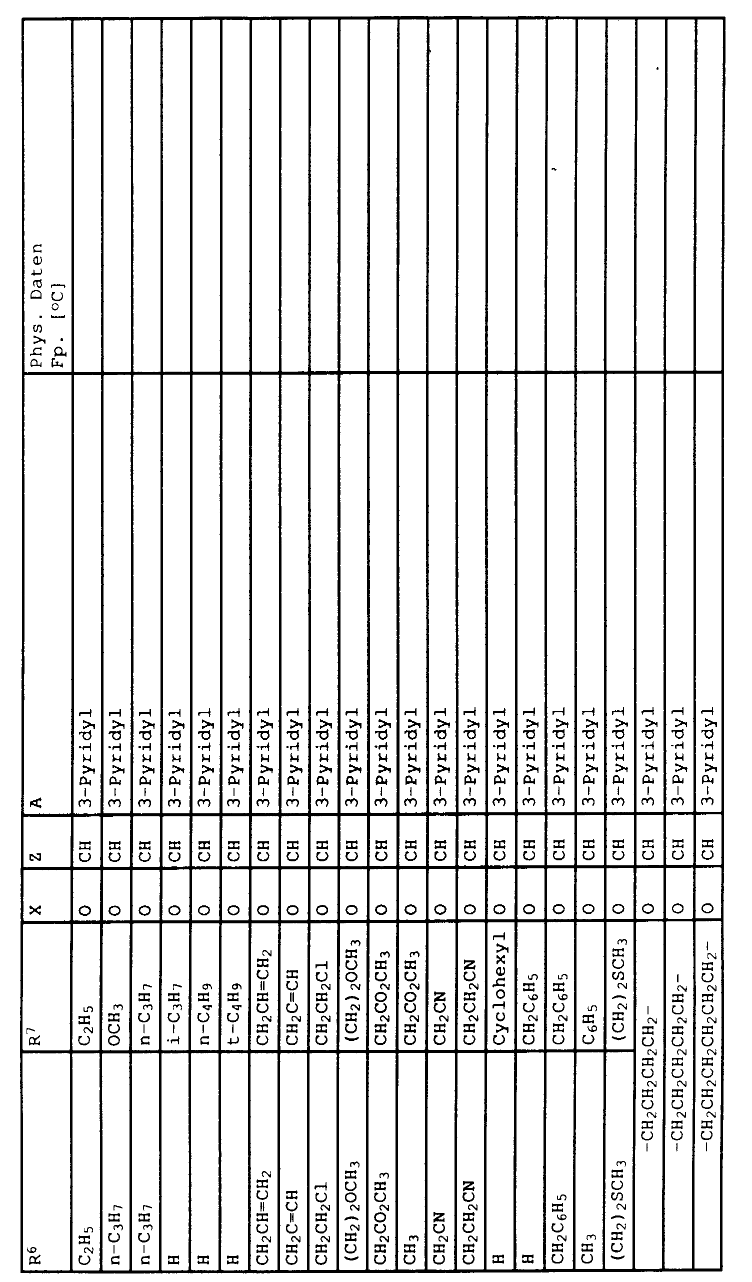

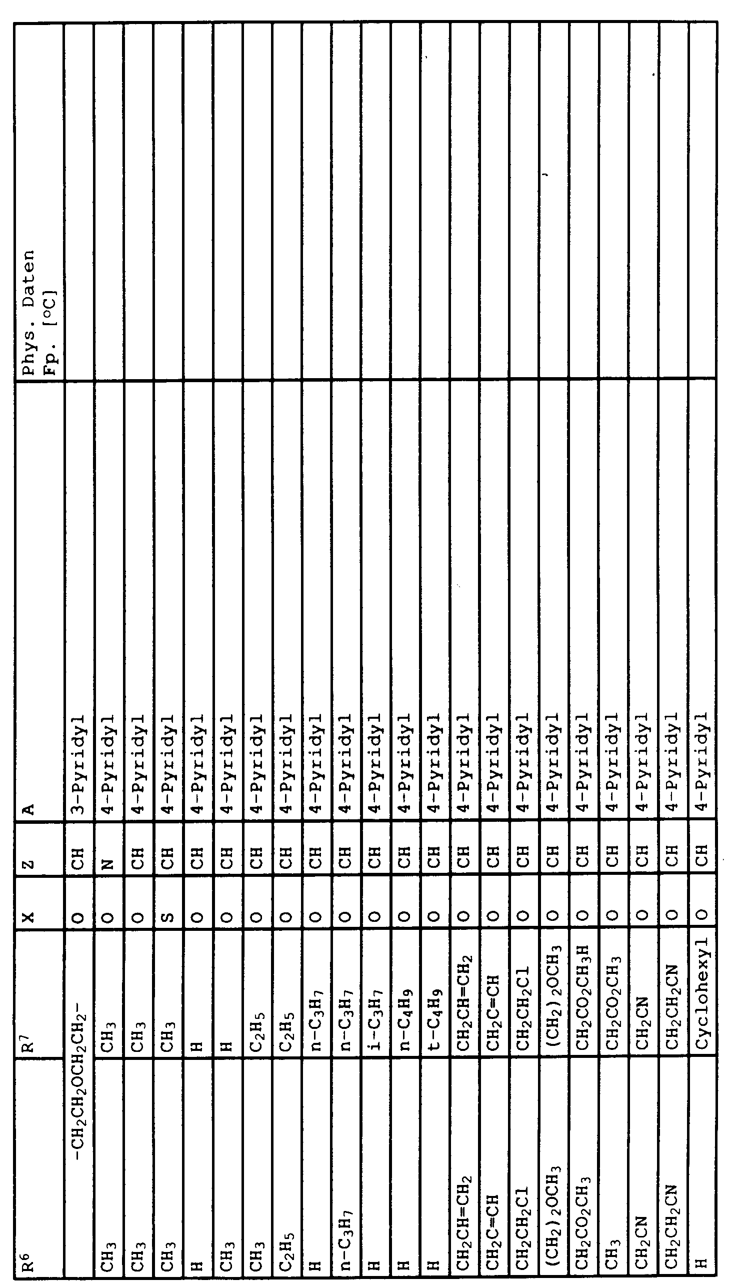

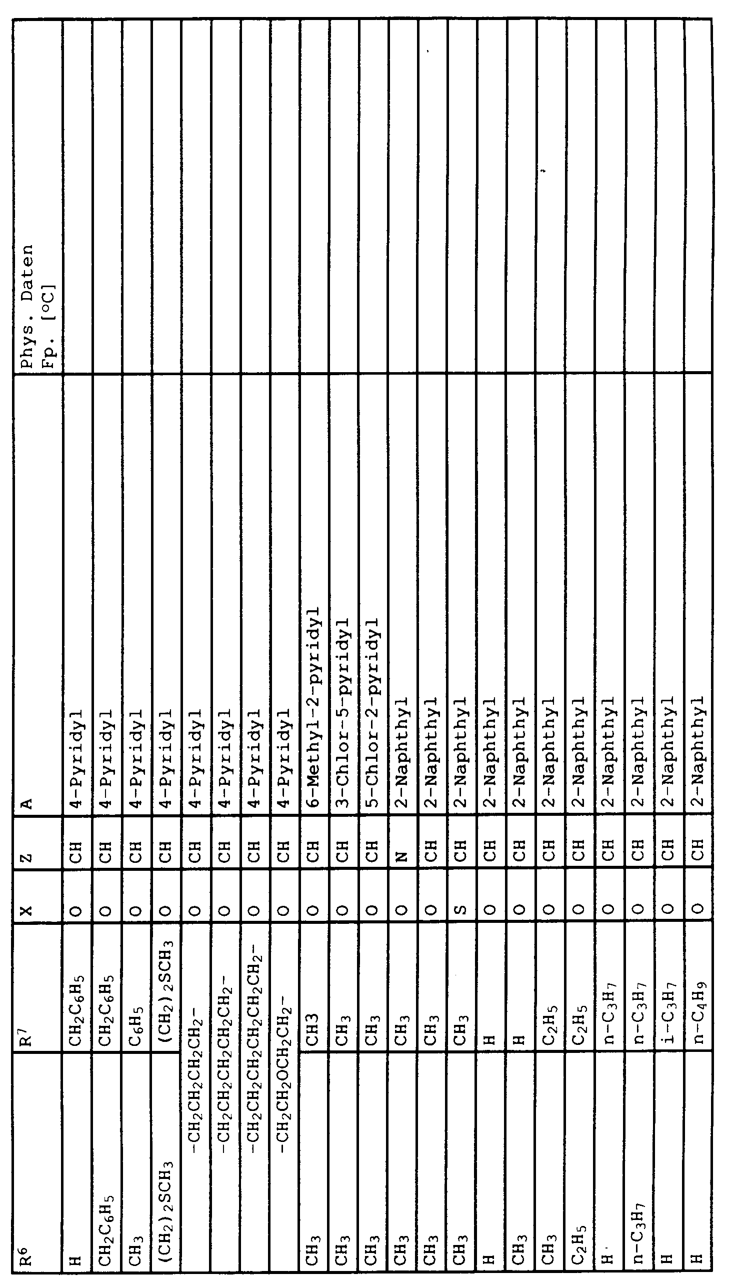

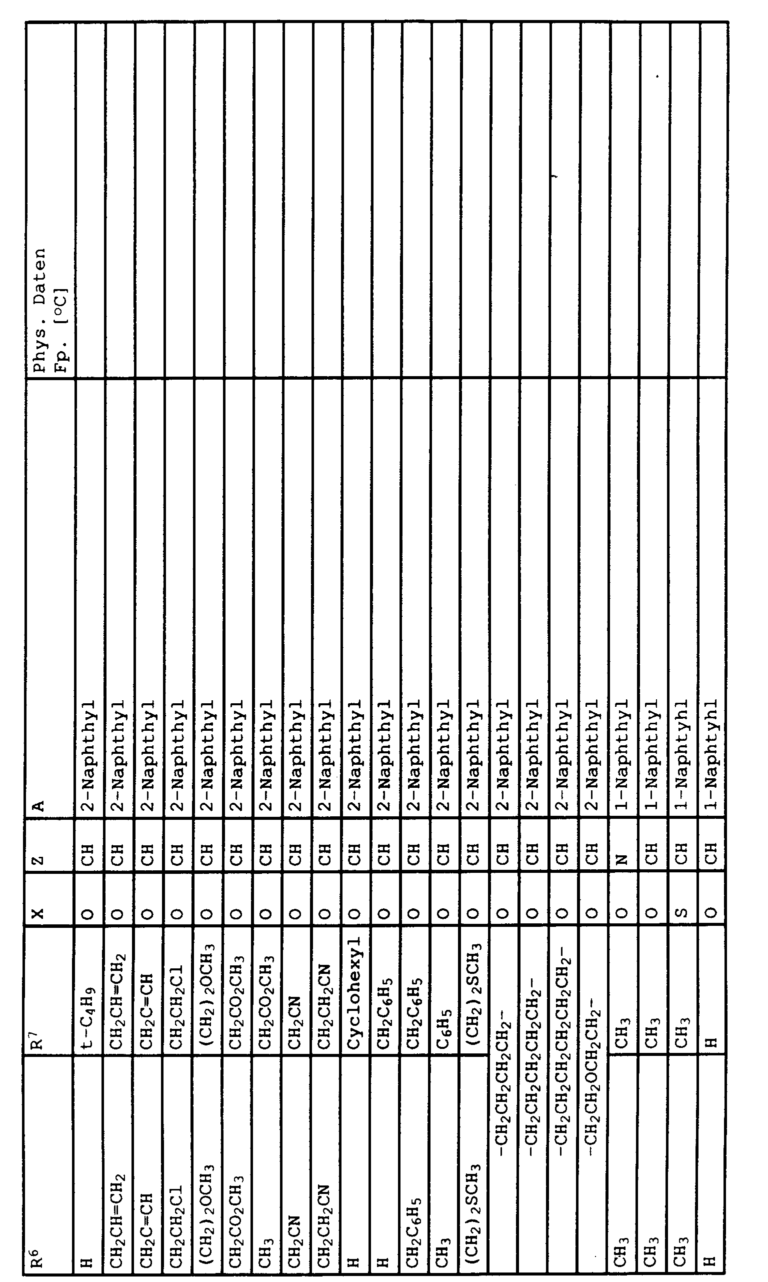

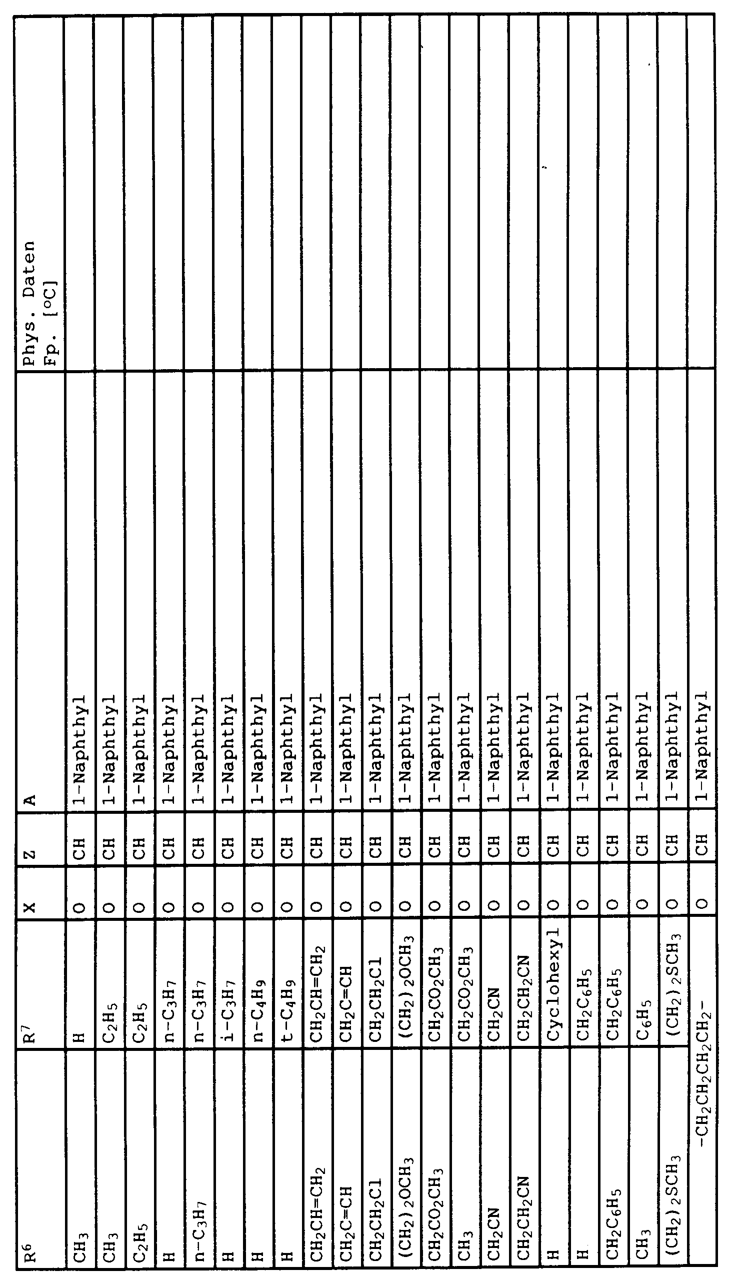

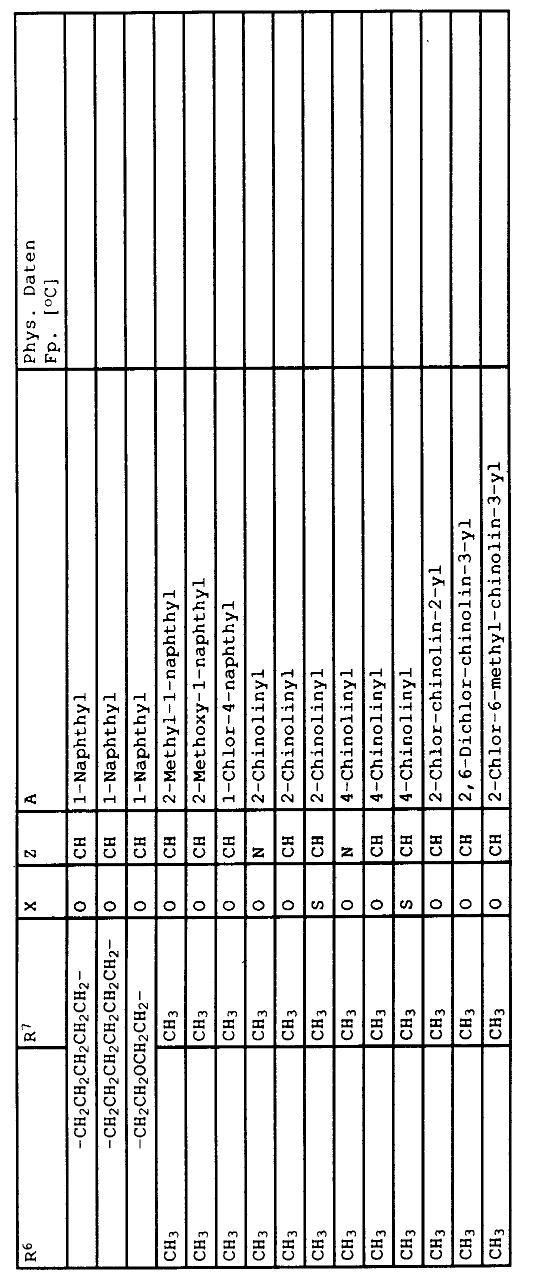

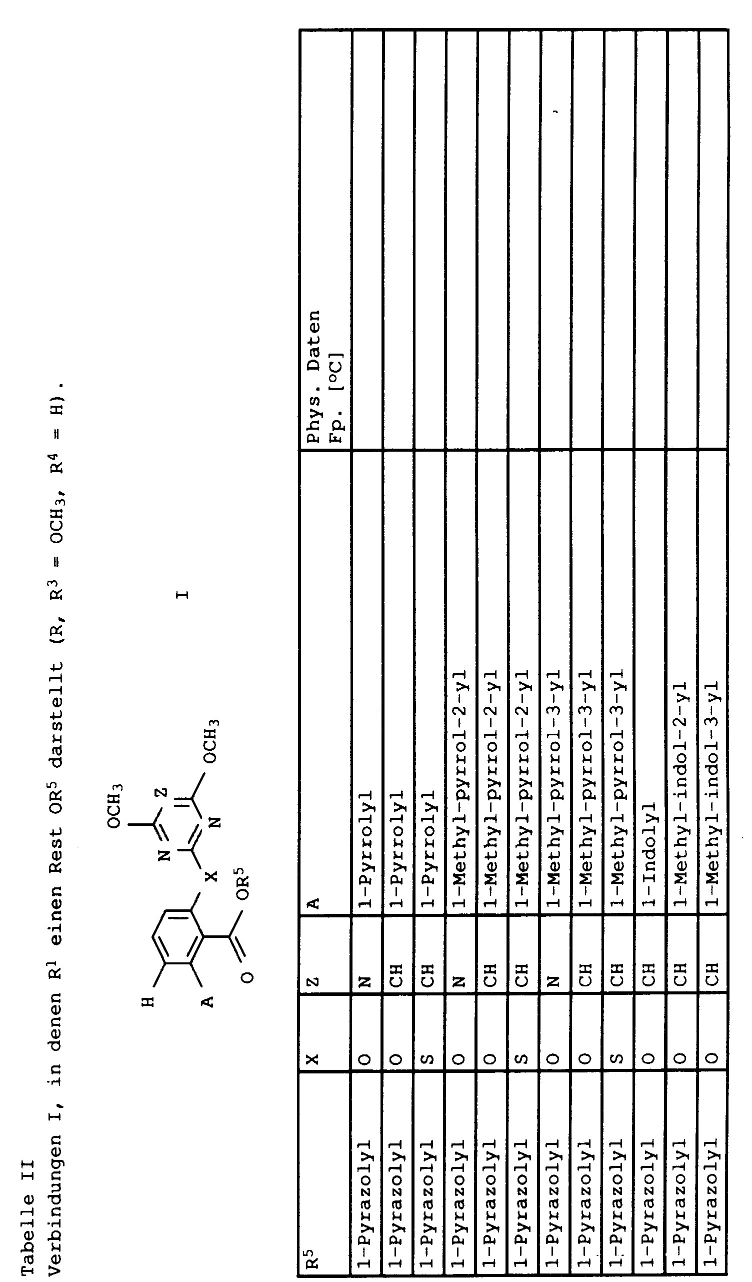

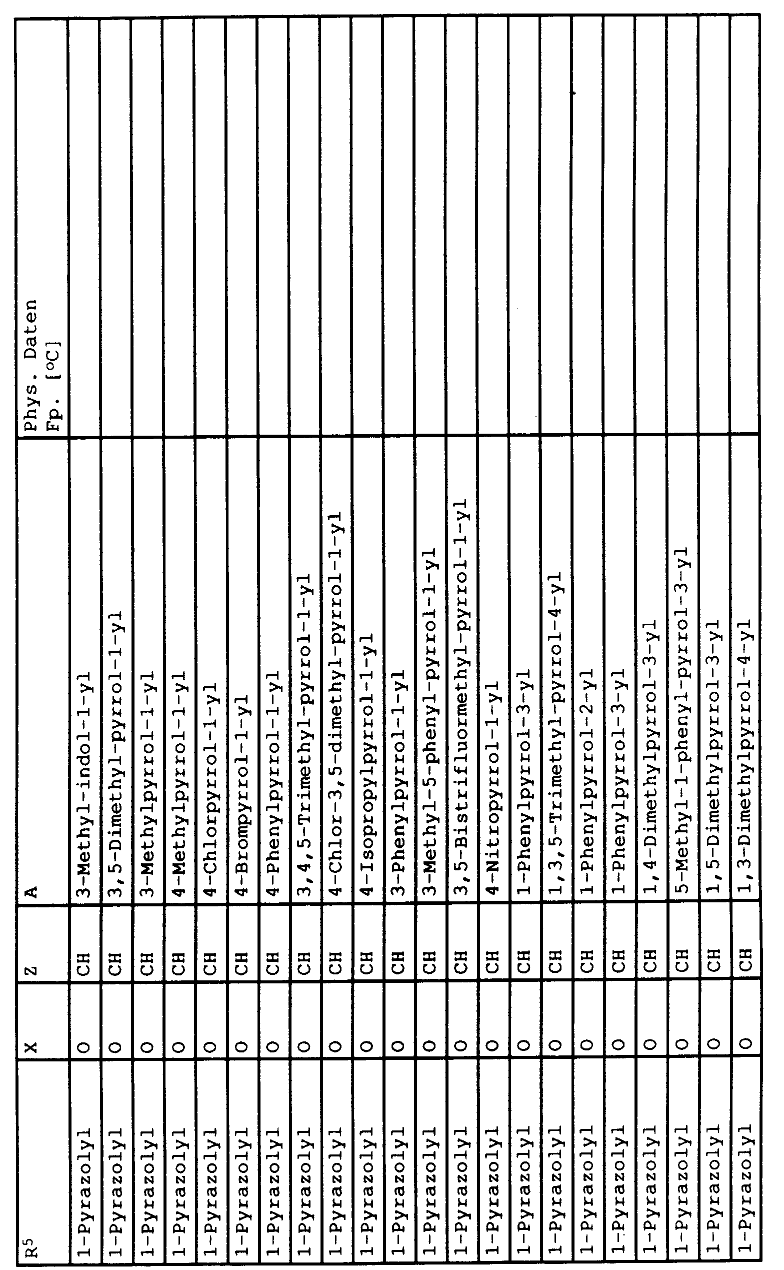

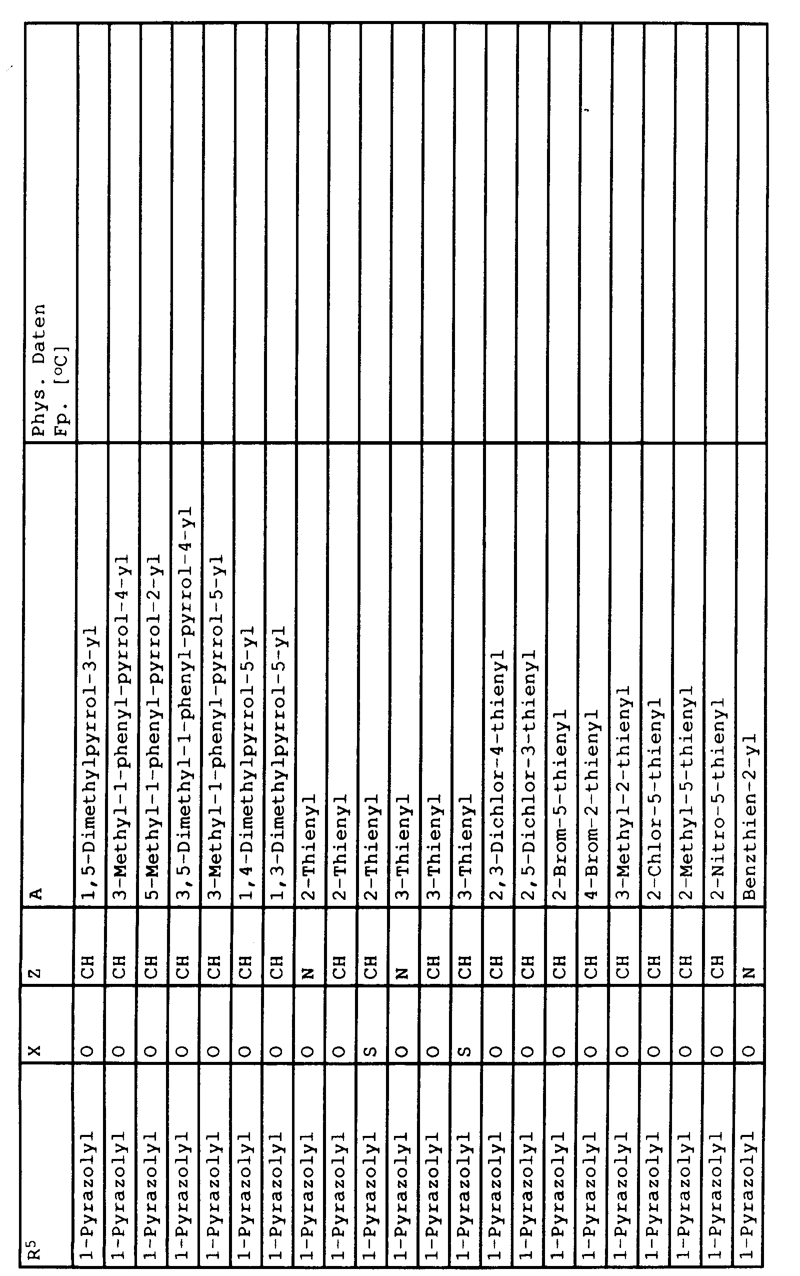

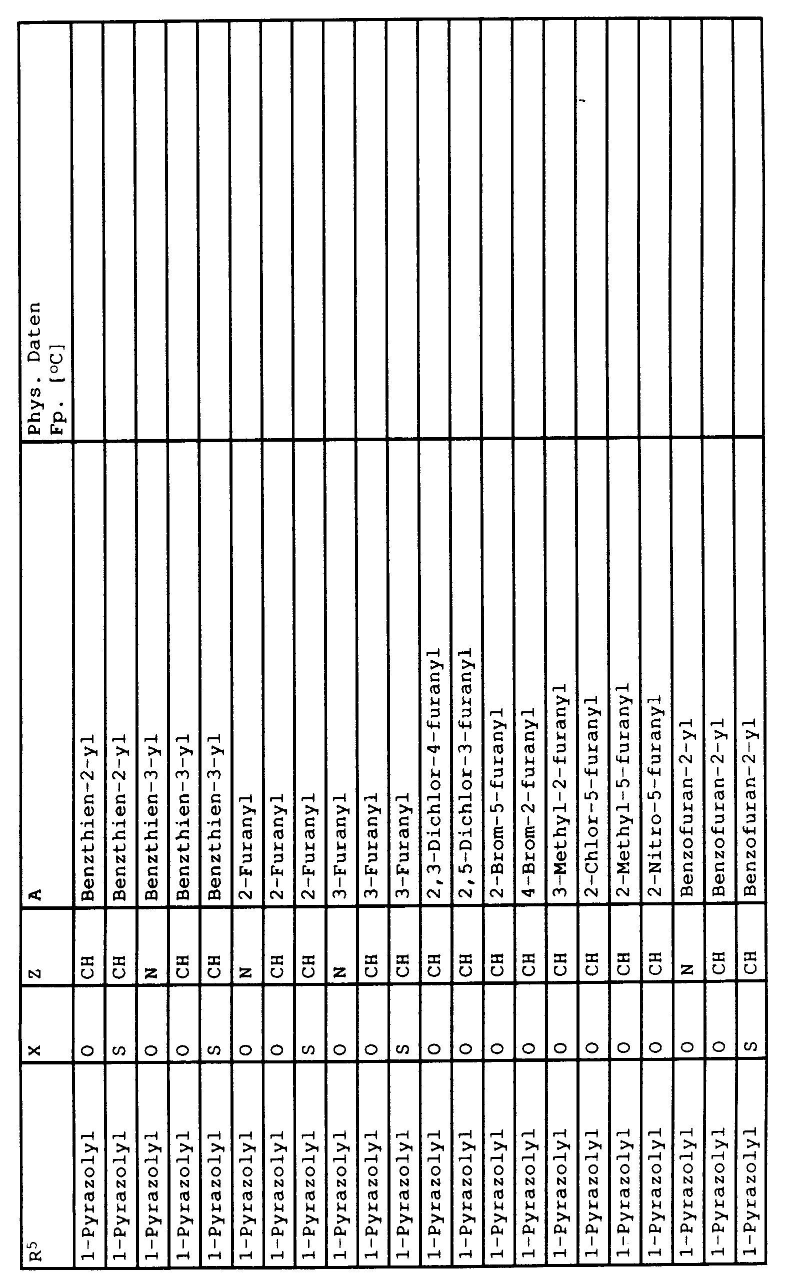

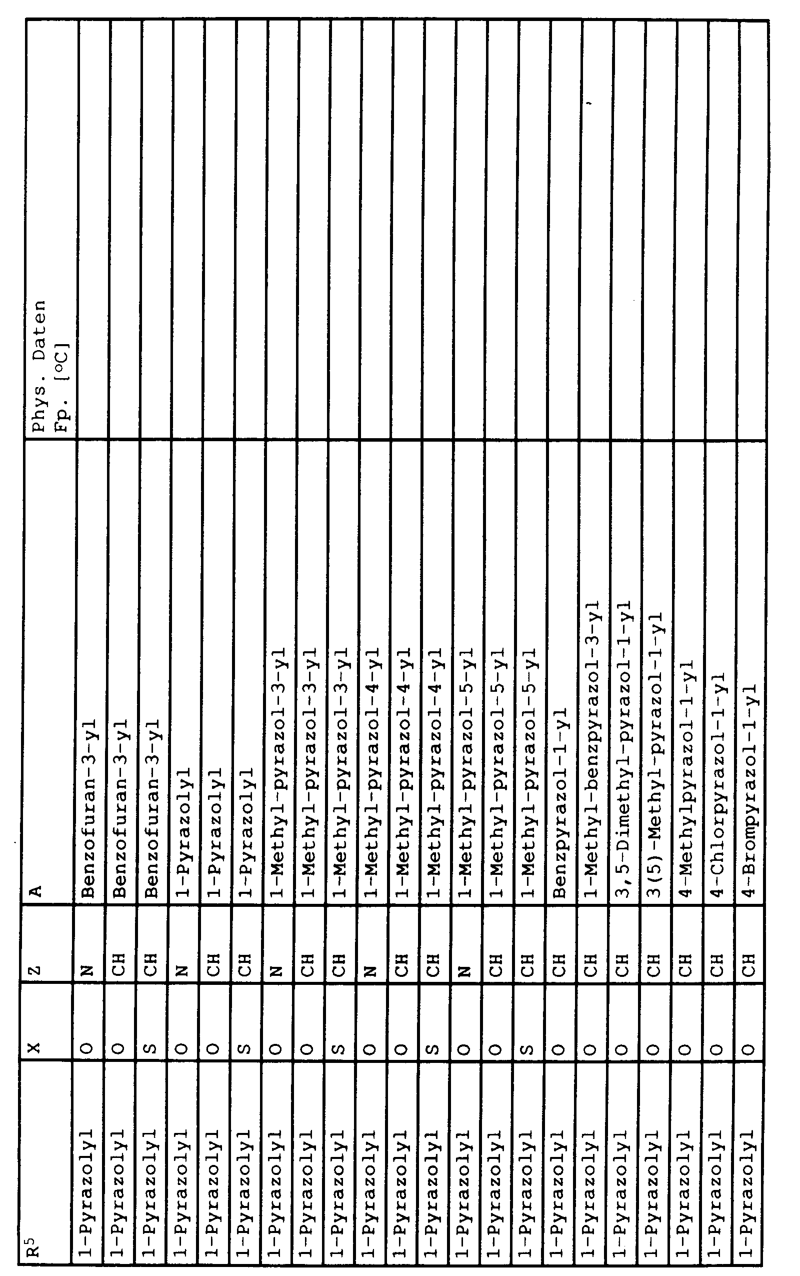

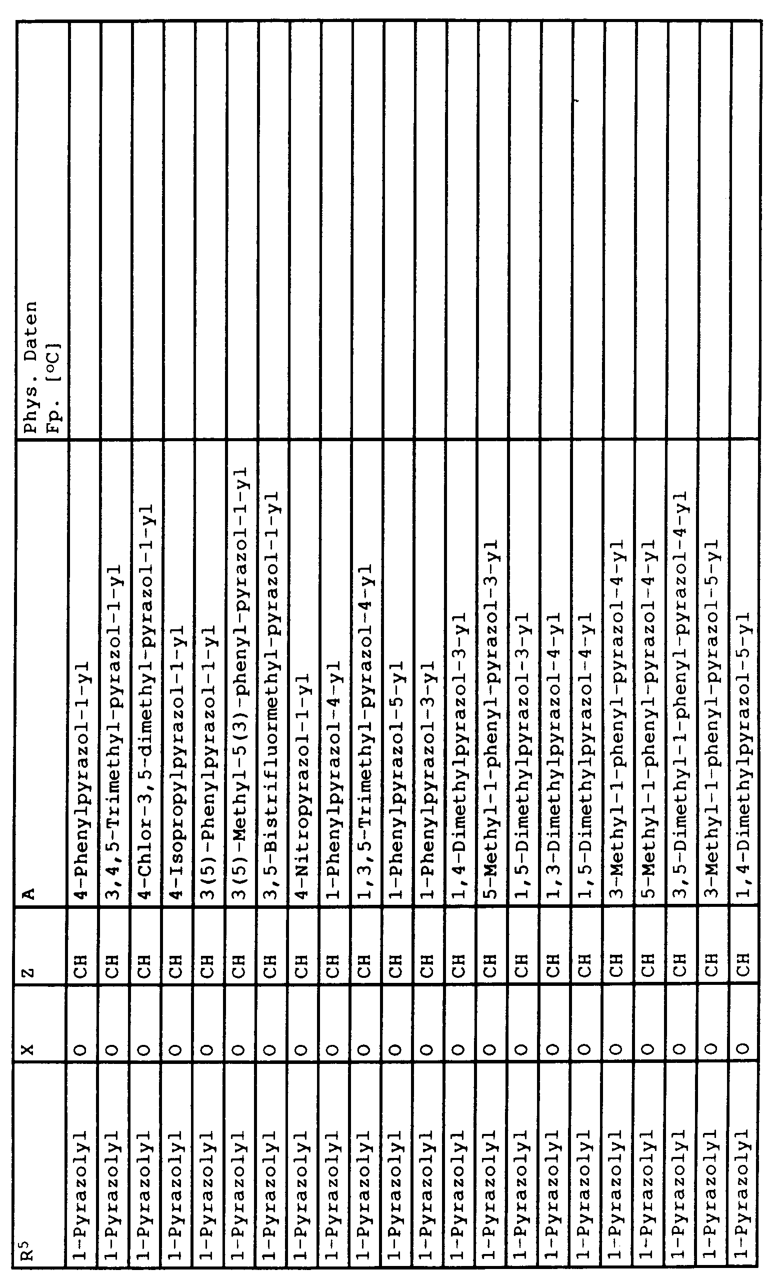

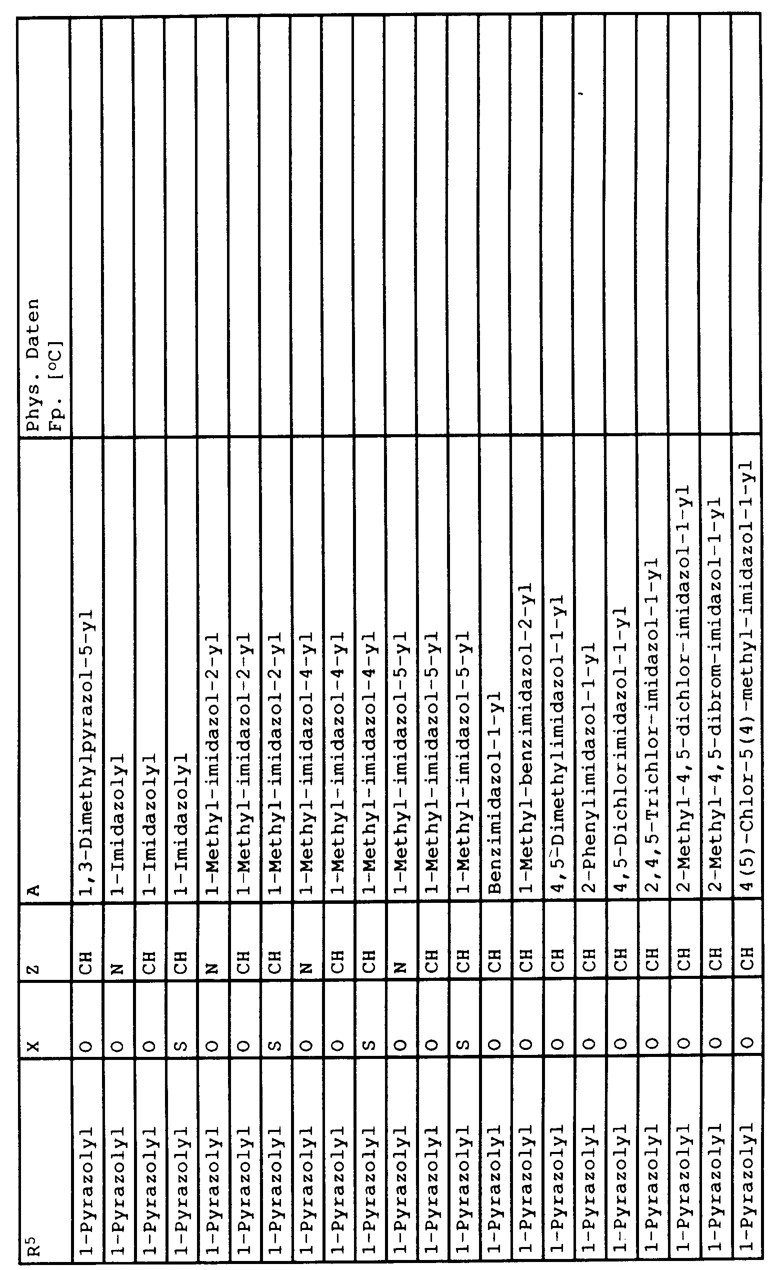

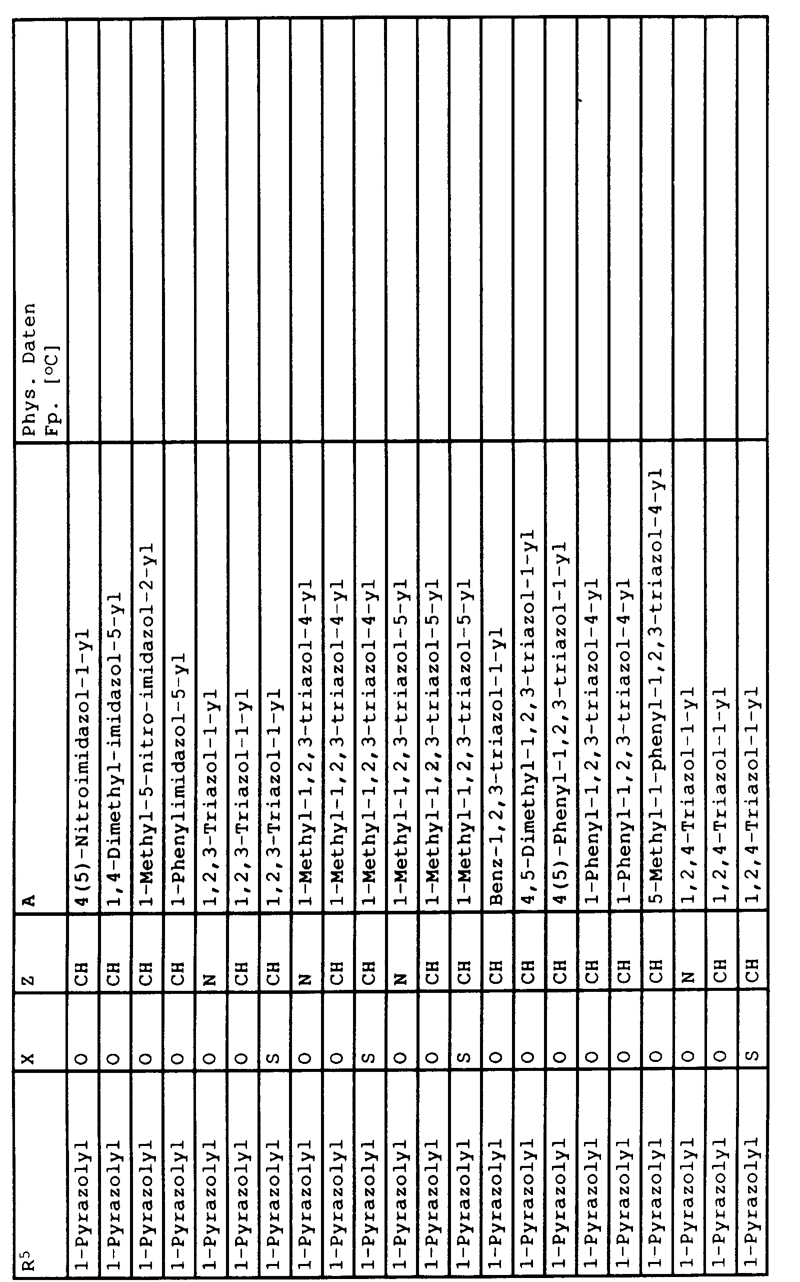

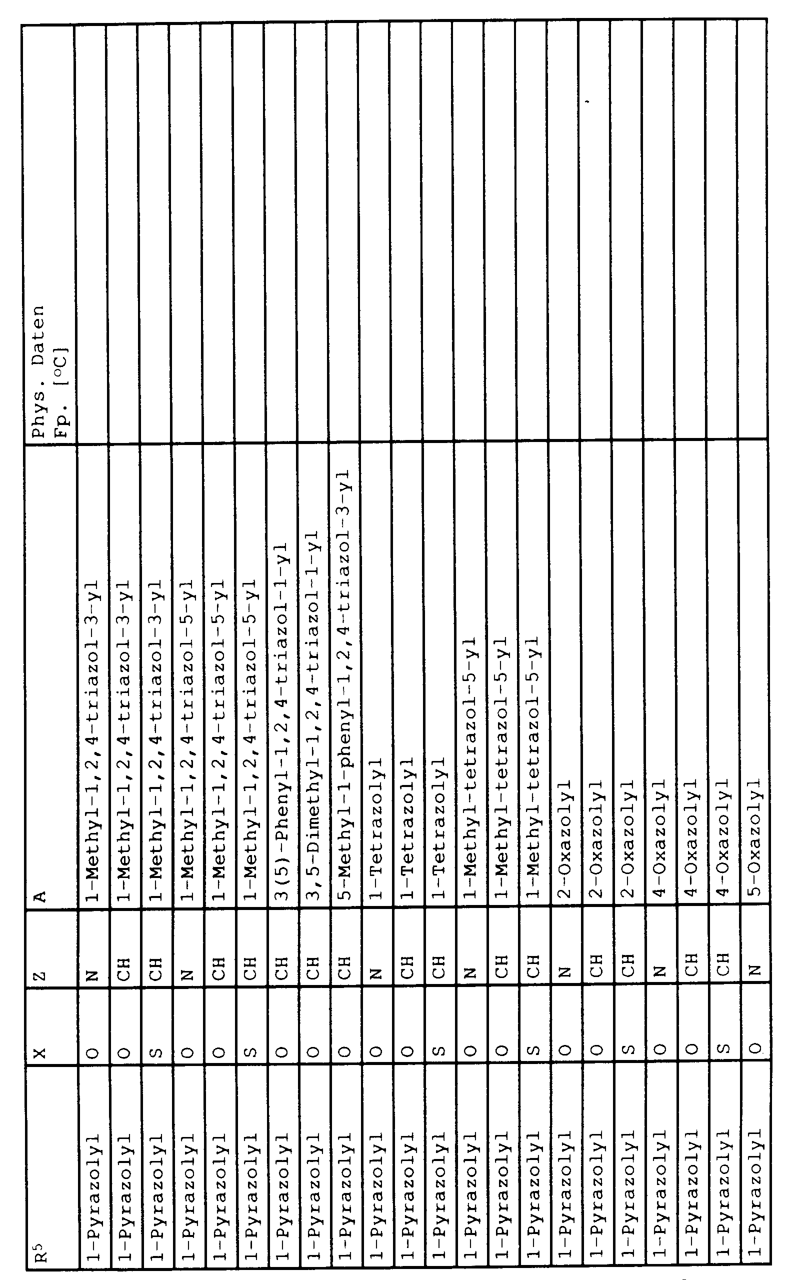

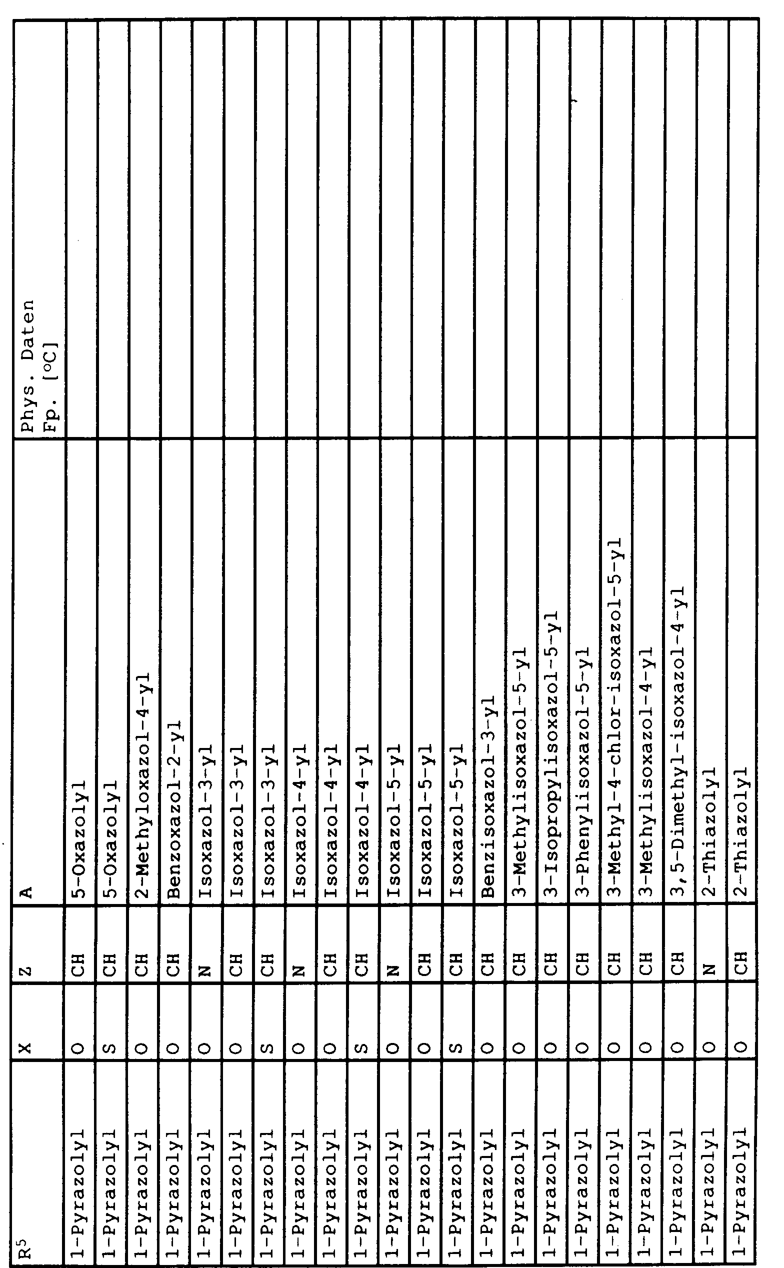

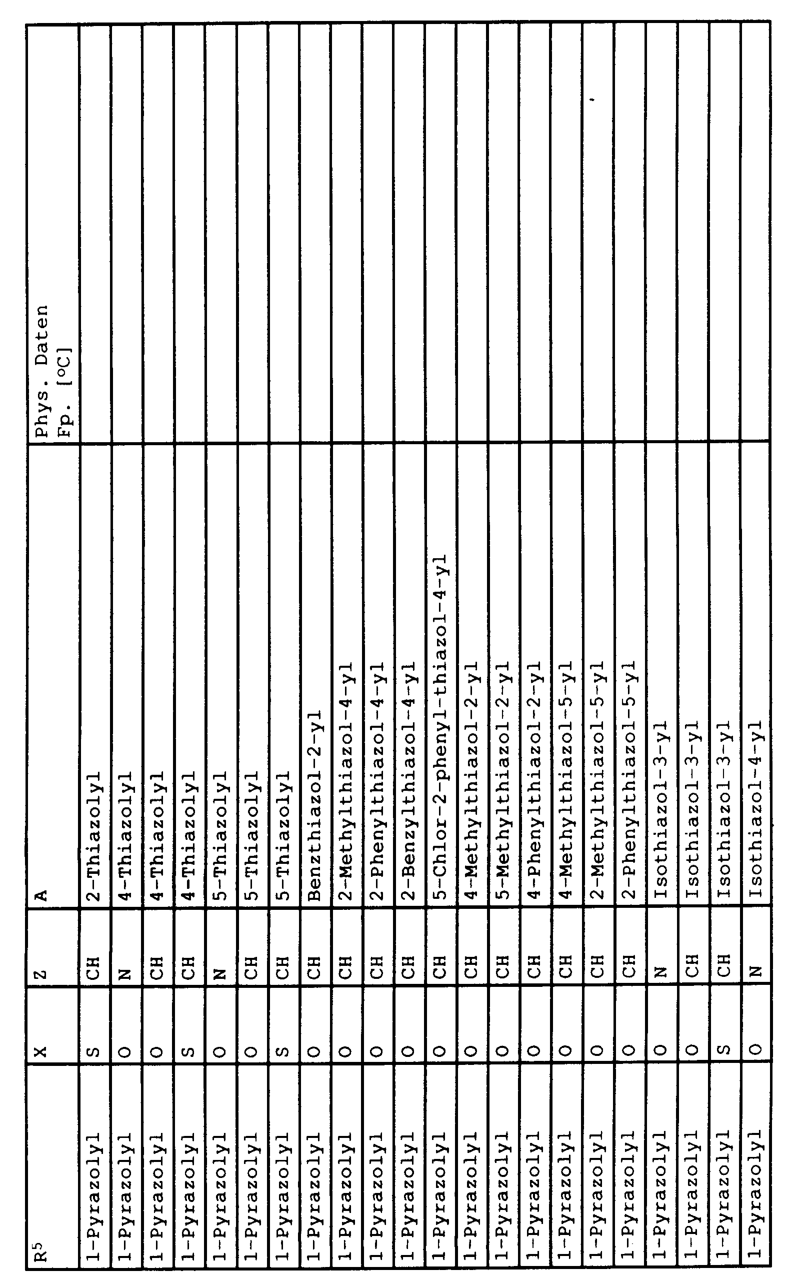

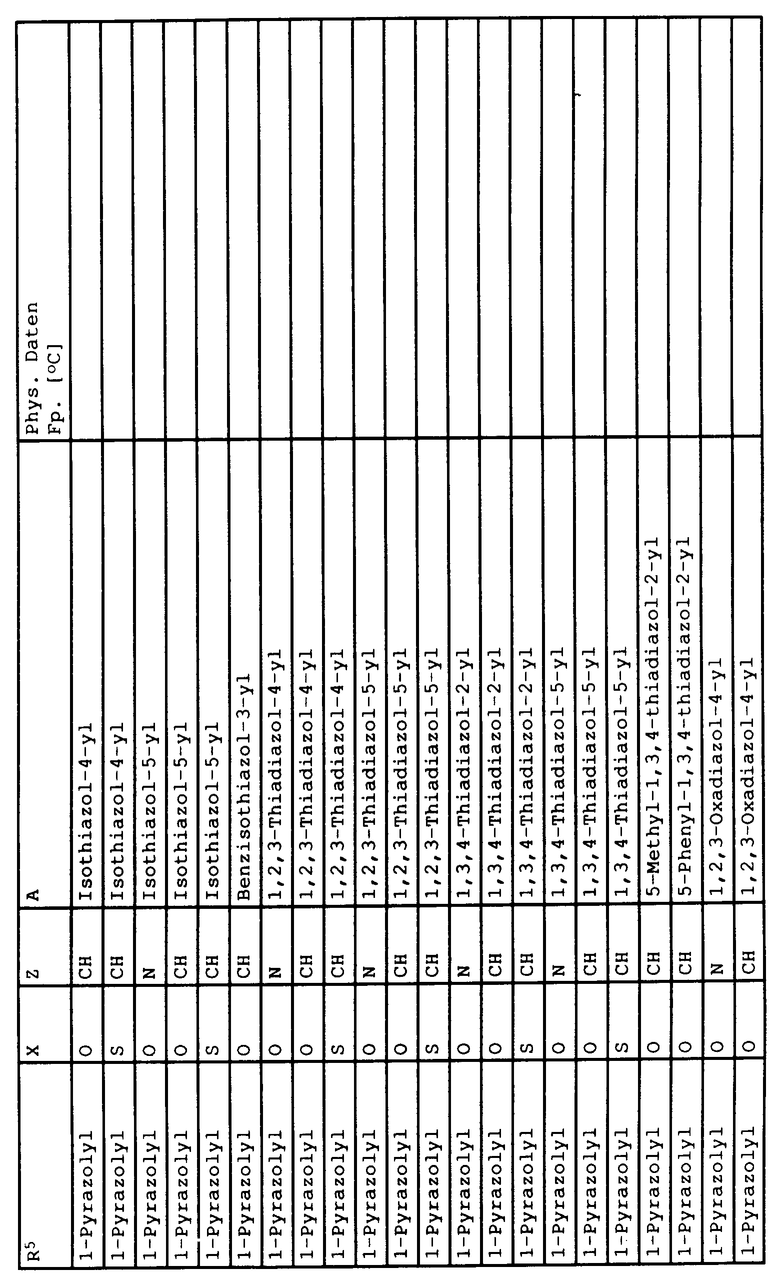

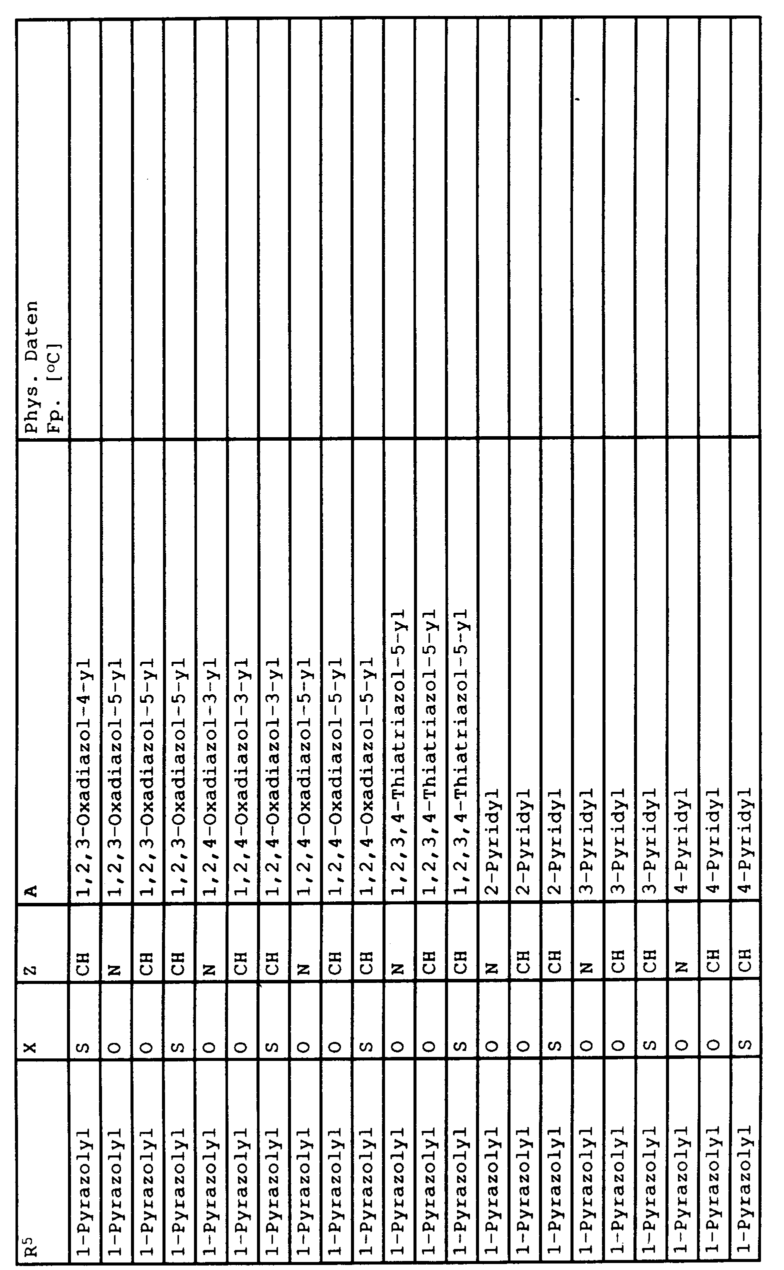

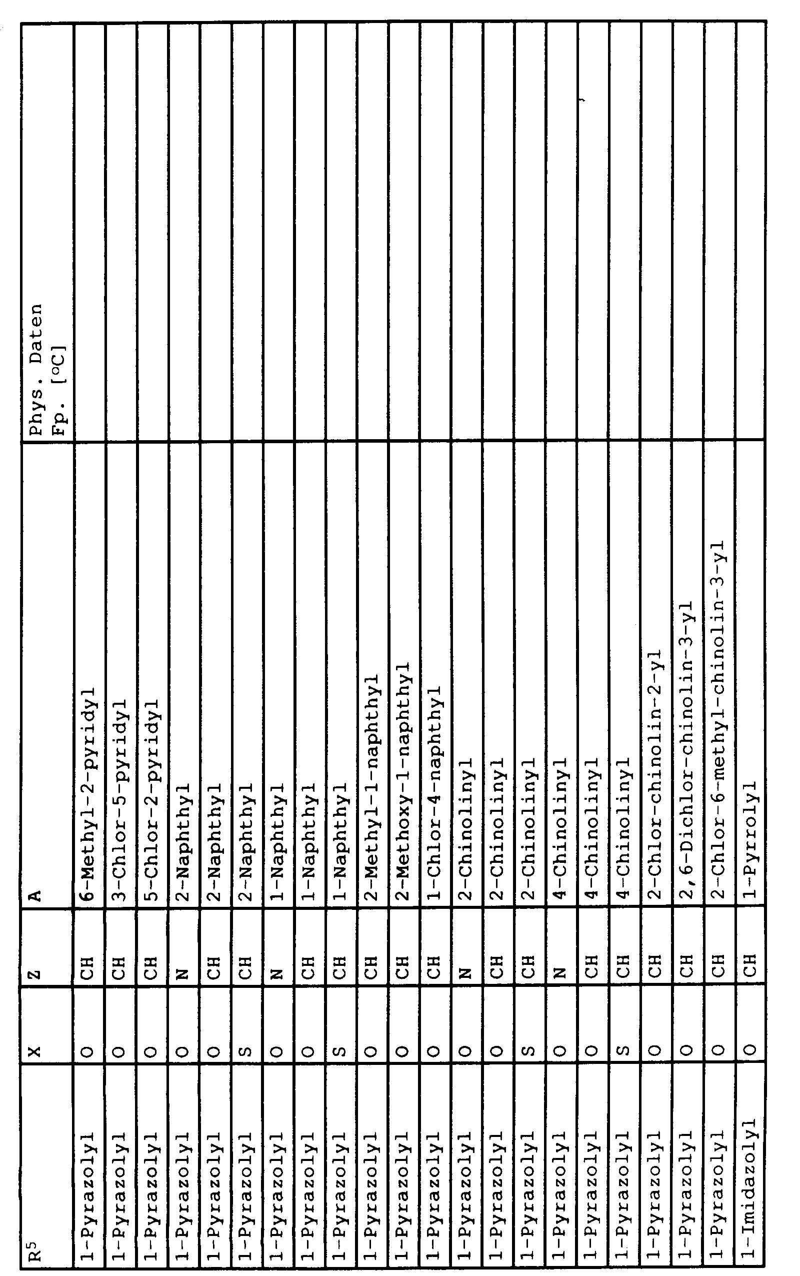

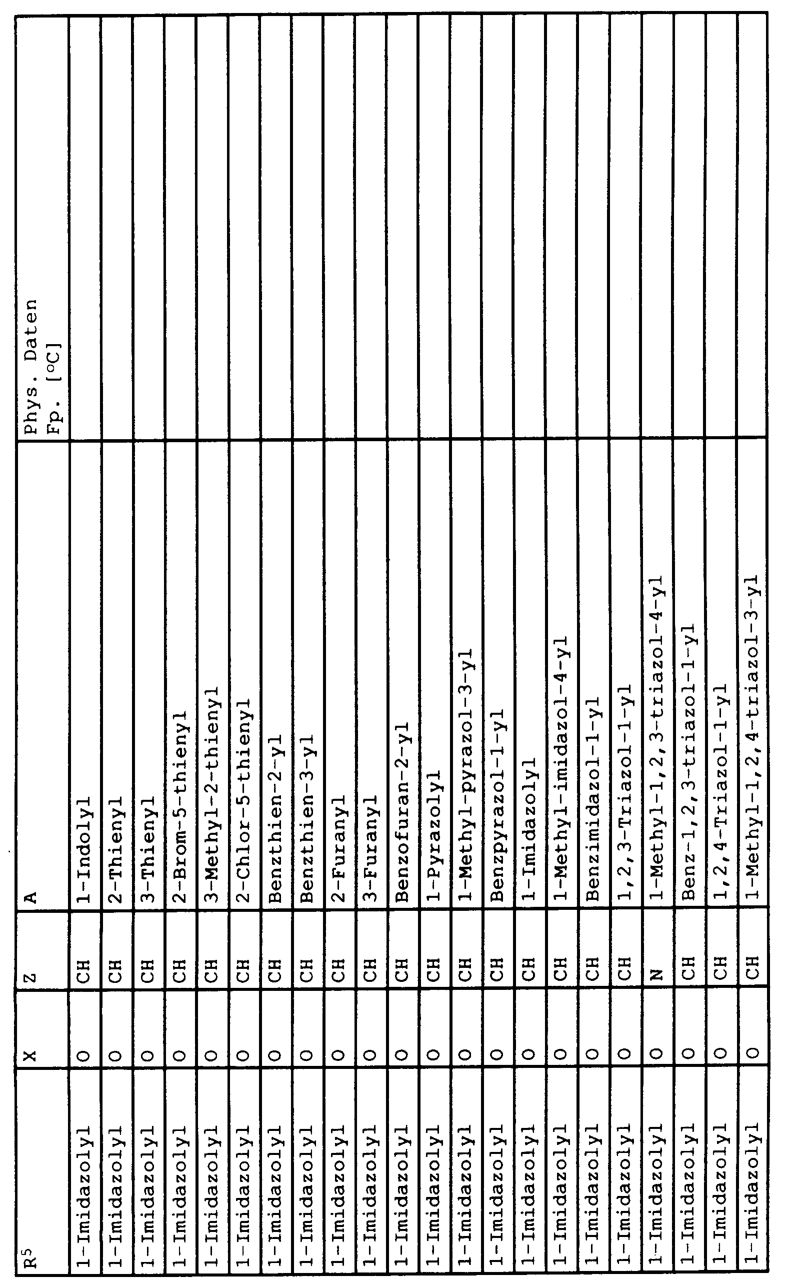

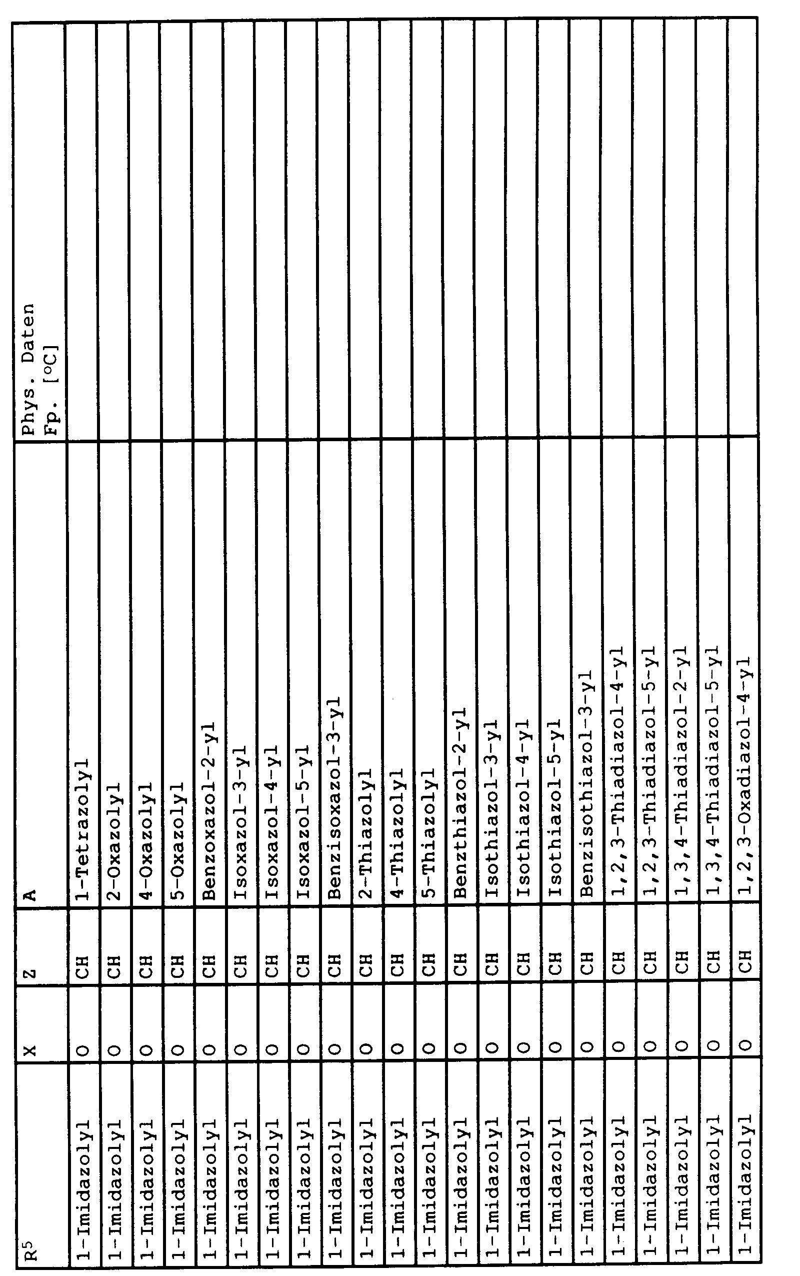

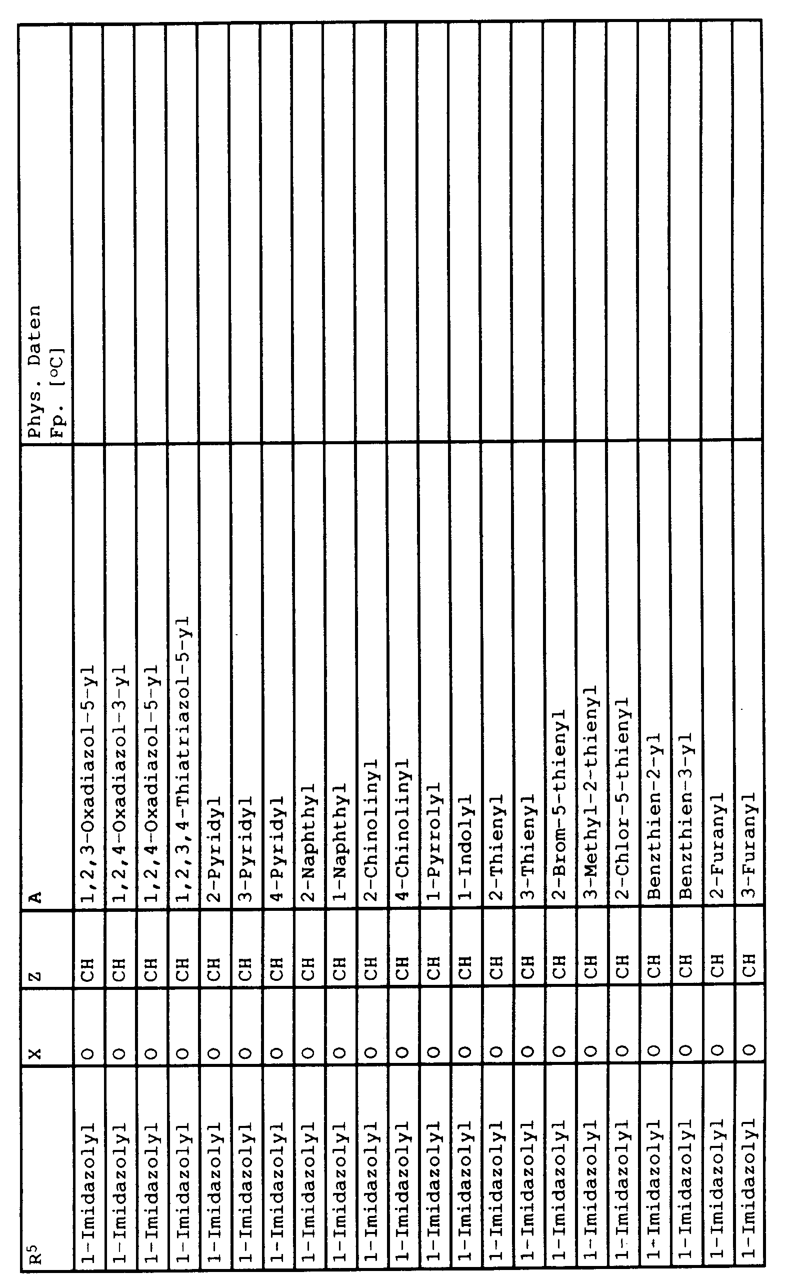

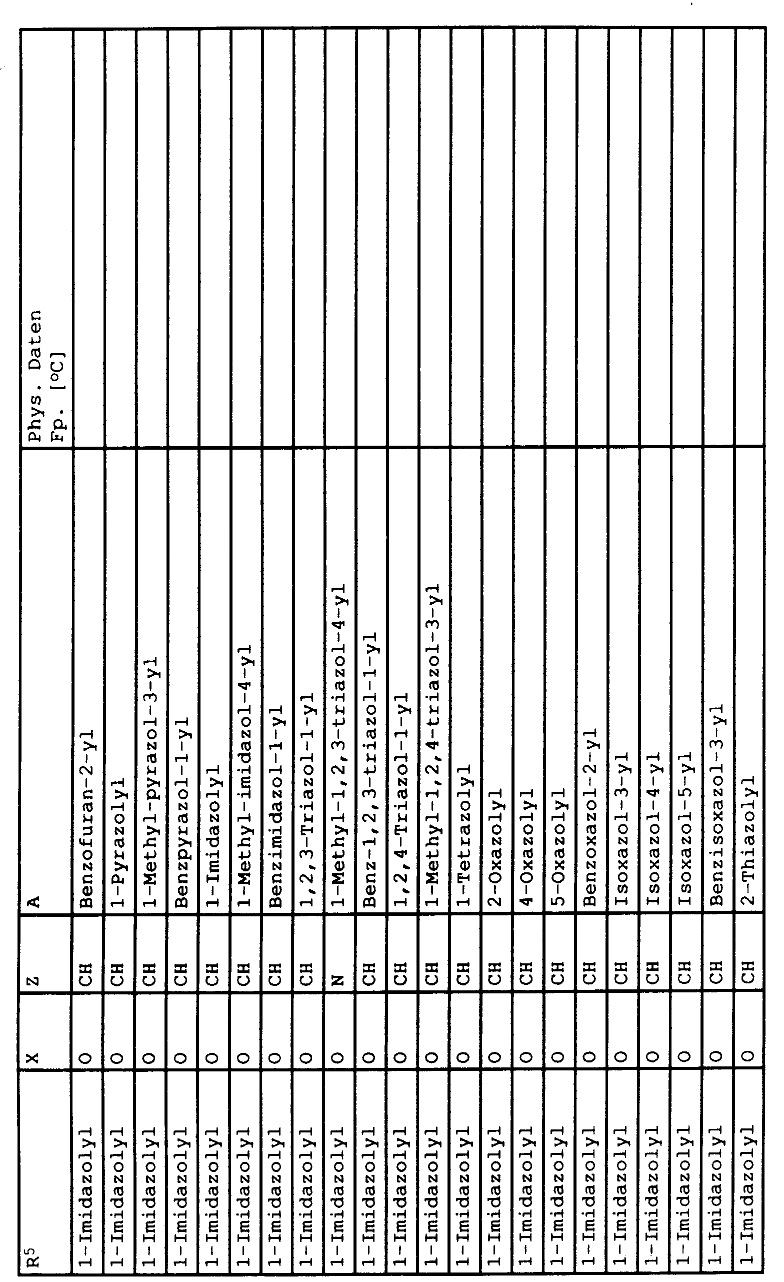

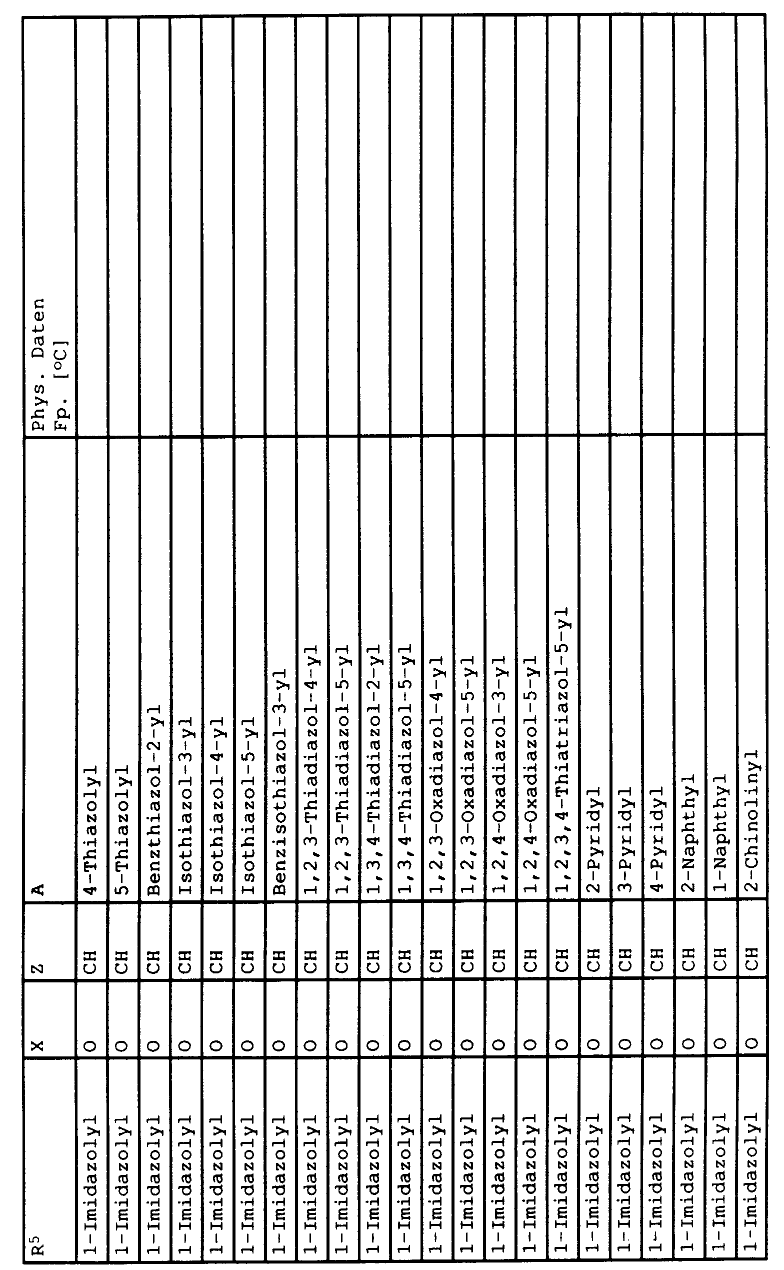

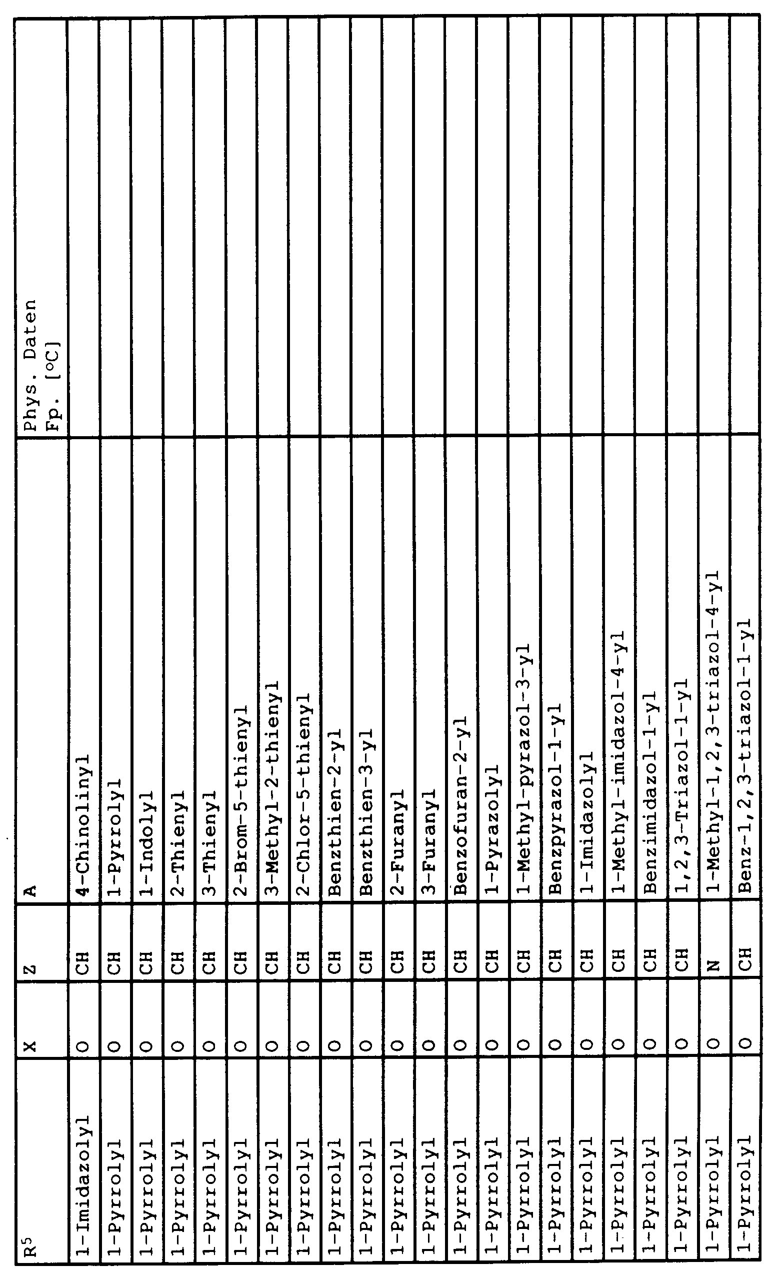

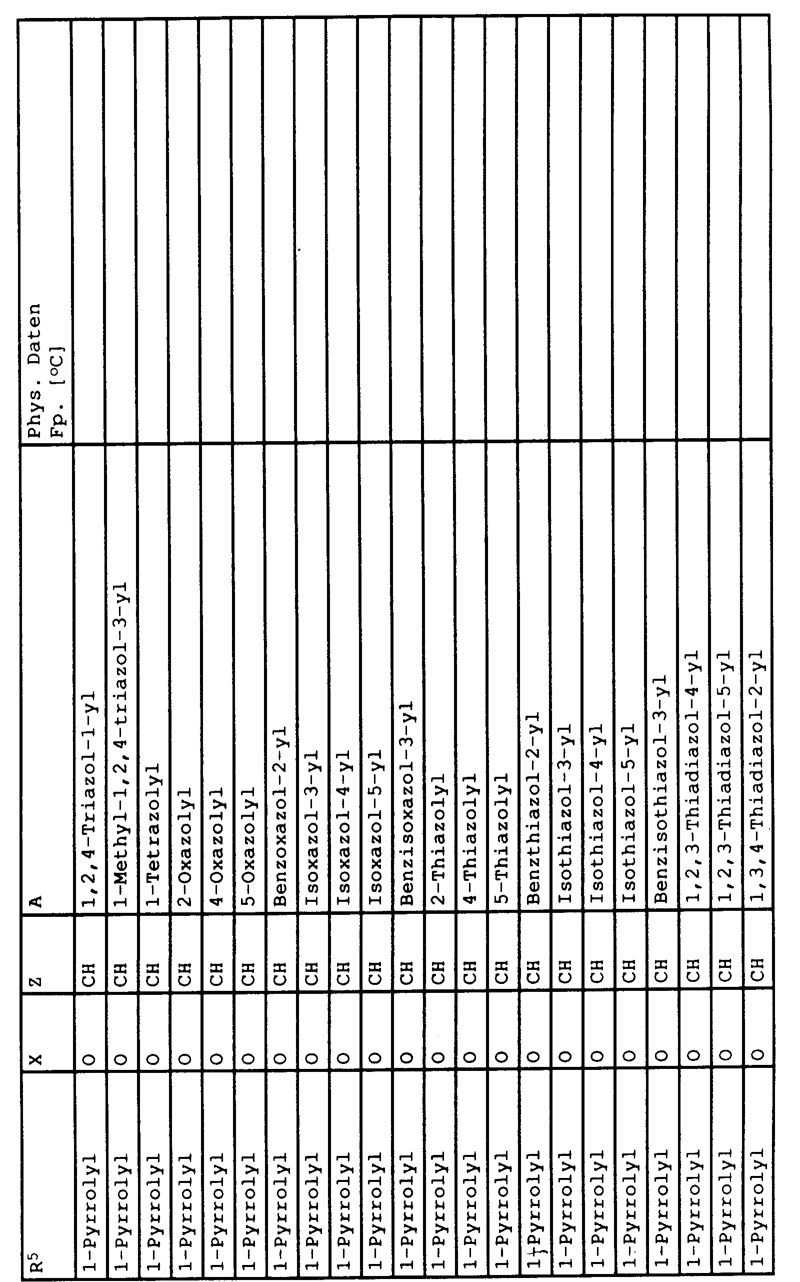

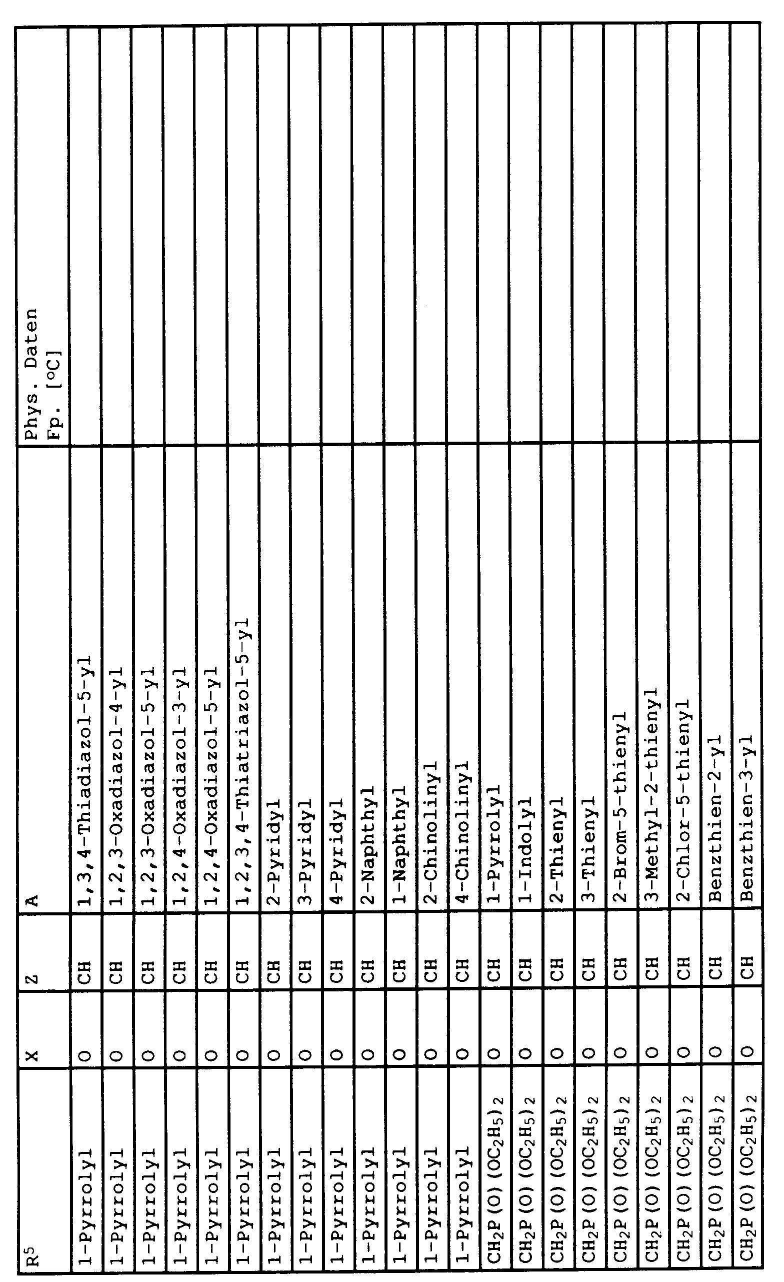

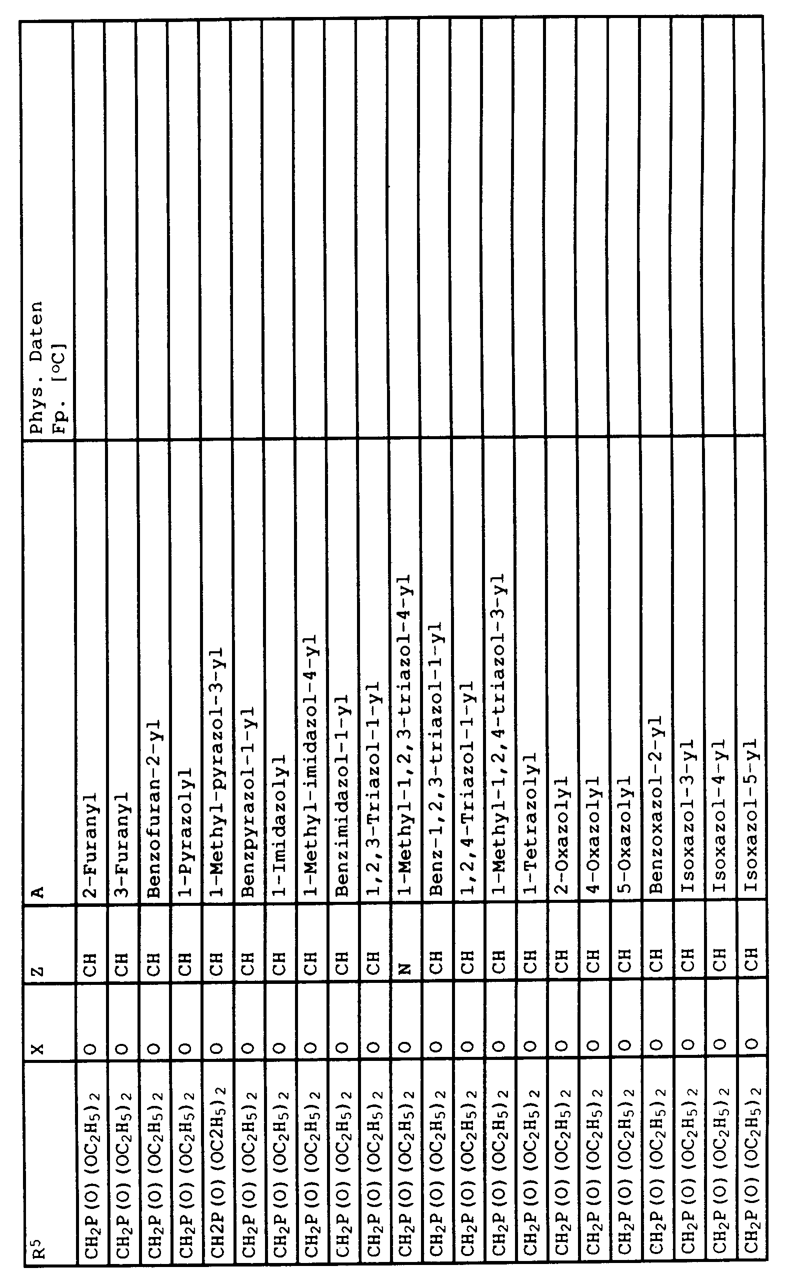

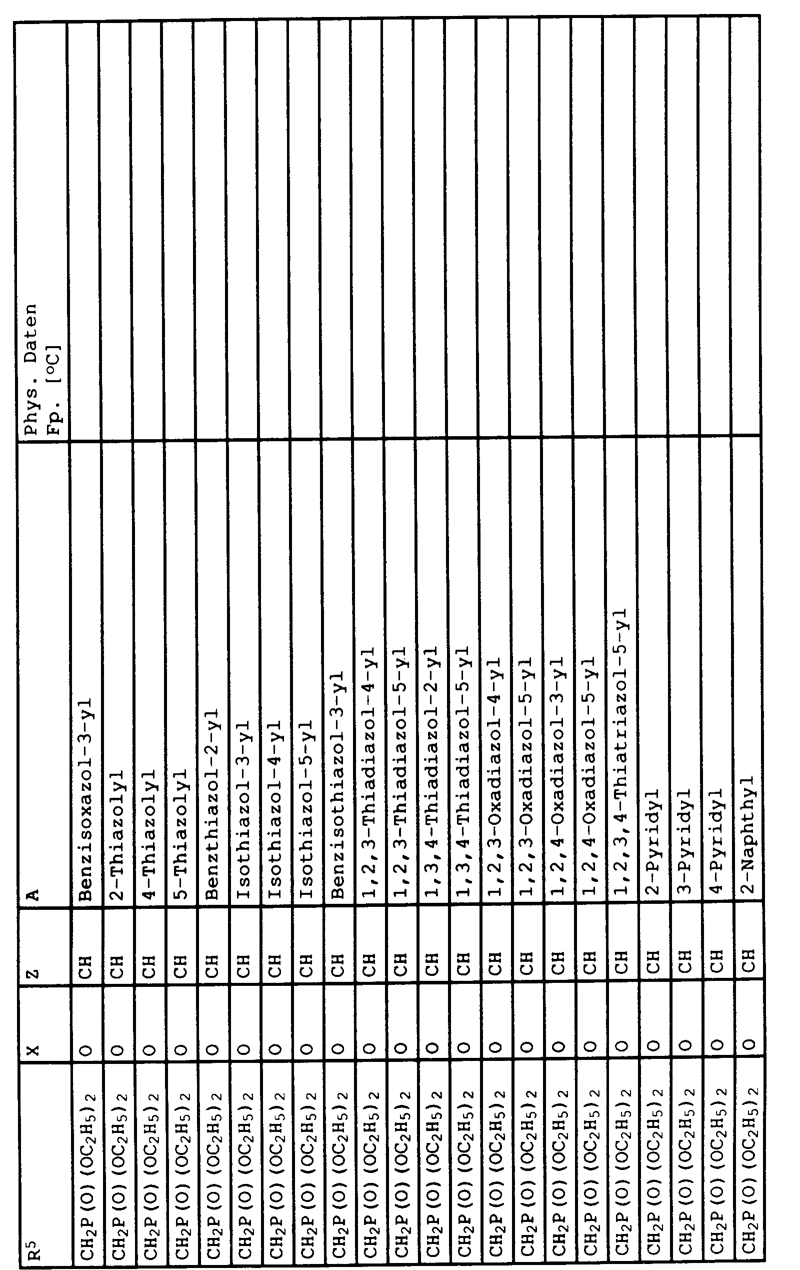

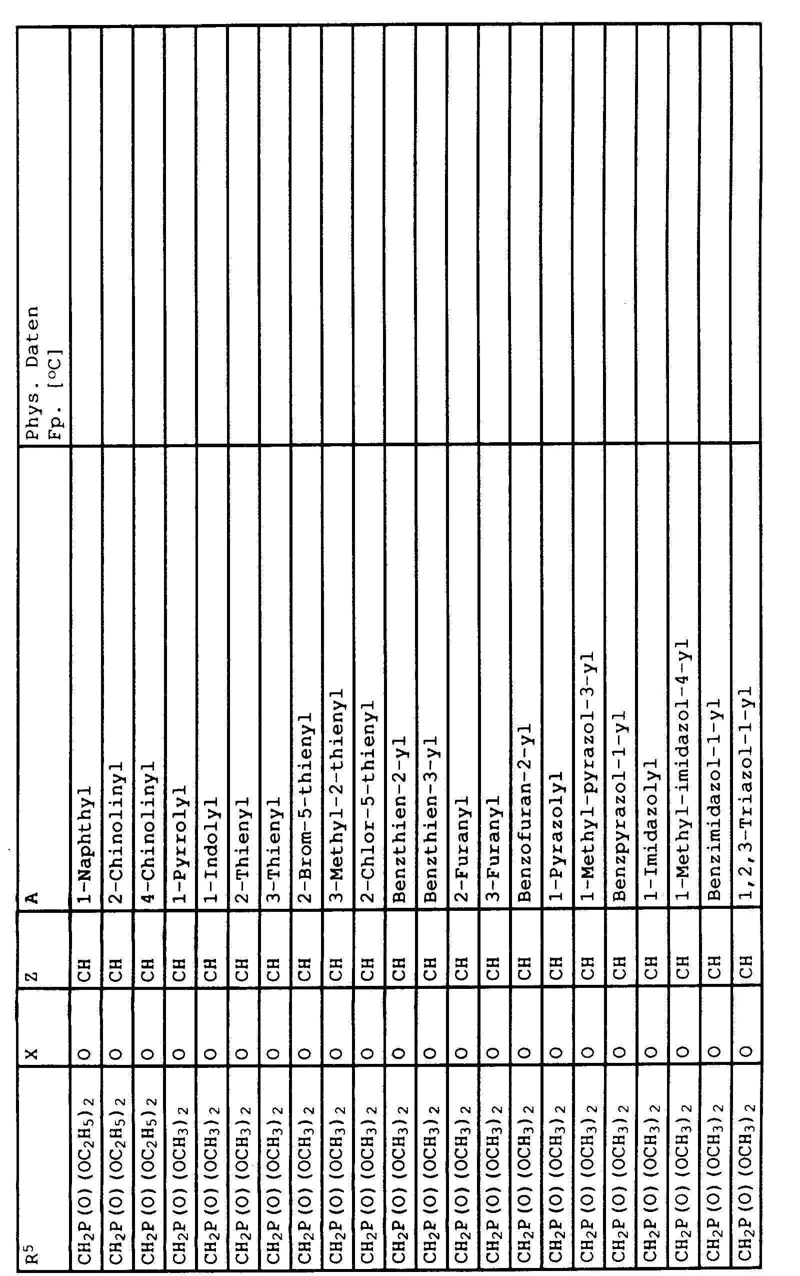

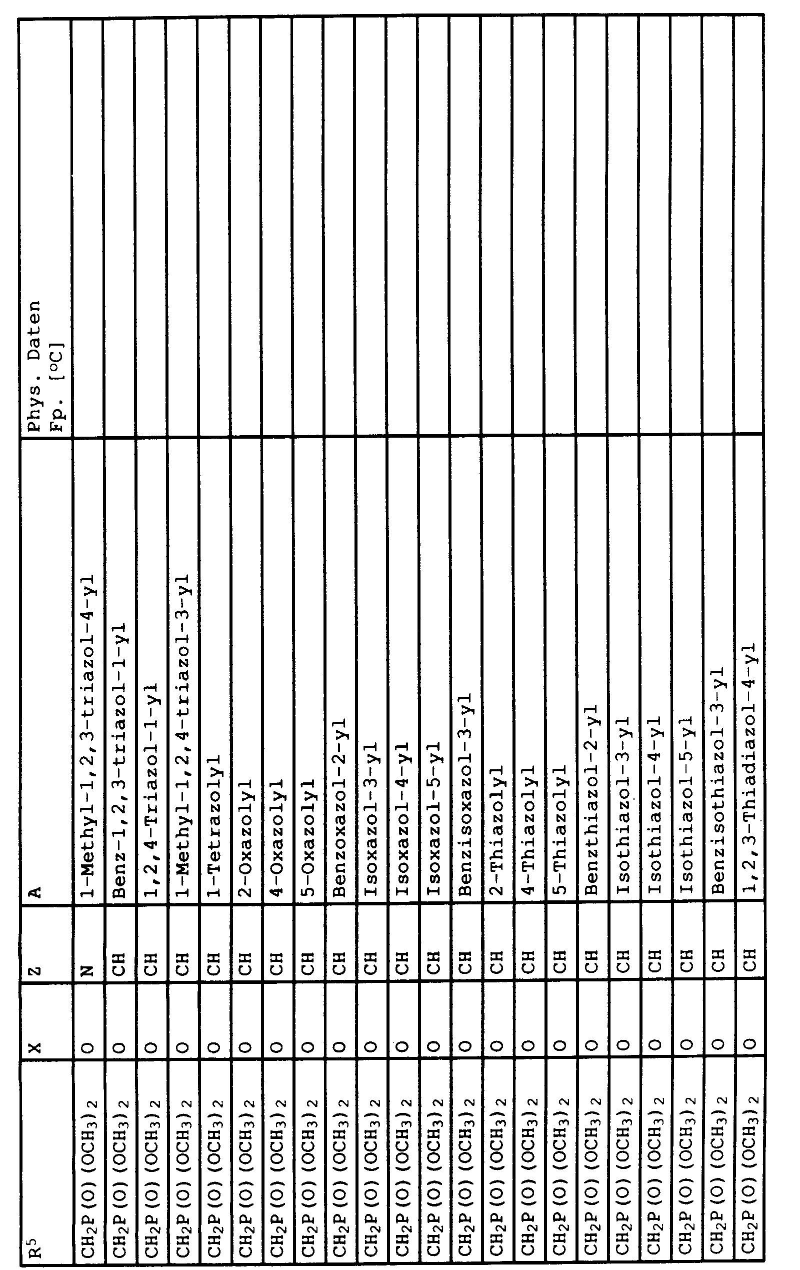

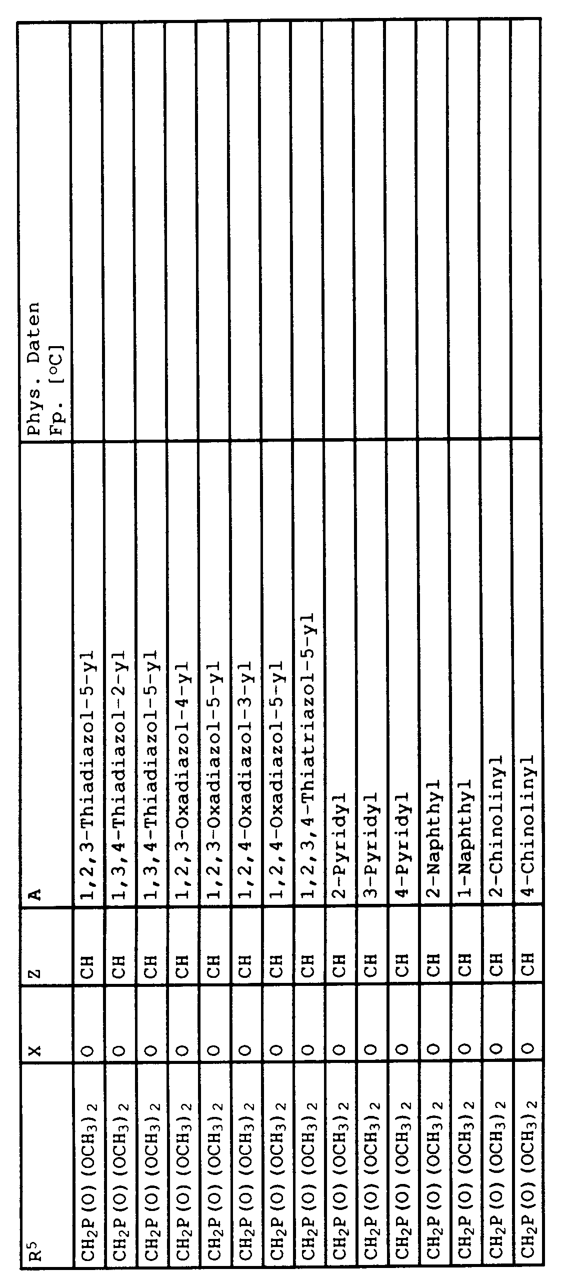

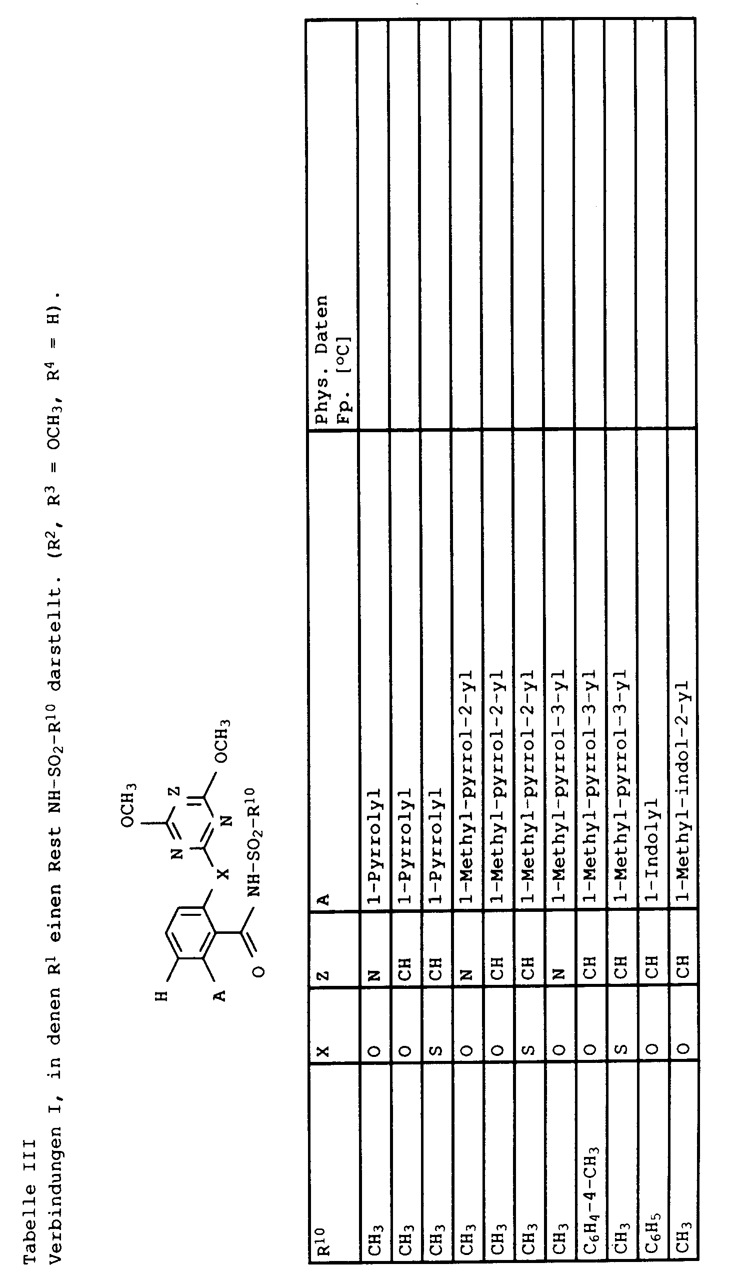

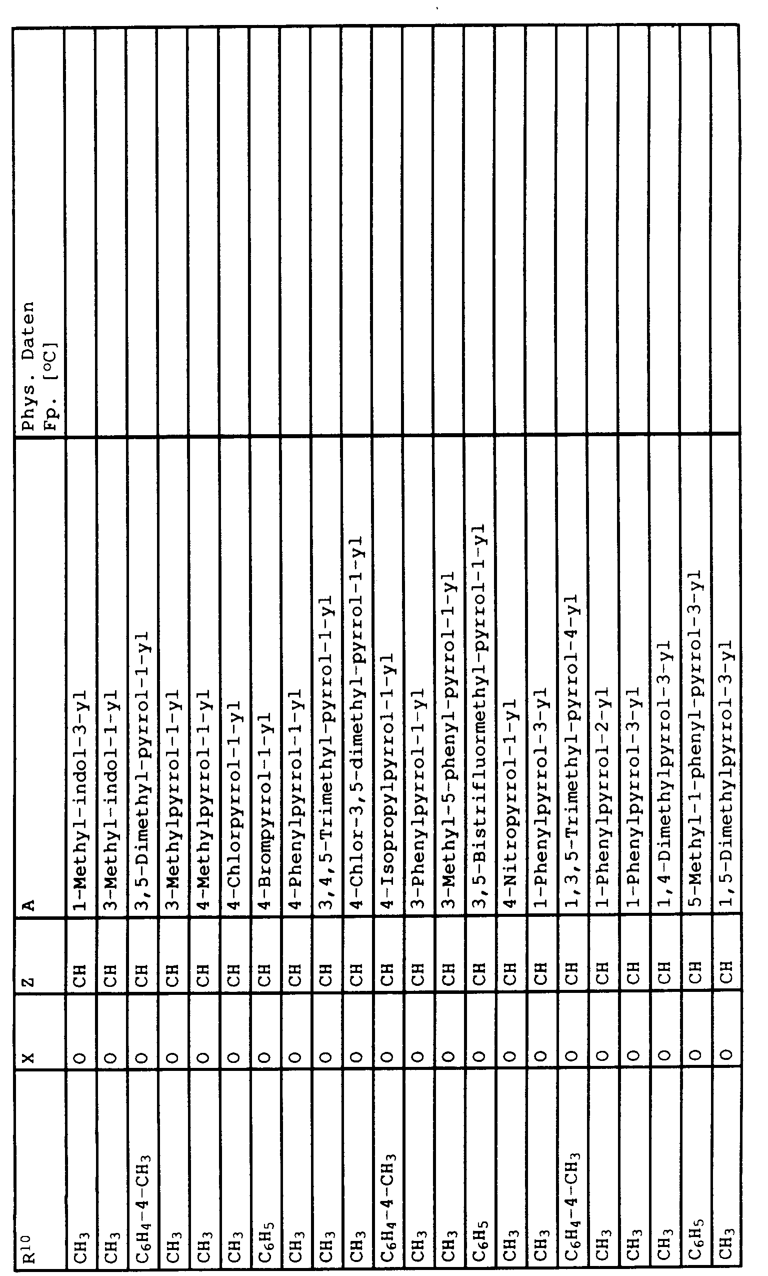

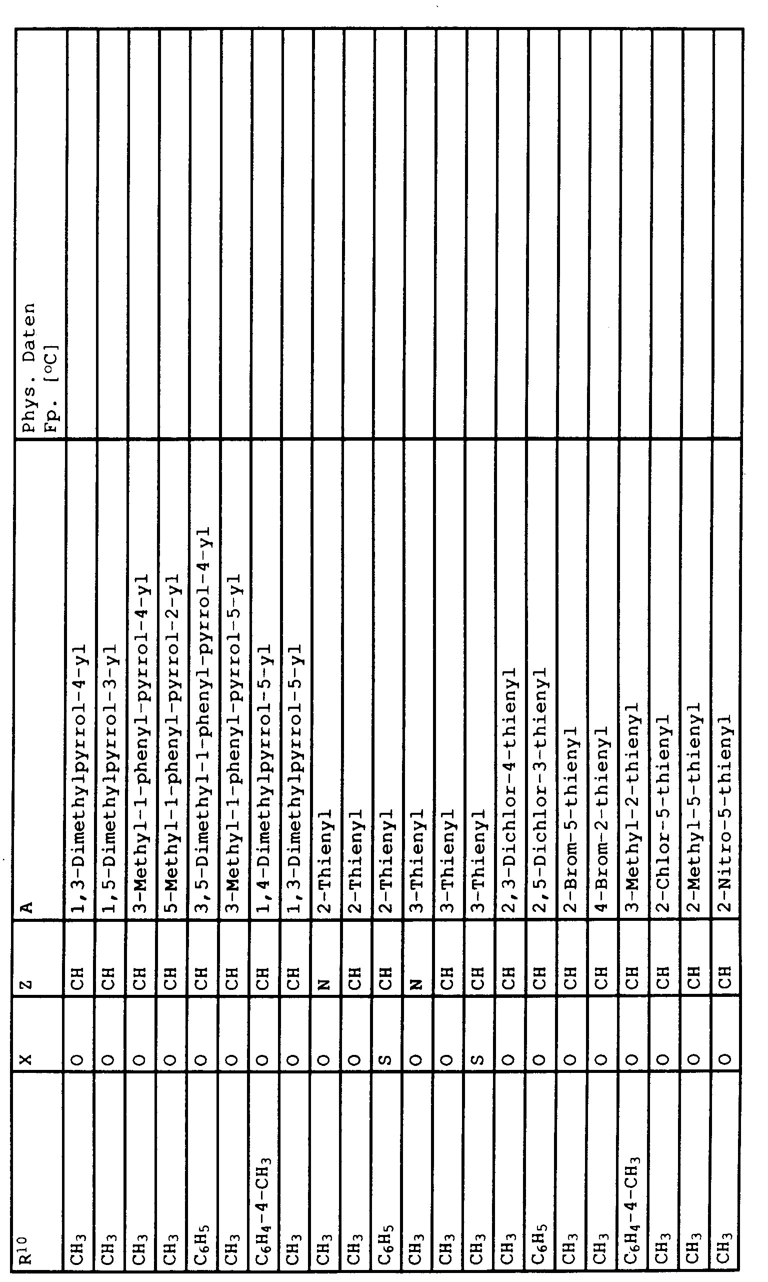

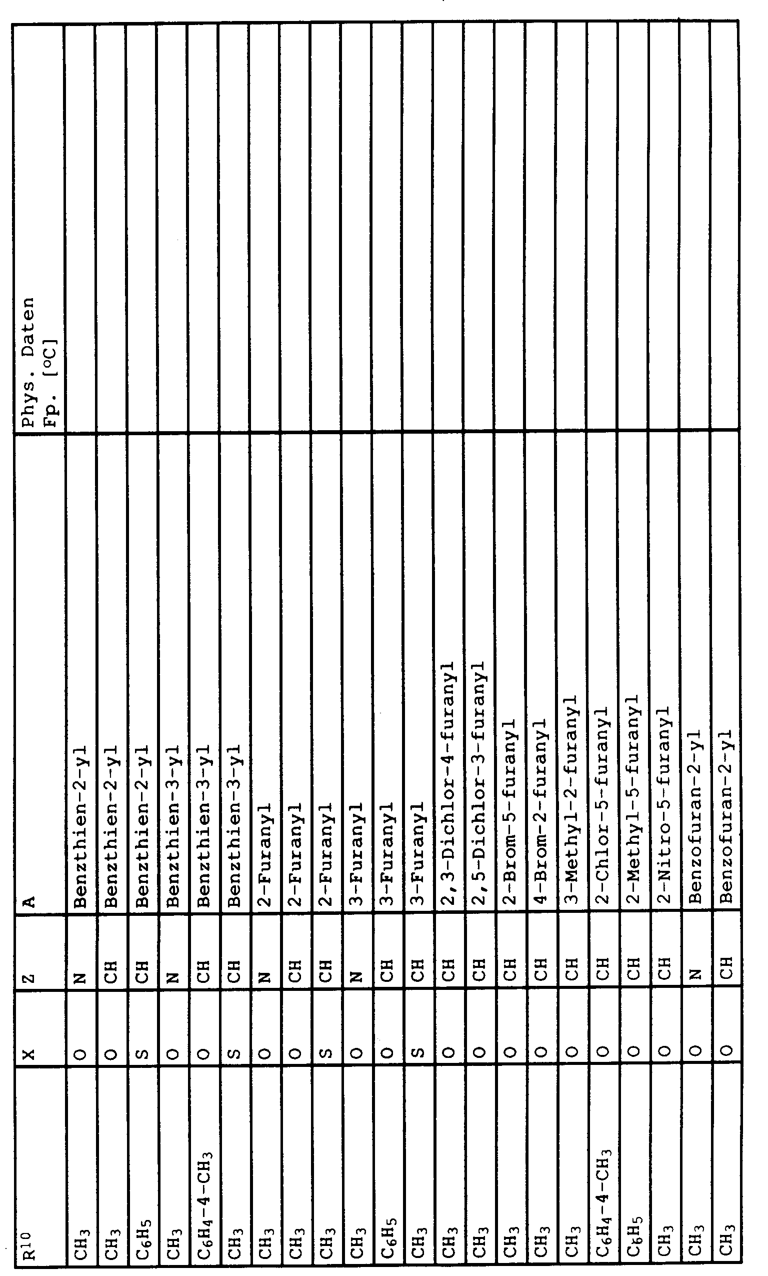

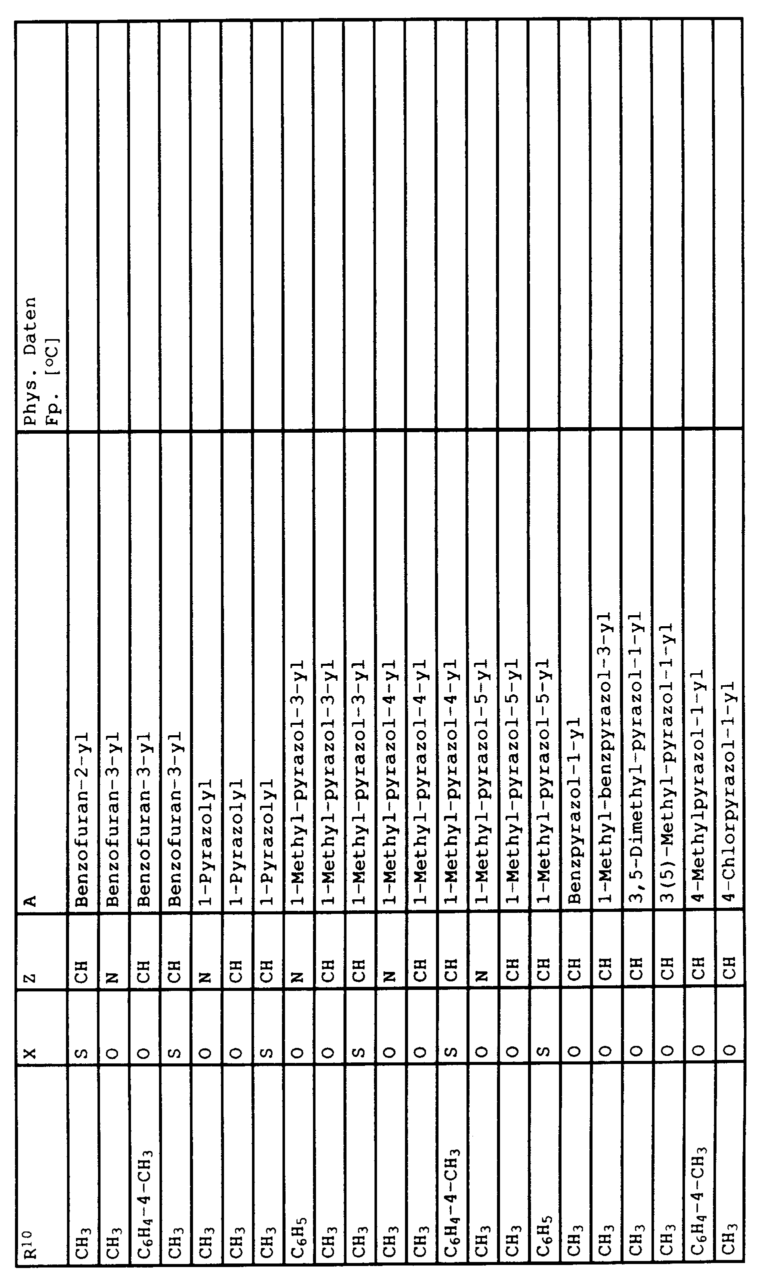

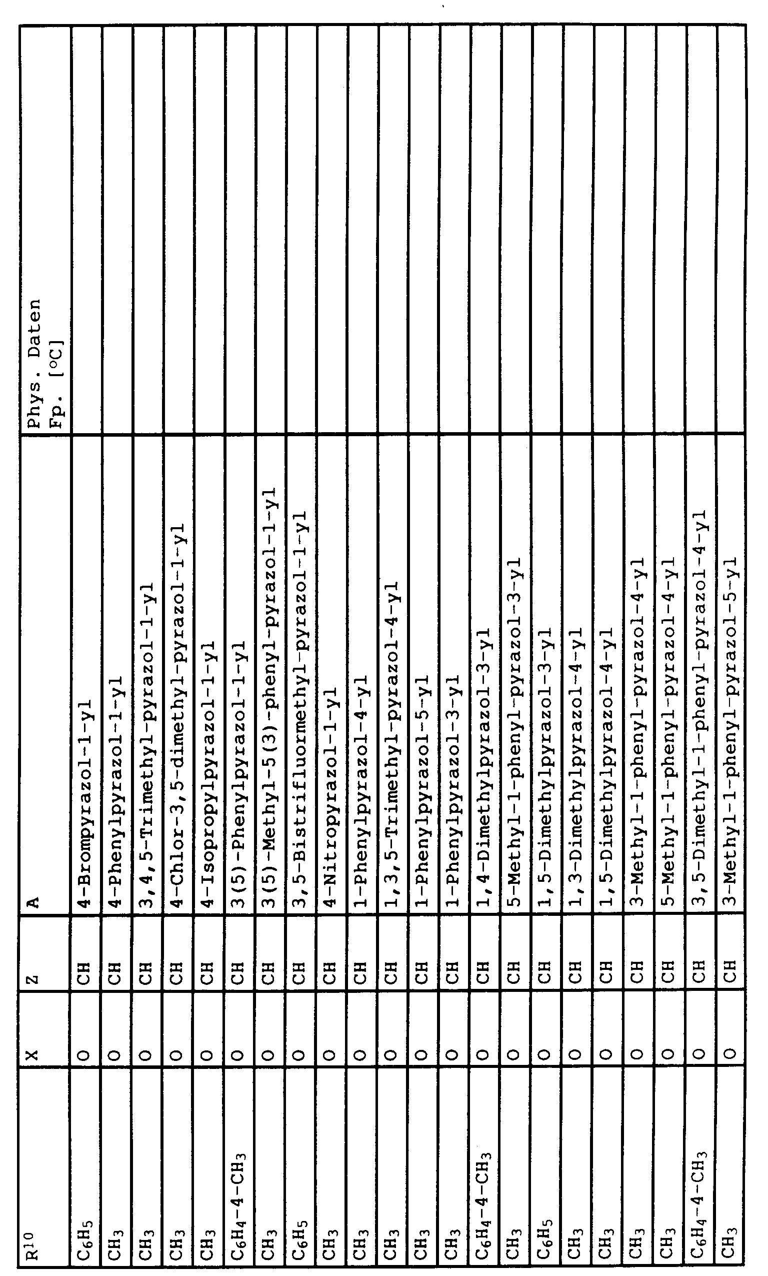

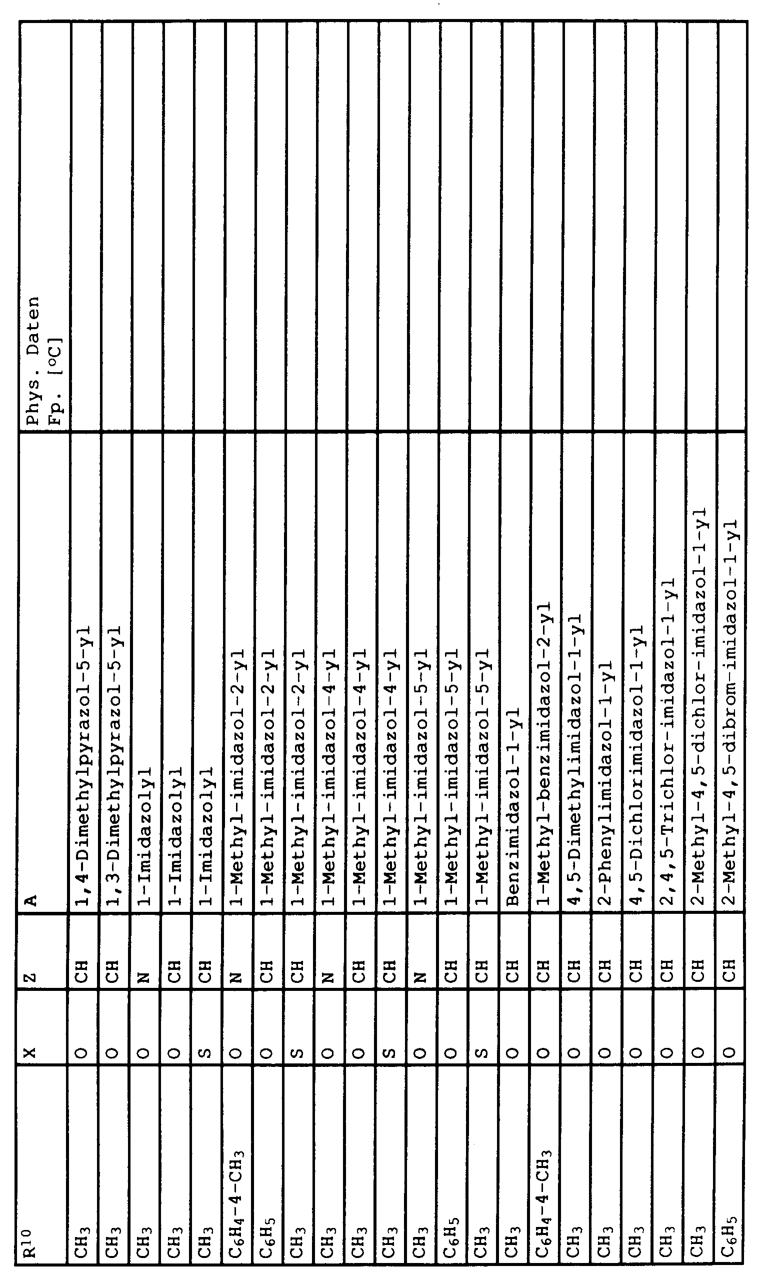

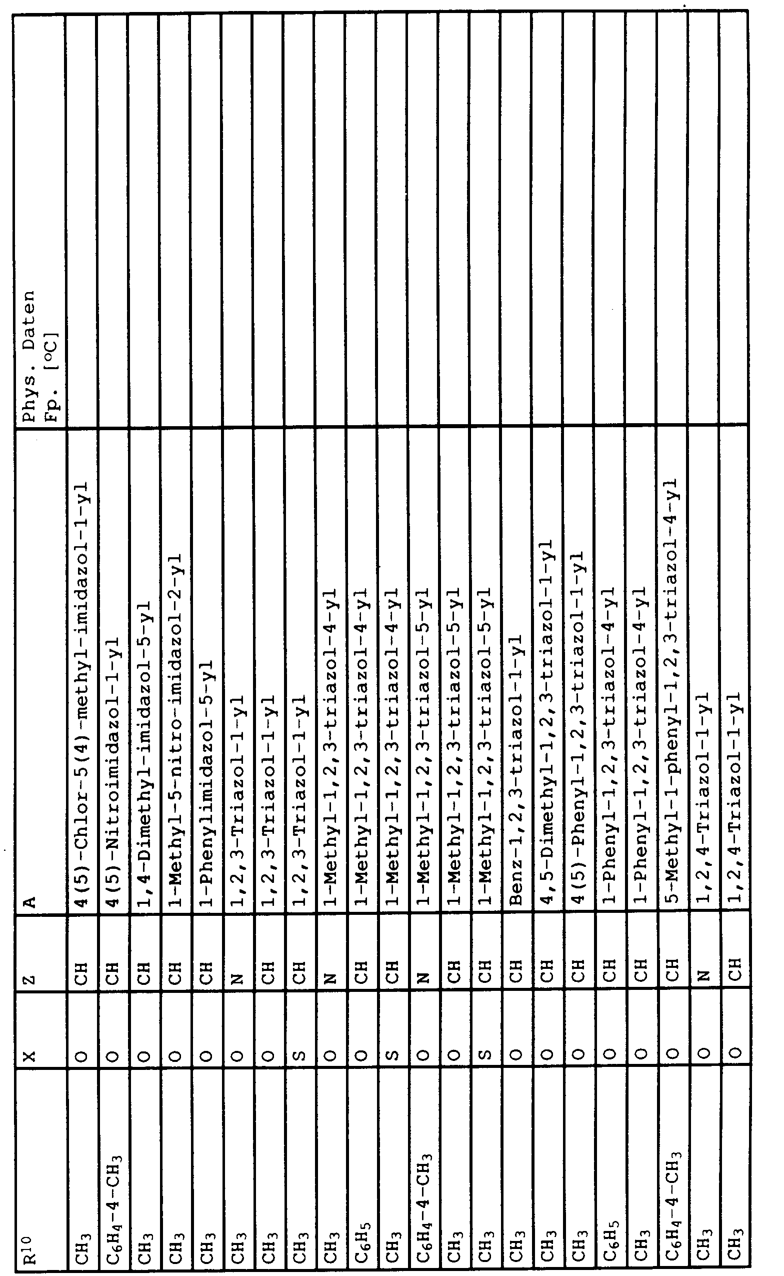

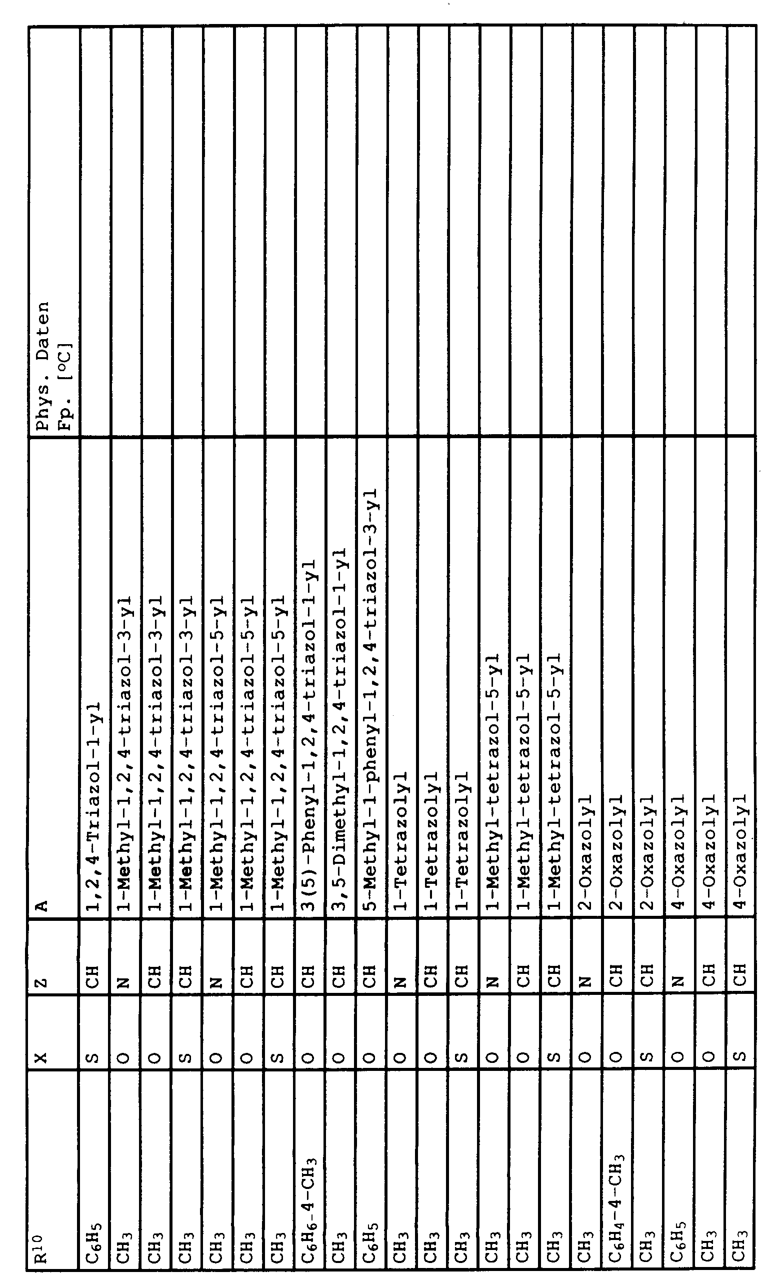

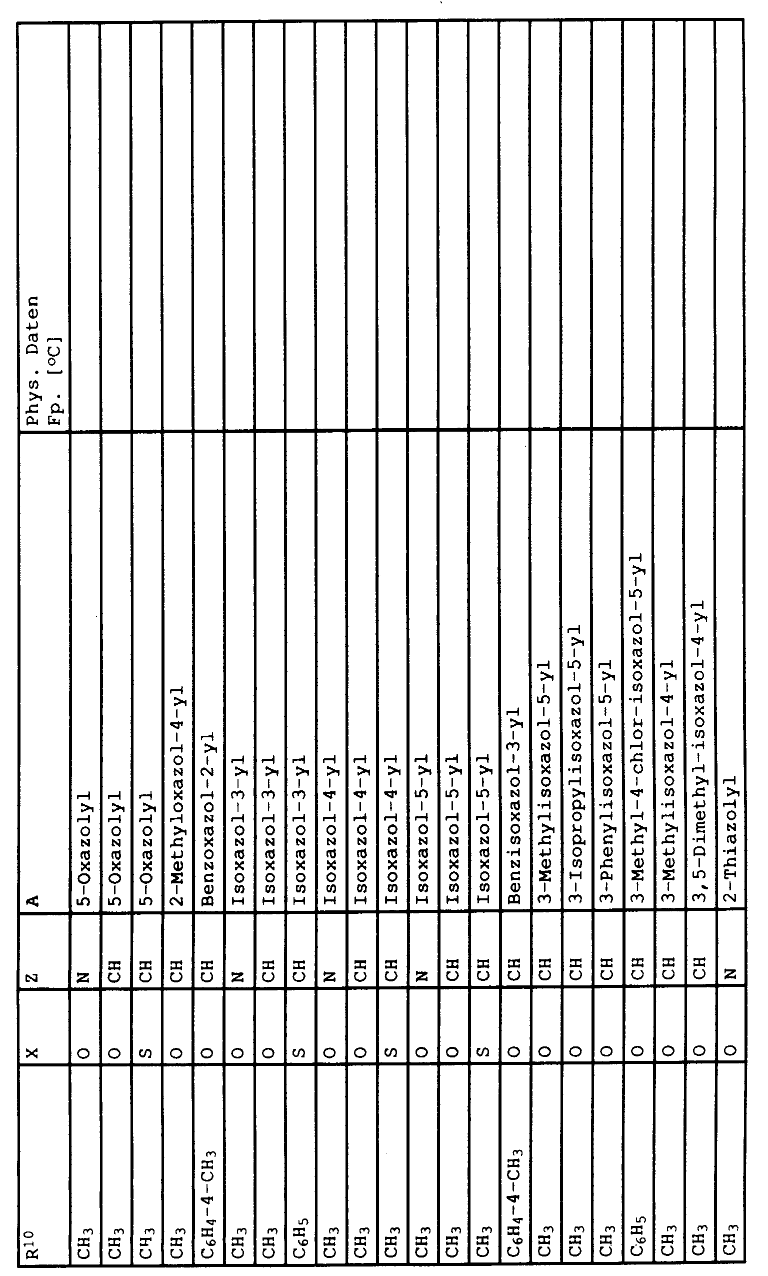

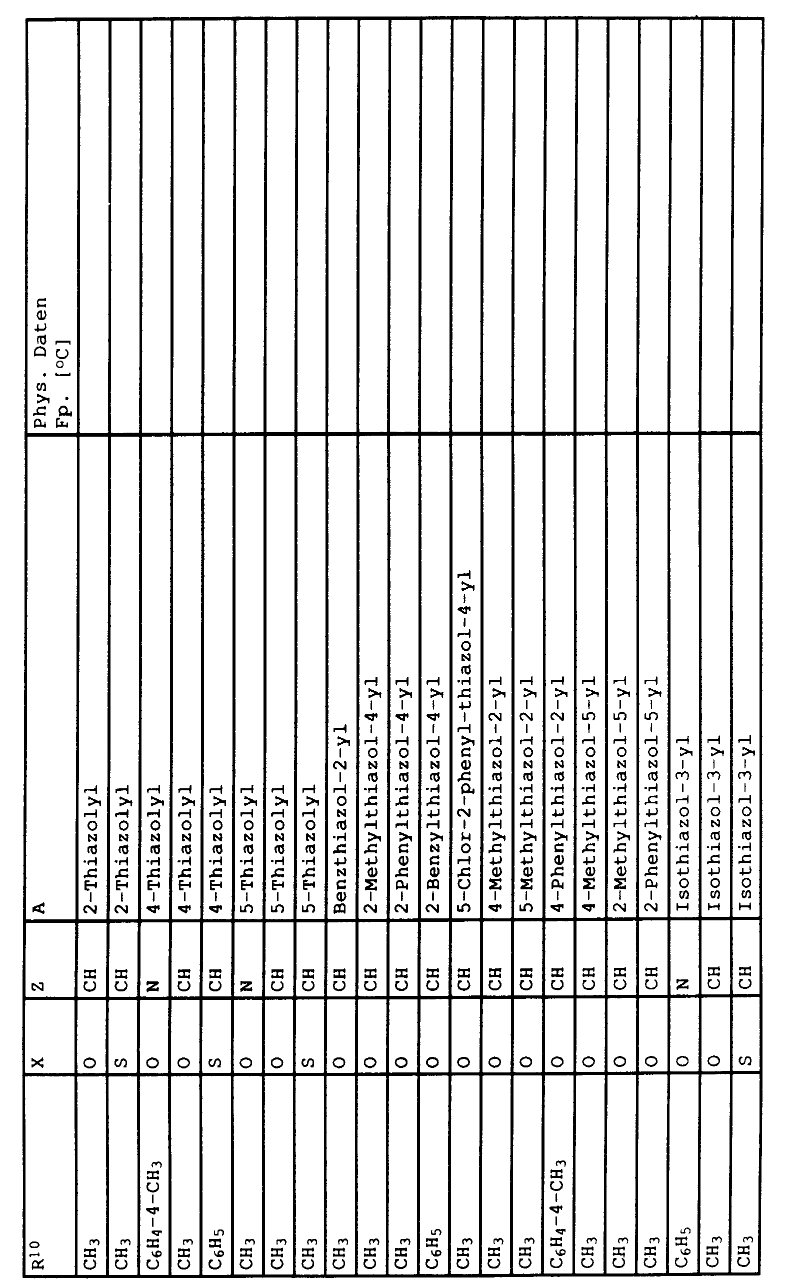

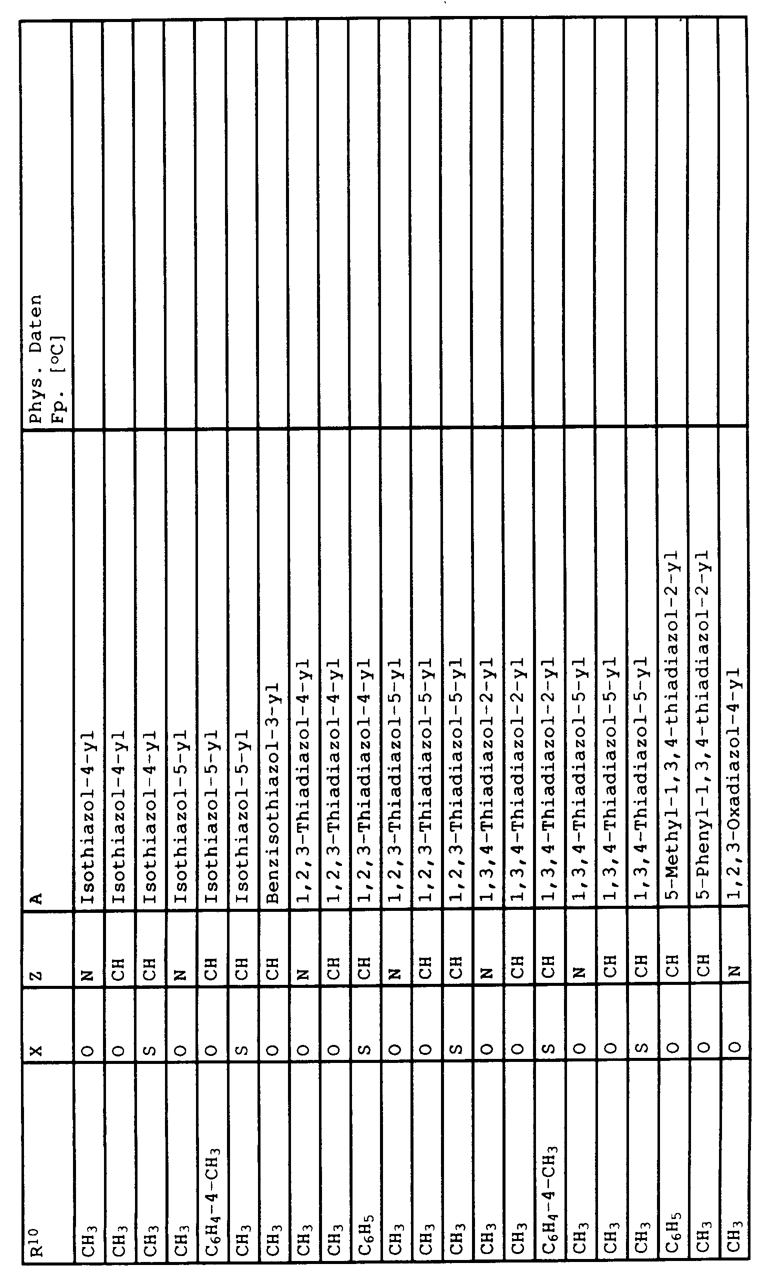

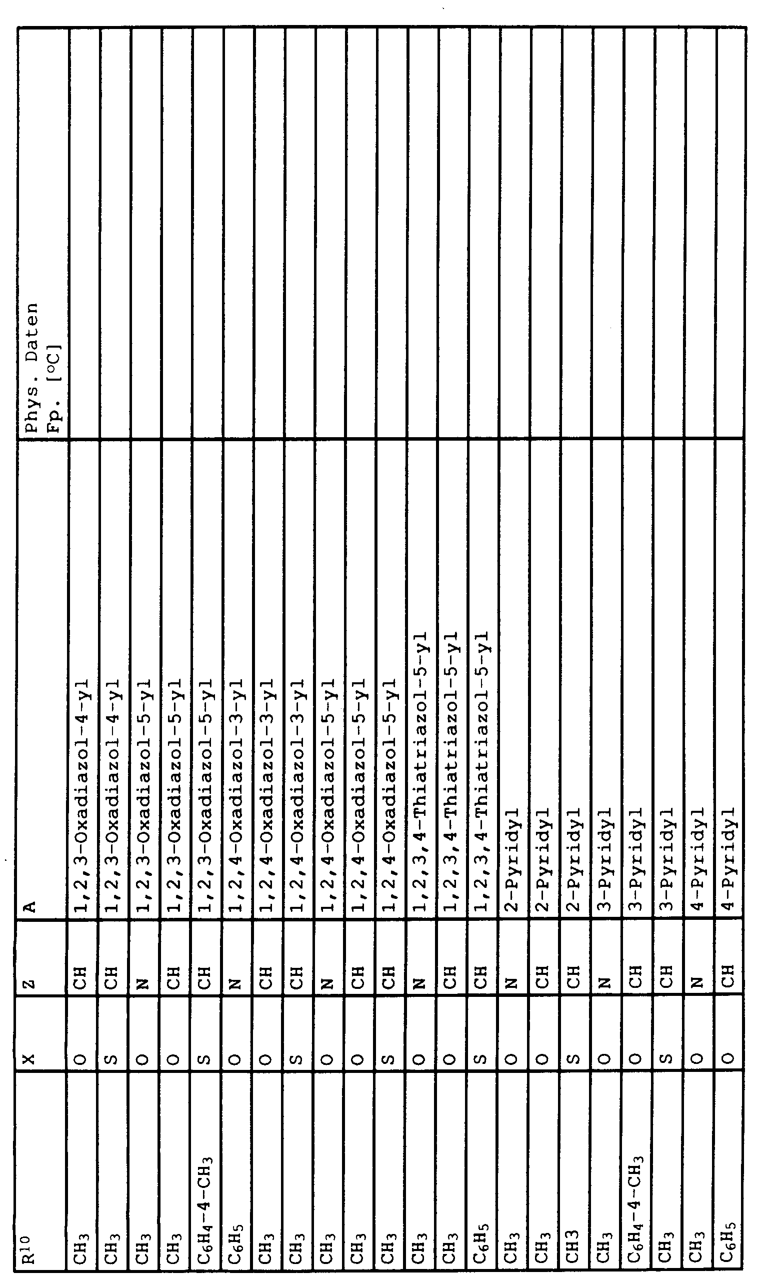

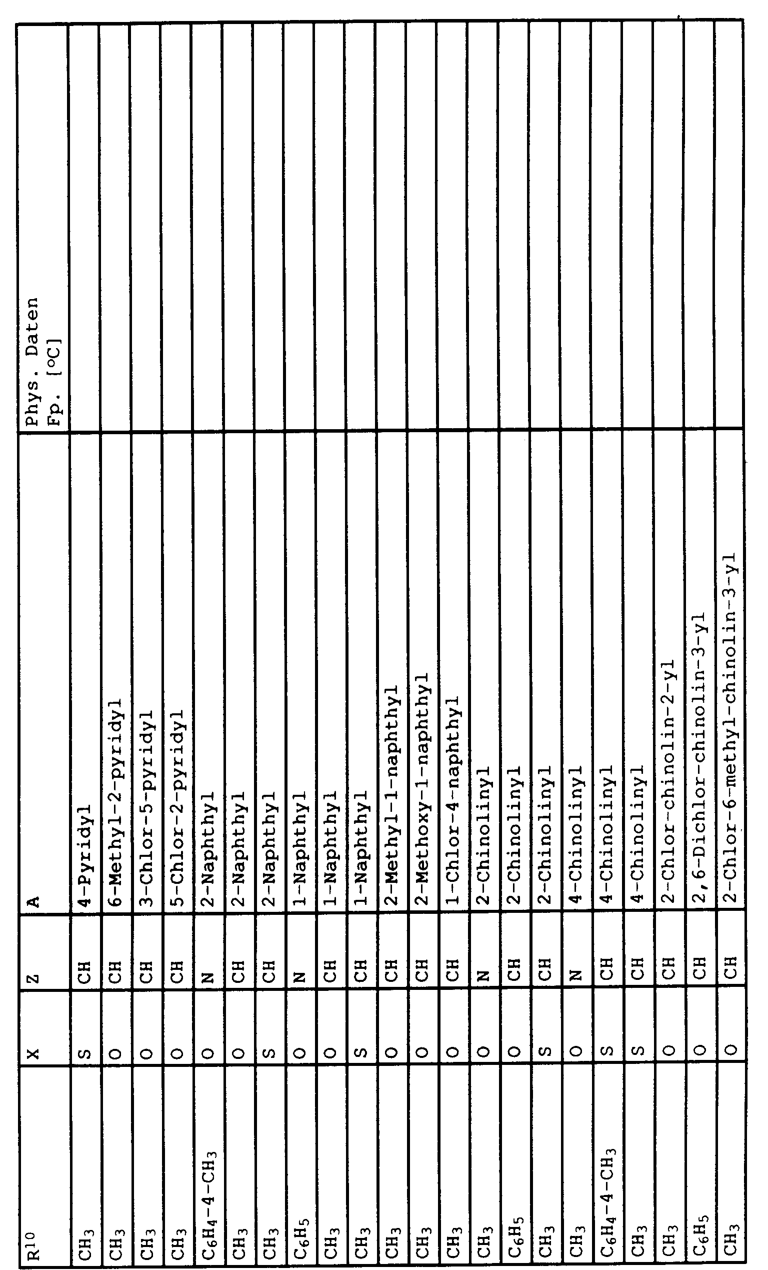

Die erfindungsgemäßen Verbindungen I bzw. die sie enthaltenden Mittel können z.B. in Form von direktversprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier-oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali- oder Ammoniumsalze von aromatischen Sulfonsäuren, z.B. Lignin-, Phenol-, Naphthalin- und Di-butyl-naphthalinsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl- und Tributylphenolpolyglykolether, Alkylarylpolyetheralkohole, Isotridecylalkohol, Fettalkohol-Ethylenoxid-Kondensate, ethoxyliertes Ricinusöl, Polyoxyethylenalkylether oder Polyoxypropylen alkylether, Laurylalkoholpolyglykoletheracetat, Sorbitester, Lignin-sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosemehl oder andere feste Trägerstoffe.

Die Formulierungen enthalten zwischen 0.1 und 95 Gew.%, vorzugsweise zwischen 0.5 und 90 Gew.% Wirkstoff. Die Wirkstoffe werden dabei in einer Reinheit von 90 % bis 100 %, vorzugsweise 95 % bis 100 %, (nach NMR-Spektrum) eingesetzt.

Die erfindungsgemäßen Verbindungen I können beispielsweise wie folgt formuliert werden:
I. Man vermischt 90 Gew.-Teile der Verbindung 1.001 mit 10 Gew.-Teilen N-Methyl-α-pyrrolidon und erhält eine Lösung, die zur Anwendung in Form kleinster Tropfen geeignet ist.
II. 20 Gew.-Teile der Verbindung 1.001 werden in einer Mischung gelöst, die aus 80 Gew.-Teilen Xylol, 10 Gew.-Teilen des Anlagerungsproduktes von 8 bis 10 mol Ethylenoxid an 1 mol Ölsäure-N-monoethanolamid, 5 Gew.-Teilen Calciumsalz der Dodecylbenzolsulfonsäure und 5 Gew.-Teilen des Anlagerungsproduktes von 40 mol Ethylenoxid an 1 mol Ricinusöl besteht. Durch Ausgießen und feines Verteilen der Lösung in 100000 Gew.-Teilen Wasser erhält man eine wäßrige Dispersion, die 0.02 Gew.% des Wirkstoffs enthält.
III. 20 Gew.-Teile der Verbindung 1.002 werden in einer Mischung gelöst, die aus 40 Gew.-Teilen Cyclohexanon, 30 Gew.-Teilen Isobutanol, 20 Gew.-Teilen des Anlagerungsproduktes von 7 mol Ethylenoxid an 1 mol Isooctylphenol und 10 Gew.-Teilen des Anlagerungsproduktes von 40 mol Ethylenoxid 1 mol Ricinusöl besteht. Durch Eingießen und feines Verteilen der Lösung in 100000 Gewichtsteilen Wasser erhält man eine wäßrige Dispersion, die 0.02 Gew.% des Wirkstoffs enthält.
IV. 20 Gew.-Teile des Wirkstoffs 1.002 werden in einer Mischung gelöst, die aus 25 Gew.-Teilen Cyclohexanon, 65 Gew.-Teilen einer Mineralölfraktion vom Siedepunkt 210 bis 280°C und 10 Gew.-Teilen des Anlagerungsproduktes von 40 mol Ethylenoxid 1 mol Ricinusöl besteht. Durch Eingießen und feines Verteilen der Lösung in 100000 Gew.-Teilen Wasser erhält man eine wäßrige Dispersion, die 0.02 Gew.% des Wirkstoffs enthält.
V. 20 Gew.-Teile des Wirkstoffs Nr. 1.001 werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teile Wasser erhält man eine Spritzbrühe, die 0.1 Gew.% des Wirkstoffs enthält.
VI. 3 Gew.-Teile des Wirkstoffs Nr. 1.001 werden mit 97 Gew.-Teilen feinteiligem Kaolin vermischt. Man erhält auf diese Weise ein Stäubemittel, das 3 Gew.-% des Wirkstoffs enthält.
VII. 30 Gew.-Teile des Wirkstoffs Nr. 1.002 werden mit einer Mischung aus 92 Gew.-Teilen pulverförmigem Kieselsäuregel 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
VIII. 20 Gew.-Teile des Wirkstoffs Nr. 1.002 werden mit 2 Gew.-Teilen Calciumsalz der Dodecylbenzolsulfonsäure, 8 Gew.-Teilen Fettalkohol-polyglykolether, 2 Gew.-Teilen Natriumsalz eines Phenol-Harnstoff-Formaldehyd-Kondensates und 68 Gew.-Teilen eines paraffinischen Mineralöls innig vermischt. Man erhält eine stabile ölige Dispersion.

Die Applikation der herbiziden und wachstumsregulierenden Mittel bzw. der Wirkstoffe kann im Vorauflauf- oder Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an herbizidem Wirkstoff betragen je nach Bekämpfungsziel, Jahreszeit, Zielpflanzen und Wachstumsstadien 0.001 bis 3.0, vorzugsweise 0.005 bis 0.5 kg/ha aktive Substanz (a.S.).

Die wachstumsregulierend wirksamen Salicylsäurederivate der Formel I können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt ab vor allem
a) von der Pflanzenart und -sorte,
b) vom Zeitpunkt der Applikation,
c) von dem Applikationsort und -verfahren (z.B. Samenbeize, Bodenbehandlung, Blattapplikation oder Stamminjektion bei Bäumen),
d) von klimatischen Faktoren,
e) von der Bodenbeschaffenheit,
f) von der Formulierung bzw. Anwendungsform des Wirkstoffs und schließlich
g) von der angewendeten Konzentration der aktiven Substanz.

Die erfindungsgemäß zu verwendenden Wirkstoffe der Formel I können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0.001 bis 50 g, vorzugsweise 0.01 bis 10 g, je Kilogramm Saatgut benötigt.

Für die Blatt- und Bodenbehandlung sind im allgemeinen Gaben von 0.001 bis 10 kg/ha, bevorzugt 0.01 bis 3 kg/ha, insbesondere 0.01 bis 0.5 kg/ha als ausreichend zu betrachten.

Die Applikation der herbiziden Mittel bzw. der Wirkstoffe kann im Vorauflauf- oder Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadien 0.001 bis 3.0, vorzugsweise 0.15 bis 1.0 kg/ha aktive Substanz (a.S.).

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

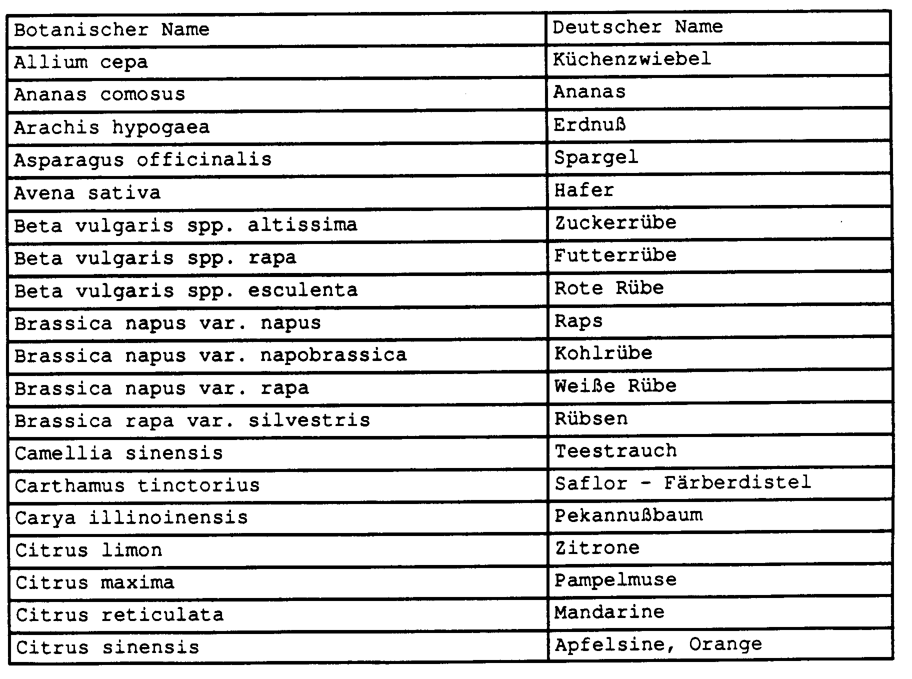

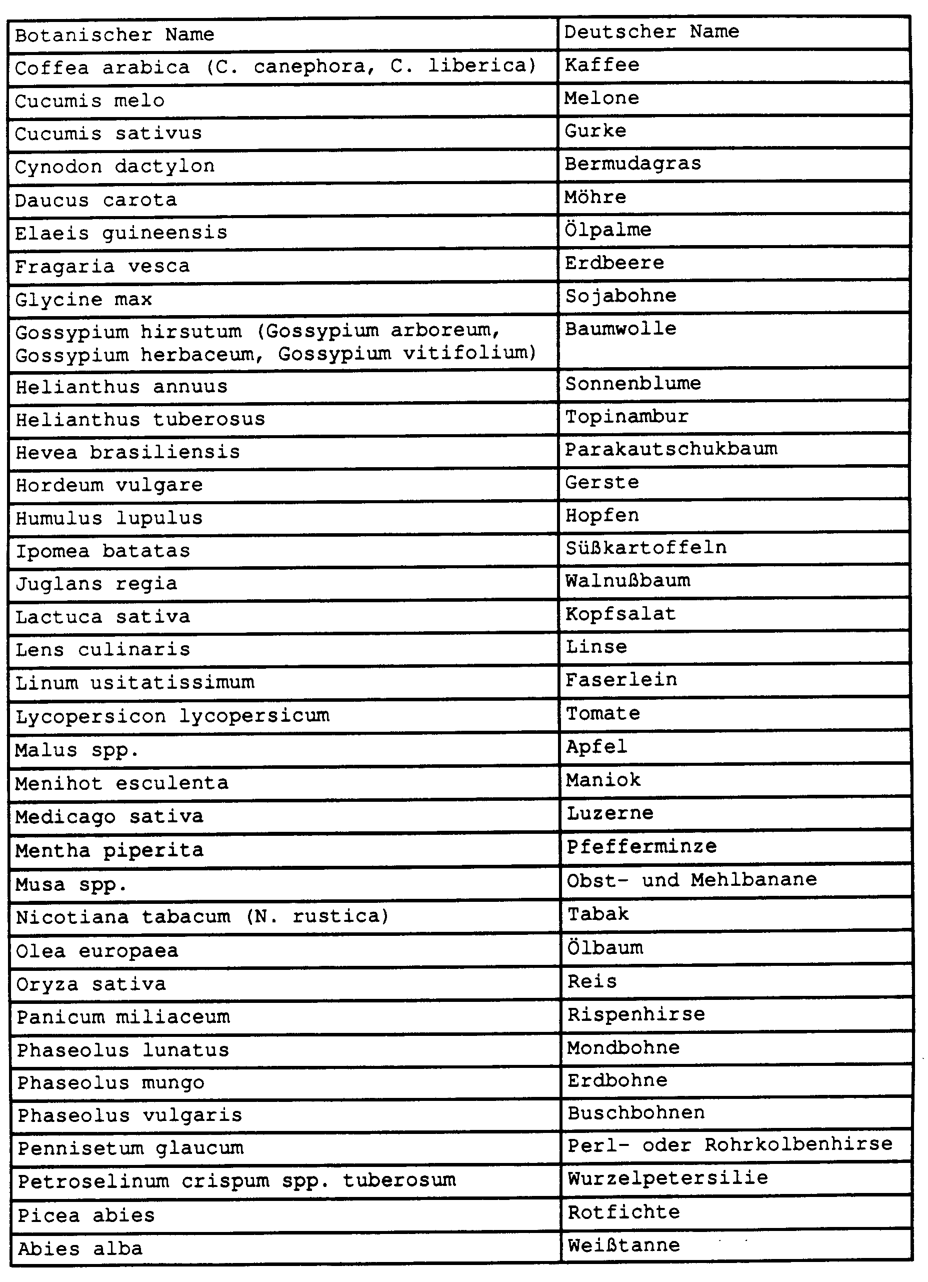

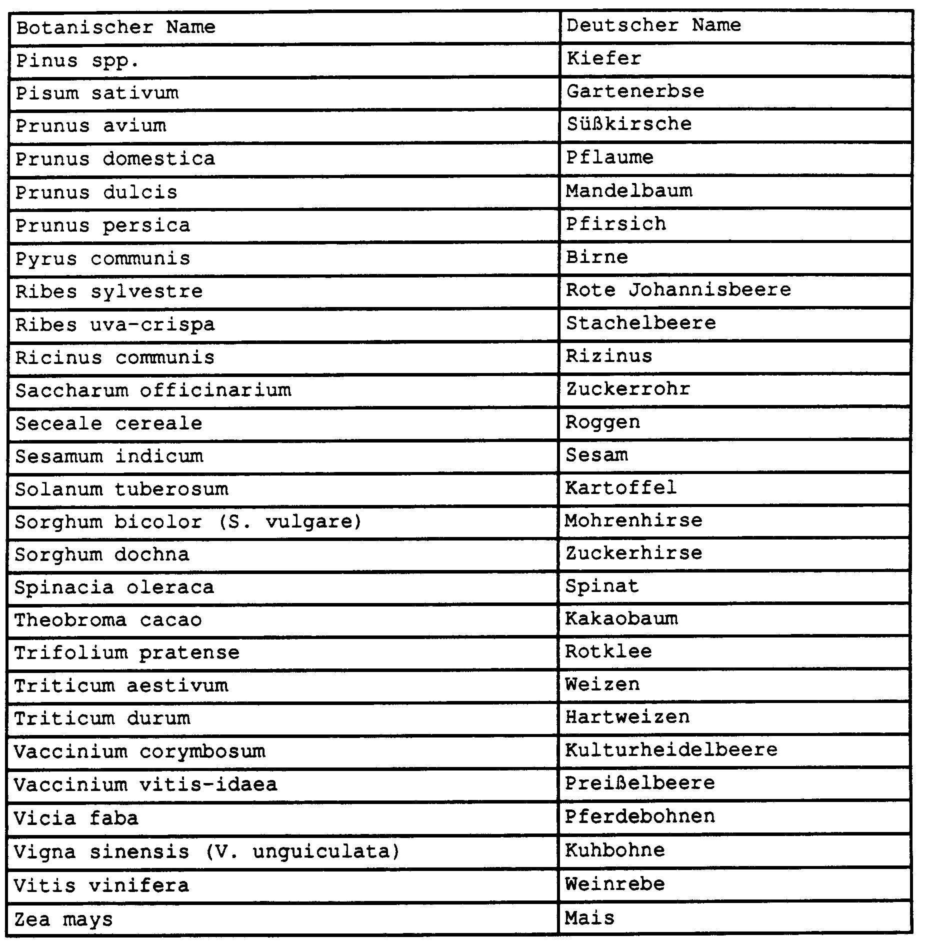

Weiterhin haben die Verbindungen I eine besondere Bedeutung für die Bekämpfung einer Vielzahl von Pilzen an verschiedenen Kulturpflanzen wie Weizen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächse sowie an den Samen dieser Pflanzen.

Speziell eignen sie sich zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaere leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pilze oder die vor Pilzbefall zu schützenden Pflanzen, Saatgüter, Materialien oder den Erdbeeren mit einer fungizid wirksamen Menge der Wirkstoffe behandelt. Die Anwendung erfolgt vor oder nach der Infektion der Materialien, Pflanzen oder Samen durch die Pilze.

Die fungiziden Mittel enthalten im allgemeinen zwischen 0,1 und 95, vorzugsweise zwischen 0,5 und 50 Gew.-% Wirkstoff.

Die Aufwandmengen liegen je nach Art des gewünschten Effektes zwischen 0,02 und 3 kg Wirkstoff pro ha. Die neuen Verbindungen können auch im Materialschutz (Holzschutz) eingesetzt werden, z.B. gegen Paecilomyces variotii.

Bei einer Saatgutbehandlung werden im allgemeinen Wirkstoffmengen von 0,001 bis 50 g, vorzugsweise 0,1 bis 10 g je Kilogramm Saatgut benötigt.

Die Mittel bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Zur Verbreiterung des Wirkungsspektrums und zur Erzielung synergistischer Effekte können die erfindungsgemäßen Verbindungen I mit zahlreichen Vertretern anderer herbizider und wachstumsregulierender Wirkstoffgruppen gemischt und gemeinsam ausgebracht werden. Beispielsweise kommen als Mischungspartner Diazine, (4H)-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Aryloxy- und Heteroaryloxyphenoxypropionsäuren sowie deren Salze, Ester, Amide und andere in Betracht.

Außerdem kann es von Nutzen sein, die Verbindungen I allein oder in Kombination mit anderen Herbiziden auch noch mit anderen Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementemängeln eingesetzt werden. Es können auch nicht phytotoxische Öle und Ölkonzentrate zugesetzt werden.

### Synthesebeispiele:

### Beispiel 1

### Allgemeine Vorschrift zur Herstellung aromatischer Carbonsäurehydroxylaminester oder ähnlicher Verbindungen der Formel I:

3,2 mmol der jeweiligen aromatischen 2-(4,6-Dimethoxypyrimidin-2-yl)oxycarbonsäure werden in 20 ml Dimethoxyethan vorgelegt und mit 3,2 mmol Natriumhydrid versetzt, wobei sofort eine Gasentwicklung auftritt. Es wird 1 h bei Raumtemperatur nachgerührt, auf 0°C gekühlt und mit 3,5 mmol Oxalylchlorid versetzt. Man rührt bei 0°C 1 h nach und verdampft dann zur Entfernung des überschüssigen Oxalylchlorids etwa 30 % des Lösungsmittels im Vakuum. Man gibt nun 4,2 mmol des jeweiligen Hydroxylamins oder einer vergleichbaren Hydroxy-Verbindung gelöst in 10 ml Dimethoxyethan und anschließend 3,2mmol Pyridin bei 0°C zu und erwärmt innerhalb von ca. 1 h auf Raumtemperatur. Die Mischung wird in 120 ml kaltes Wasser gegossen und mit Methylenchlorid extrahiert. Die organische Phase wird über Natriumsulfat getrocknet und im Vakuum eingeengt. Die zurückbleibende Substanz kann durch Chromatographie an Silica-Gel weiter gereinigt werden.

### Beispiel 2

### Allgemeine Vorschrift zur Herstellung aromatischer Carbonsäurehydroxylaminester oder ähnlicher Verbindungen der Formel I:

10 mmol der entsprechenden 6-Hetaryl-2-(4,6-dimethoxypyrimidin-2-yloxy)-benzoesäure werden in 30ml Tetrahydrofuran gelöst und mit 1.75 g (10,8 mmol) N,N'-Carbonylbisimidazol versetzt. Nach 30 min Rühren bei Raumtemperatur fügt man 9,9 mmol der entsprechenden Hydroxyverbindung hinzu und rührt weitere 14 h. Das Reaktionsgemisch wird sodann mit 300 ml 1 N-Phosphorsäure hydrolysiert und das resultierende Gemisch mehrfach mit Methyl-tert.-butylether extrahiert. Die organischen Phasen werden über Natriumsulfat getrocknet und im Vakuum eingeengt. Der verbleibende Rückstand kann durch Säulenchromatographie oder Umkristallisieren weiter gereinigt werden.

### Beispiel 3

### Allgemeine Vorschrift zur Herstellung aromatischer Carbonsäurehydroxylaminester oder ähnlicher Verbindungen der Formel I:

10 mmol der entsprechenden 6-Hetaryl-salicylsäure werden in 30 ml Dioxan gelöst und mit 1.75 g (10,8 mmol) N,N'-Carbonylbisimidazol versetzt. Nach 30 min Rühren bei Raumtemperatur fügt man 9.9 mmol der entsprechenden Hydroxyverbindung hinzu und rührt weitere 14 h. Der Ansatz wird sodann mit 300 ml 1N-Phosphorsäure hydrolysiert und das resultierende Gemisch mehrfach mit Methyl-tert.-butylether extrahiert. Die organischen Phasen werden vereint, über Natriumsulfat getrocknet und im Vakuum eingeengt. Der Rückstand wird mit 40 ml Dimethylformamid aufgenommen und mit 280 mg Natriumhydrid (85 % in Paraffin, 10 mmol) versetzt. Nach 30 min Rühren bei Raumtemperatur fügt man 1,97 g (9 mmol) 4,6-Dimethoxy-2-methylsulfonyl-pyrimidin hinzu und rührt 14 h nach. Das Reaktionsgemisch wird auf 300 ml 0.1 n Phosphorsäure gegeben und mit Diethylether extrahiert. Die Etherphase wird über Natriumsulfat getrocknet, eingeengt und der Rückstand durch Säulenchromatographie oder Umkristallisieren gereinigt.

### Beispiel 4

### Allgemeine Vorschrift zur Herstellung aromatischer Carbonsäurehydroxylaminester oder ähnlicher Verbindungen der Formel I:

Zu 15 mmol 6-Hetaryl-salicylsäureazolylester gelöst in 25 ml getrocknetem Dimethylformamid werden bei 10°C 0,46 g (0,015 mol) Natriumhydrid (80 %ig) gegeben und bei 30°C 3 h nachgerührt. Dann werden 3,27 g (0,015 mol) 2-Methylsulfonyl-4,6-dimethoxypyrimidin zugegeben und 12 h bei Raumtemperatur nachgerührt. Das Reaktionsgemisch wird in 500 ml Wasser gegeben, dem vorher 2,5 ml Orthophosphorsäure zugesetzt wurden. Das sich abscheidende Öl wird in Essigsäureethylester aufgenommen und über Natriumsulfat getrocknet. Der nach dem Einengen verbleibende schmierige Rückstand wird durch Säulenchromatographie oder Umkristallisieren gereinigt.

### Beispiel 5

### 6-Pyrazolyl-2-(4,6-dimethoxypyrimidin-2-yloxy)-1-(N,N-dimethylaminooxy-carbonyl)-benzol

2.47 g (10 mmol) 3-Pyrazolyl-2-(N,N-dimethylaminooxycarbonyl)phenol werden in 25 ml getrocknetem Dimethylformamid gelöst, bei 10°C mit 0,30 g (0,01 mol) Natriumhydrid (80 %ig) versetzt und bei 30°C 3 h nachgerührt. Dann werden 2,18 g (0,01 mol) 2-Methylsulfonyl-4,6-dimethoxypyrimidin zugegeben und 12 h bei Raumtemperatur nachgerührt. Das Reaktionsgemisch wird in 500 ml Wasser gegeben, dem vorher 2,5 ml Orthophosphorsäure zugesetzt wurden. Das sich abscheidende Öl wird in Essigsäureethylester aufgenommen und über Natriumsulfat getrocknet. Der nach dem Einengen verbleibende schmierige Rückstand wird durch Säulenchromatographie gereinigt und man erhält einen farblosen Feststoff.

### Beispiel 6

### 6-(2-Thienyl)-2-(4,6-dimethoxypyrimidin-2-yloxy)-1-(N,N-dimethylaminooxy-carbonyl)-benzol

1,14 g (3,2 mmol) 6-(2-Thienyl)-2-(4,6-dimethoxypyrimidin-2-yl)bezoesäure werden in 20 ml Dimethoxyethan vorgelegt und mit 3,2 mmol Natriumhydrid versetzt, wobei sofort eine Gasentwicklung auftritt. Es wird 1 h bei Raumtemperatur nachgerührt, auf 0°C gekühlt und mit 3,5 mmol Oxalylchlorid versetzt. Man rührt bei 0°C 1 h nach und verdampft dann zur Entfernung des überschüssigen Oxalylchlorids etwa 30 % des Lösungsmittels im Vakuum. Man gibt nun eine Lösung von 4,2 mmol N-N-Dimethylhydroxylamin in 10 ml Dimethoxyethan (aus 230 mg N,N-Dimethylhydroxylamin-hydrochlorid und 332 mg Pyridin) und anschließend 3,2 mmol Pyridin bei 0°C zu und erwärmt innerhalb von 1 h auf Raumtemperatur. Die Mischung wird in 120 ml kaltes Wasser gegossen und mit Methylenchlorid extrahiert. Die organische Phase wird über Natriumsulfat getrocknet und im Vakuum eingeengt. Das zurückbleibende Öl wird durch Chromatographie an Silica-Gel weiter gereinigt. Man erhält einen farblosen Feststoff, Fp. 102-112°C (Wirkstoff Nr. 1.001)

### Beispiel 7

### 6-Imidazolyl-2-(3,5-dimethoxy-s-triazin-1-yloxy)-1-[(1-pyrazolyl)oxy-carbonyl]-benzol

1,88 g (10 mmol) 6-Imidazolyl-salicylsäure werden in 30 ml Tetrahydrofuran gelöst und mit 1.75 g(10.8 mmol) N,N'-Carbonylbisimidazol versetzt. Nach 30 min Rühren bei Raumtemperatur fügt man 9,9 mmol N-Hydroxypyrazol hinzu und rührt weitere 14 h. Das Reaktionsgemisch wird sodann mit 300 ml 1N-Phosphorsäure hydrolysiert und das resultierende Gemisch mehrfach mit Methyl-tert.-butylether extrahiert. Die organischen Phasen werden über Natriumsulfat getrocknet und im Vakuum eingeengt. Der Rückstand wird mit 40 ml Dimethylformamid aufgenommen und mit 280 mg Natriumhydrid (85 % in Paraffin, 10 mmol) versetzt. Nach 30 min Rühren bei Raumtemperatur fügt man 1,58 g (9 mmol) 1-Chlor-3,5-dimethoxy-s-triazin hinzu und rührt 14 h nach. Der Ansatz wird auf 300 ml 0.1n Phosphorsäure gegeben und mit Diethylether extrahiert. Die Etherphase wird über Natriumsulfat getrocknet, eingeengt und der Rückstand durch Säulenchromatographie gereinigt.

### Beispiel 8

### 6-(2-Thienyl)-2-(4,6-dimethoxytriazin-2-yloxy)-1-(N,N-dimethylaminooxy-carbonyl)-benzol

4,4 g (20 mmol) 6-(2-Thienyl)-salicylsäure werden in 30 ml Thionylchlorid gelöst und 90 min unter Rückfluß erhitzt. Nach dem Abkühlen engt man die Reaktionslösung ein und versetzt zum Entfernen des Thionylchlorids noch zweimal mit je 100 ml Toluol und engt ein. Der Rückstand wird in 50 ml Toluol gelöst, mit einer Lösung von N,N-Dimethylhydroxylamin (aus 6,85 g N,N-Dimethylhydroxylamin-Hydrochlorid, 15 g Kaliumcarbonat und 30 ml Toluol) versetzt und 14 h bei Raumtemperatur gerührt. Die Reaktionslösung wird in ein Gemisch aus 150 ml Wasser und 4,5 g Orthophosphorsäure gegeben, das Gemisch mit Essigester extrahiert, die vereinigten organischen Phasen mit Wasser und gesättigter Kochsalzlösung gewaschen, über Natriumsulfat getrocknet und dann eingeengt. Nach Trocknen an der Ölpumpe verbleiben 5 g eines farblosen Öls, das in 50 ml DMF gelöst und mit 0,64 g Natriumhydrid (85 %) versetzt wird. Nach 30 min Rühren fügt man 3,67 g (21 mmol) Chlordimethoxytriazin hinzu und erhitzt für zwei Stunden auf 50°C. Die Reaktionslösung wird in 400 ml Wasser/5 ml Orthophosphorsäure gegossen, mit Essigester extrahiert und nach Trocknen über Natriumsulfat und Einengen an Kieselgel chromatographiert. Man erhält 2,2 g des Produktes als farbloses, zähes Öl. ¹H-NMR in CDCl₃, 270 mHz, δ in ppm: 2,58 (s, 6H), 3,99 (s, 6H), 7,0-7,6 (m, 6H) (Wirkstoff Nr. 1.002).

Die in den Tabellen 1 und 2 wiedergegebenen Strukturen beschreiben besonders bevorzugte Wirkstoffe der Formel I und lassen sich unter entsprechender Abwandlung der Ausgangsverbindungen gemäß den voranstehenden Synthesebeispielen herstellen.

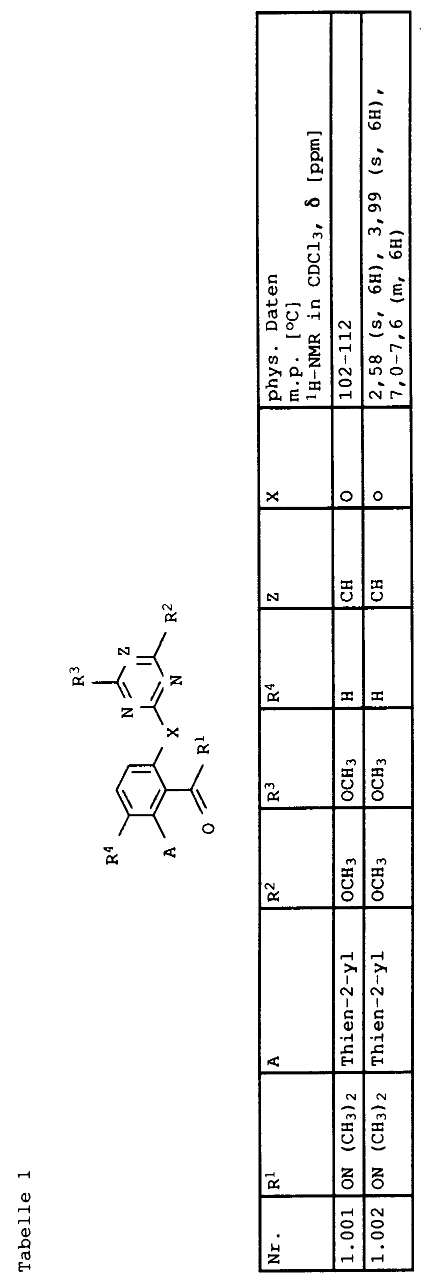

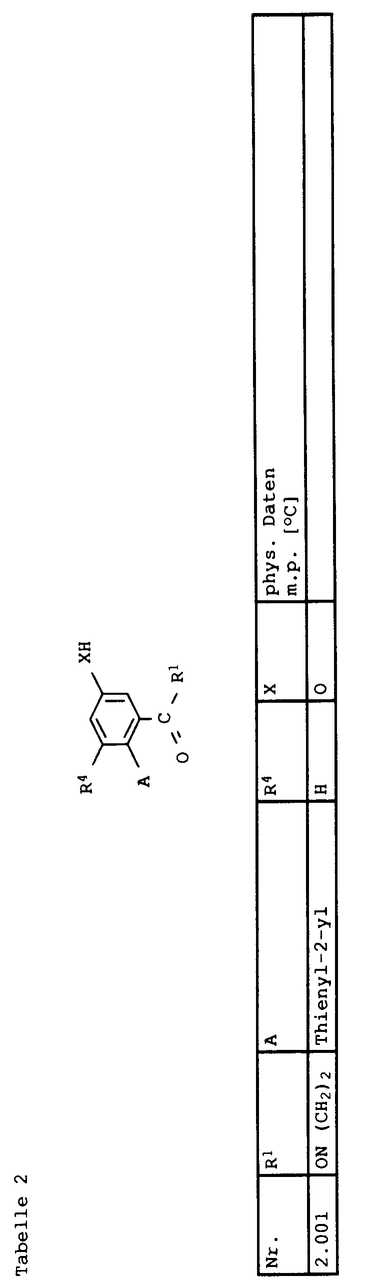

### Anwendungsbeispiele

Die herbizide Wirkung der Salicyl(thio)etherderivate der Formel I ließ sich durch Gewächshausversuche zeigen:
Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,5 kg/ha a.S.

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurden ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzen sich aus folgenden Arten zusammen:

| Lateinischer Name | Deutscher Name | Englischer Name |
|---|---|---|
| Echinocloa crus-galli | Hühnerhirse | barzinyard grass |
| Galium aparine | Klettenlabkraut | catchweed |
| Centaurea cyanus | Kornblume | cornflower |

Mit 0,5 kg/ha a.S. im Nachauflaufverfahren eingesetzt, lassen sich mit den Beispielen 1.001 und 1.002 unerwünschte Pflanzen sehr gut bekämpfen.

## Patentansprüche (Patentansprüche für folgende(n) Vertragsstaat(en): AT, BE, CH, DE, FR, GB, IT, LI, NL, SE)

1. Salicyl(thio)etherderivate der Formel I, 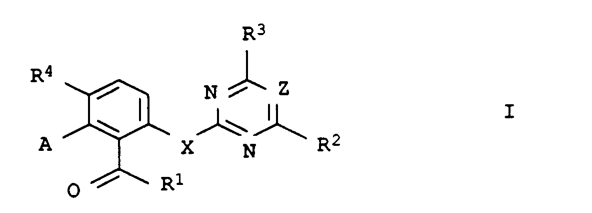 in der die Substituenten folgende Bedeutung haben:
R¹ ein Rest 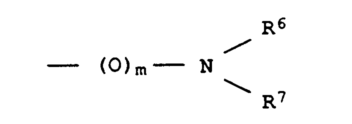 in dem m für 0 oder 1 steht und R⁶ und R⁷, die folgende Bedeutung haben:
Wasserstoff;
C₁-C₆-Alkyl, C₃-C₆-Alkenyl, C₃-C₆-Alkinyl, wobei diese Reste jeweils ein bis fünf Halogenatome und/oder ein bis zwei der folgenden Gruppen tragen können: C₁-C₆-Alkoxy, C₃-C₆-Alkenyloxy, C₃-C₆-Alkinyloxy, C₁-C₆-Alkylthio, C₃-C₆-Alkenylthio, C₃-C₆-Alkinylthio, C₁-C₆-Halogenalkoxy, Cyano, C₁-C₆-Alkylcarbonyl, C₃-C₆-Alkenylcarbonyl, C₃-C₆-Alkinylcarbonyl, C₁-C₆-Alkoxycarbonyl, C₃-C₆-Alkenyloxycarbonyl, C₃-C₆-Alkinyloxycarbonyl, C₁-C₆-Dialkylamino, C₁-C₆-Cycloalkyl;
ggf. substituiertes C₁-C₆-Cycloalkyl;
ggf. substituiertes Phenyl;
R⁶ zusammen mit R⁷ eine ggf. substituierte C₄-C₇-Alkylenkette, in der eine CH₂-Gruppe durch Sauerstoff, Schwefel oder -NH ersetzt sein kann;
eine Gruppe 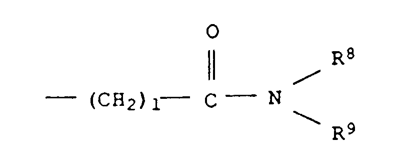 in der R⁸ und R⁹ für Wasserstoff, C₁-C₆-Alkyl, C₃-C₆-Alkenyl oder C₃-C₆-Alkinyl stehen und l die Werte 1, 2, 3 oder 4 annimmt;
oder eine Gruppe
―(CH₂)ₗ―S(=O)ₖ-R¹⁰
in der R¹⁰ für C₁-C₆-Alkyl, C₃-C₆-Alkenyl oder C₃-C₆-Alkinyl steht, l für 1, 2, 3 oder 4 und k für 0, 1 oder 2 stehen;
ein Rest OR⁵, worin
R⁵ ein über ein Stickstoffatom gebundener ggf. substituierter 5-gliedriger aromatischer Heterocyclus mit ein bis vier Stickstoffatomen im Ring
oder einen Rest 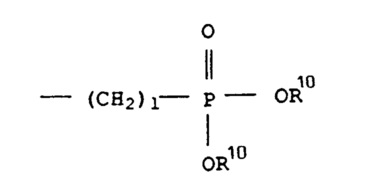 bedeutet, in dem R¹⁰ und l die oben genannte Bedeutung haben,
oder ein Rest
-HN-SO₂-R¹²
in dem R¹² für die Reste C₁-C₆-Alkyl, C₁-C₆-Halogenalkyl oder Phenyl steht, die ihrerseits ein bis vier der folgenden Substituenten tragen können: Halogen, Nitro, Cyano, C₁-C₆-Alkyl;
R²,R³ C₁-C₄-Alkyl, C₁-C₄-Halogenalkyl, C₁-C₄-Alkoxy, C₁-C₄-Halogenalkoxy und/oder C₁-C₄-Alkylthio;
X ein Sauerstoff- oder Schwefelatom;
Z Stickstoff oder die Methingruppe;
R⁴ Wasserstoff, Halogen, Nitro, C₁-C₄-Alkyl, Cyano oder C₁-C₄-Halogenalkyl;
A ein 5-gliedriger Heteroaromat mit ein bis vier Stickstoffatomen oder ein bis drei Stickstoffatomen sowie zusätzlich einem Schwefel- oder Sauerstoffatom im Ring, welcher ein bis drei Halogenatome und/oder einen bis drei der folgenden Reste tragen kann: Nitro, Cyano, C₁-C₄-Alkyl, C₁-C₄-Alkylthio, C₁-C₄-Halogenalkyl oder Phenyl, das unsubstituiert oder durch ein bis drei Halogenatome und/oder ein bis drei Methylgruppen substituiert ist;
ein benzokondensierter 5-gliedriger Heteroaromat, der ein bis drei Stickstoffatome oder ein Stickstoffatom und zusätzlich ein Sauerstoff- oder Schwefelatom enthalten und einen der folgenden Reste tragen kann: C₁-C₄-Alkyl, Halogen, Cyano;
ein Thienyl- oder Furylrest, der ein bis drei Halogenatome und/oder einen bis drei der folgenden Reste tragen kann: C₁-C₄-Alkyl, C₁-C₂-Halogenalkyl, ggf. substituiertes Phenyl oder Nitro;
ein Benzthienyl- oder Benzofurylrest, der ein Halogenatom und/oder einen der folgenden Reste tragen kann: C₁-C₄-Alkyl, C₁-C₂-Halogenalkyl oder Nitro;
ein Pyridylrest, der ein bis drei Halogenatome und/oder einen bis drei der folgenden Reste tragen kann: C₁-C₄-Alkyl, C₁-C₂-Halogenalkyl, ggf. substituiertes Phenyl oder Nitro;
ein Naphthyl- oder Chinolinylrest, welcher jeweils ein bis drei Halogenatome und/oder ein bis drei der folgenden Reste tragen kann: C₁-C₄-Alkyl oder C₁-C₂-Halogenalkyl,
wobei der Ausdruck ggf. substituiert in den oben genannten Fällen jeweils bedeutet, daß die entsprechenden Gruppen einen oder mehrere der folgenden Substituenten tragen können: Halogen, Nitro, Cyano, C₁-C₆-Alkyl, C₁-C₆-Halogenalkyl, C₁-C₆-Alkoxy, C₁-C₆-Alkylthio.

2. Salicylsäurederivate der Formel I gemäß Anspruch 1, in der
R¹ eine Gruppe 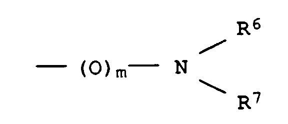 darstellt, in der m 0 oder 1 und R⁶, R⁷ Wasserstoff oder Methyl bedeuten oder R⁶ mit R⁷ eine C₄- oder C₅-Alkylenkette bildet;
R², R³ Methoxy, Methyl, Difluormethoxy oder Chlor;
R⁴ Wasserstoff oder Methyl;
X Sauerstoff;
Y Stickstoff;
Z die Methingruppe und
A die in Anspruch 1 genannte Bedeutung haben.

3. Salicylsäurederivate der Formel I gemäß Anspruch 1, in der R¹ O-N(CH₃)₂, X Sauerstoff, Y Stickstoff, Z die Methingruppe und R² und R³ Methoxy darstellen und A die oben genannte Bedeutung hat.

4. Neue Salicylsäurederivate der Formel II 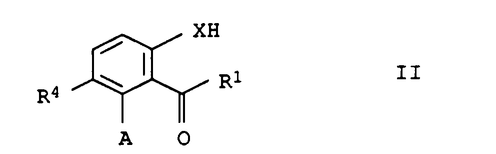 in der die Reste folgende Bedeutung haben:
R¹ ein Rest 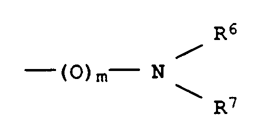 in dem m für 0 oder 1 steht und R⁶ und R⁷ die folgende Bedeutung haben:
Wasserstoff;
C₁-C₆-Alkyl, C₃-C₆-Alkenyl, C₃-C₆-Alkinyl, wobei diese Reste jeweils ein bis fünf Halogenatome und/oder ein bis zwei der folgenden Gruppen tragen können: C₁-C₆-Alkoxy, C₃-C₆-Alkenyloxy, C₃-C₆-Alkinyloxy, C₁-C₆-Alkylthio, C₃-C₆-Alkenylthio, C₃-C₆-Alkinylthio, C₁-C₆-Halogenalkoxy, Cyano, C₁-C₆-Alkylcarbonyl, C₃-C₆-Alkenylcarbonyl, C₃-C₆-Alkinylcarbonyl, C₁-C₆-Alkoxycarbonyl, C₃-C₆-Alkenyloxycarbonyl, C₃-C₆-Alkinyloxycarbonyl, C₁-C₆-Dialkylamino, C₃-C₆-Cycloalkyl;
ggf. substituiertes C₁-C₆-Cycloalkyl;
ggf. substituiertes Phenyl;
R⁶ zusammen mit R⁷ eine ggf. substituierte C₄-C₇-Alkylenkette, in der eine CH₂-Gruppe durch Sauerstoff, Schwefel oder -NH ersetzt sein kann;
eine Gruppe 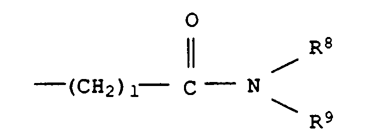 in der R⁸ und R⁹ für Wasserstoff, C₁-C₆-Alkyl, C₃-C₆-Alkenyl oder C₃-C₆-Alkinyl stehen und l die Werte 1, 2, 3 oder 4 annimmt;
oder eine Gruppe
―(CH₂)ₗ―S(=O)ₖ-R¹⁰
in der R¹⁰ für C₁-C₆-Alkyl, C₃-C₆-Alkenyl oder C₃-C₆-Alkinyl steht, l für 1, 2, 3 oder 4 und k für 0, 1 oder 2 stehen;
ein Rest OR⁵, worin
R⁵ ein über ein Stickstoffatom gebundener ggf. substituierter 5-gliedriger aromatischer Heterocyclus mit ein bis vier Stickstoffatomen im Ring
oder einen Rest 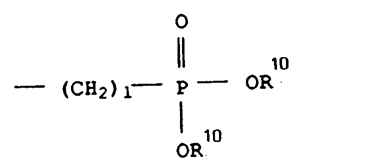 bedeutet, in dem R¹¹ und l die oben genannte Bedeutung haben,
oder ein Rest
-HN-SO₂-R¹²
in dem R¹² für die Reste C₁-C₆-Alkyl, C₁-C₆-Halogenalkyl oder Phenyl steht, die ihrerseits ein bis vier der folgenden Substituenten tragen können: Halogen, Nitro, Cyano, C₁-C₆-Alkyl;
R²,R³ C₁-C₄-Alkyl, C₁-C₄-Halogenalkyl, C₁-C₄-Alkoxy, C₁-C₄-Halogenalkoxy und/oder C₁-C₄-Alkylthio;
X ein Sauerstoff- oder Schwefelatom;
Z Stickstoff oder die Methingruppe;
R⁴ Wasserstoff, Halogen, Nitro, C₁-C₄-Alkyl, Cyano oder C₁-C₄-Halogenalkyl;
A ein 5-gliedriger Heteroaromat mit ein bis vier Stickstoffatomen oder ein bis drei Stickstoffatomen sowie zusätzlich einem Schwefel- oder Sauerstoffatom im Ring, welcher ein bis drei Halogenatome und/oder einen bis drei der folgenden Reste tragen kann: Nitro, Cyano, C₁-C₄-Alkyl, C₁-C₄-Alkylthio, C₁-C₄-Halogenalkyl oder Phenyl, das unsubstituiert oder durch ein bis drei Halogenatome und/oder ein bis drei Methylgruppen substituiert ist;
ein benzokondensierter 5-gliedriger Heteroaromat, der ein bis drei Stickstoffatome oder ein Stickstoffatom und zusätzlich ein Sauerstoff- oder Schwefelatom enthalten und einen der folgenden Reste tragen kann: C₁-C₄-Alkyl, Halogen, Cyano;
ein Thienyl- oder Furylrest, der ein bis drei Halogenatome und/oder einen bis drei der folgenden Reste tragen kann: C₁-C₄-Alkyl, C₁-C₂-Halogenalkyl, ggf. substituiertes Phenyl oder Nitro;
ein Benzthienyl- oder Benzofurylrest, der ein Halogenatom und/oder einen der folgenden Reste tragen kann: C₁-C₄-Alkyl, C₁-C₂-Halogenalkyl oder Nitro;
ein Pyridylrest, der ein bis drei Halogenatome und/oder einen bis drei der folgenden Reste tragen kann: C₁-C₄-Alkyl, C₁-C₂-Halogenalkyl, ggf. substituiertes Phenyl oder Nitro;
ein Naphthyl- oder Chinolinylrest, welcher jeweils ein bis drei Halogenatome und/oder ein bis drei der folgenden Reste tragen kann: C₁-C₄-Alkyl oder C₁-C₂-Halogenalkyl;
wobei der Ausdruck ggf. substituiert in den oben genannten Fällen jeweils bedeutet, daß die entsprechenden Gruppen einen oder mehrere der folgenden Substituenten tragen können: Halogen, Nitro, Cyano, C₁-C₆-Alkyl, C₁-C₆-Halogenalkyl, C₁-C₆-Alkoxy, C₁-C₆-Alkylthio.

5. Verfahren zur Herstellung der Verbindungen der Formel I gemäß Anspruch 1, dadurch gekennzeichnet, daß man ein entsprechendes Salicylsäure-oder Thiosalicylsäurederivat der Formel II 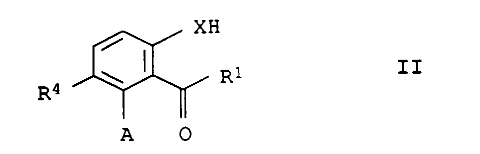 in an sich bekannter Weise mit einem Heteroaromaten der Formel III, 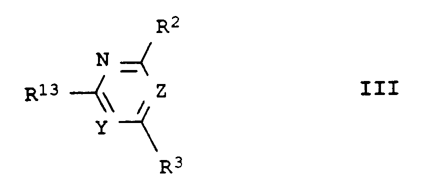 worin R¹³ für eine nucleofuge Abgangsgruppe steht, in Gegenwart einer anorganischen oder organischen Base umsetzt.

6. Verfahren zur Herstellung der Verbindungen der Formel I gemäß Anspruch 1, dadurch gekennzeichnet, daß man die freie Salicylsäure bzw. ihre Schwefelanalogen der Formel I' 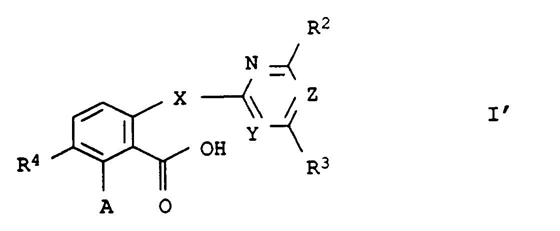 zunächst in an sich bekannter Weise in das Halogenid oder eine andere aktivierte Form der Carbonsäure überführt und diese dann gegebenenfalls in Gegenwart einer anorganischen oder organischen Base mit einem gegebenenfalls substituierten Hydroxylamin, N-Hydroxyazol, Hydroxyalkylphosphonat oder Sulfonamid umsetzt.

7. Herbizides Mittel, enthaltend eine Verbindung der Formel I gemäß Anspruch 1 und übliche inerte Zusatzstoffe.

8. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Derivates I gemäß Anspruch 1 behandelt.

9. Verfahren zur Regulierung des Pflanzenwachstums, dadurch gekennzeichnet, daß man eine regulativ wirksame Menge eines Salicylsäurederivats der allgemeinen Formel I gemäß Anspruch 1 auf die Samen, die Pflanzen und/oder deren Lebensraum einwirken läßt.

10. Fungizides und/oder nitrifikationshemmendes Mittel und/oder Mittel zur Beeinflussung des Pflanzenwachstums, enthaltend ein Salicylsäurederivat der Formel I gemäß Anspruch 1 und übliche inerte Zusatzstoffe.

## Patentansprüche (Patentansprüche für folgende(n) Vertragsstaat(en): ES)

1. Mittel, enthaltend neben üblichen inerten Zusatzstoffen Salicyl(thio)etherderivate der Formel I, 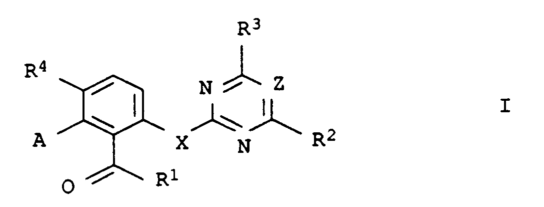 in der die Substituenten folgende Bedeutung haben:
R¹ ein Rest 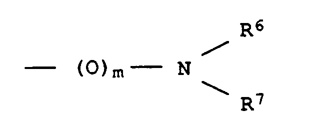 in dem m für 0 oder 1 steht und R⁶ und R⁷, die folgende Bedeutung haben:
Wasserstoff;
C₁-C₆-Alkyl, C₃-C₆-Alkenyl, C₃-C₆-Alkinyl, wobei diese Reste jeweils ein bis fünf Halogenatome und/oder ein bis zwei der folgenden Gruppen tragen können: C₁-C₆-Alkoxy, C₃-C₆-Alkenyloxy, C₃-C₆-Alkinyloxy, C₁-C₆-Alkylthio, C₃-C₆-Alkenylthio, C₃-C₆-Alkinylthio, C₁-C₆-Halogenalkoxy, Cyano, C₁-C₆-Alkylcarbonyl, C₃-C₆-Alkenylcarbonyl, C₃-C₆-Alkinylcarbonyl, C₁-C₆-Alkoxycarbonyl, C₃-C₆-Alkenyloxycarbonyl, C₃-C₆-Alkinyloxycarbonyl, C₁-C₆-Dialkylamino, C₁-C₆-Cycloalkyl;
ggf. substituiertes C₁-C₆-Cycloalkyl;
ggf. substituiertes Phenyl;
R⁶ zusammen mit R⁷ eine ggf. substituierte C₄-C₇-Alkylenkette, in der eine CH₂-Gruppe durch Sauerstoff, Schwefel oder -NH ersetzt sein kann;
eine Gruppe 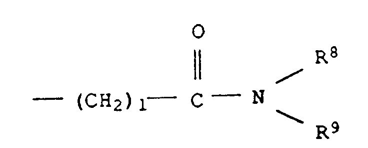 in der R⁸ und R⁹ für Wasserstoff, C₁-C₆-Alkyl, C₃-C₆-Alkenyl oder C₃-C₆-Alkinyl stehen und l die Werte 1, 2, 3 oder 4 annimmt;
oder eine Gruppe
―(CH₂)ₗ―S(=O)ₖ-R¹⁰
in der R¹⁰ für C₁-C₆-Alkyl, C₃-C₆-Alkenyl oder C₃-C₆-Alkinyl steht, l für 1, 2, 3 oder 4 und k für 0, 1 oder 2 stehen;
ein Rest OR⁵, worin
R⁵ ein über ein Stickstoffatom gebundener ggf. substituierter 5-gliedriger aromatischer Heterocyclus mit ein bis vier Stickstoffatomen im Ring
oder einen Rest 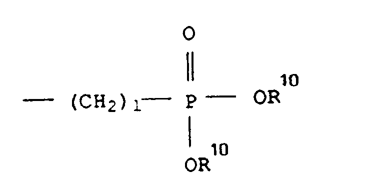 bedeutet, in dem R¹⁰ und l die oben genannte Bedeutung haben,
oder ein Rest
-HN-SO₂-R¹²
in dem R¹² für die Reste C₁-C₆-Alkyl, C₁-C₆-Halogenalkyl oder Phenyl steht, die ihrerseits ein bis vier der folgenden Substituenten tragen können: Halogen, Nitro, Cyano, C₁-C₆-Alkyl;
R²,R³ C₁-C₄-Alkyl, C₁-C₄-Halogenalkyl, C₁-C₄-Alkoxy, C₁-C₄-Halogenalkoxy und/oder C₁-C₄-Alkylthio;
X ein Sauerstoff- oder Schwefelatom;
Z Stickstoff oder die Methingruppe;
R⁴ Wasserstoff, Halogen, Nitro, C₁-C₄-Alkyl, Cyano oder C₁-C₄-Halogenalkyl;
A ein 5-gliedriger Heteroaromat mit ein bis vier Stickstoffatomen oder ein bis drei Stickstoffatomen sowie zusätzlich einem Schwefel- oder Sauerstoffatom im Ring, welcher ein bis drei Halogenatome und/oder einen bis drei der folgenden Reste tragen kann: Nitro, Cyano, C₁-C₄-Alkyl, C₁-C₄-Alkylthio, C₁-C₄-Halogenalkyl oder Phenyl, das unsubstituiert oder durch ein bis drei Halogenatome und/oder ein bis drei Methylgruppen substituiert ist;
ein benzokondensierter 5-gliedriger Heteroaromat, der ein bis drei Stickstoffatome oder ein Stickstoffatom und zusätzlich ein Sauerstoff- oder Schwefelatom enthalten und einen der folgenden Reste tragen kann: C₁-C₄-Alkyl, Halogen, Cyano;
ein Thienyl- oder Furylrest, der ein bis drei Halogenatome und/oder einen bis drei der folgenden Reste tragen kann: C₁-C₄-Alkyl, C₁-C₂-Halogenalkyl, ggf. substituiertes Phenyl oder Nitro;
ein Benzthienyl- oder Benzofurylrest, der ein Halogenatom und/oder einen der folgenden Reste tragen kann: C₁-C₄-Alkyl, C₁-C₂-Halogenalkyl oder Nitro;
ein Pyridylrest, der ein bis drei Halogenatome und/oder einen bis drei der folgenden Reste tragen kann: C₁-C₄-Alkyl, C₁-C₂-Halogenalkyl, ggf. substituiertes Phenyl oder Nitro;
ein Naphthyl- oder Chinolinylrest, welcher jeweils ein bis drei Halogenatome und/oder ein bis drei der folgenden Reste tragen kann: C₁-C₄-Alkyl oder C₁-C₂-Halogenalkyl,
wobei der Ausdruck ggf. substituiert in den oben genannten Fällen jeweils bedeutet, daß die entsprechenden Gruppen einen oder mehrere der folgenden Substituenten tragen können: Halogen, Nitro, Cyano, C₁-C₆-Alkyl, C₁-C₆-Halogenalkyl, C₁-C₆-Alkoxy, C₁-C₆-Alkylthio.

2. Mittel, enthaltend Salicylsäurederivate der Formel I gemäß Anspruch 1, in der
R¹ eine Gruppe 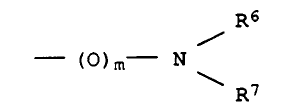 darstellt, in der m 0 oder 1 und R⁶, R⁷ Wasserstoff oder Methyl bedeuten oder R⁶ mit R⁷ eine C₄- oder C₅-Alkylenkette bildet;
R², R³ Methoxy, Methyl, Difluormethoxy oder Chlor;
R⁴ Wasserstoff oder Methyl;
X Sauerstoff;
Y Stickstoff;
Z die Methingruppe und
A die in Anspruch 1 genannte Bedeutung haben.

3. Mittel, enthaltend Salicylsäurederivate der Formel I gemäß Anspruch 1, in der R¹ O-N(CH₃)₂, X Sauerstoff, Y Stickstoff, Z die Methingruppe und R² und R³ Methoxy darstellen und A die oben genannte Bedeutung hat.

4. Neue Salicylsäurederivate der Formel II 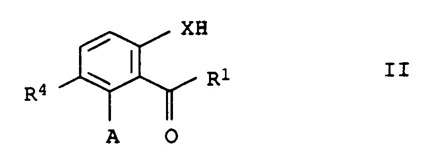 in der die Reste folgende Bedeutung haben:
R¹ ein Rest 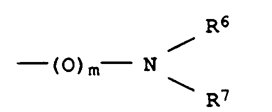 in dem m für 0 oder 1 steht und R⁶ und R⁷ die folgende Bedeutung haben:
Wasserstoff;
C₁-C₆-Alkyl, C₃-C₆-Alkenyl, C₃-C₆-Alkinyl, wobei diese Reste jeweils ein bis fünf Halogenatome und/oder ein bis zwei der folgenden Gruppen tragen können: C₁-C₆-Alkoxy₁ C₃-C₆-Alkenyloxy, C₃-C₆-Alkinyloxy, C₁-C₆-Alkylthio, C₃-C₆-Alkenylthio, C₃-C₆-Alkinylthio, C₁-C₆-Halogenalkoxy, Cyano, C₁-C₆-Alkylcarbonyl, C₃-C₆-Alkenylcarbonyl, C₃-C₆-Alkinylcarbonyl, C₁-C₆-Alkoxycarbonyl, C₃-C₆-Alkenyloxycarbonyl, C₃-C₆-Alkinyloxycarbonyl, C₁-C₆-Dialkylamino, C₃-C₆-Cycloalkyl;
ggf. substituiertes C₁-C₆-Cycloalkyl;
ggf. substituiertes Phenyl;
R⁶ zusammen mit R⁷ eine ggf. substituierte C₄-C₇-Alkylenkette, in der eine CH₂-Gruppe durch Sauerstoff, Schwefel oder -NH ersetzt sein kann;
eine Gruppe 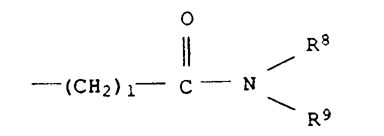 in der R⁸ und R⁹ für Wasserstoff, C₁-C₆-Alkyl, C₃-C₆-Alkenyl oder C₃-C₆-Alkinyl stehen und l die Werte 1, 2, 3 oder 4 annimmt;
oder eine Gruppe
―(CH₂)ₗ―S(=O)ₖ-R¹⁰
in der R¹⁰ für C₁-C₆-Alkyl, C₃-C₆-Alkenyl oder C₃-C₆-Alkinyl steht, l für 1, 2, 3 oder 4 und k für 0, 1 oder 2 stehen;
ein Rest OR⁵, worin
R⁵ ein über ein Stickstoffatom gebundener ggf. substituierter 5-gliedriger aromatischer Heterocyclus mit ein bis vier Stickstoffatomen im Ring
oder einen Rest 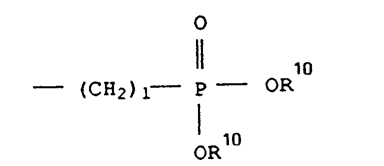 bedeutet, in dem R¹⁰ und l die oben genannte Bedeutung haben,
oder ein Rest
-HN-SO₂-R¹²
in dem R¹² für die Reste C₁-C₆-Alkyl, C₁-C₆-Halogenalkyl oder Phenyl steht, die ihrerseits ein bis vier der folgenden Substituenten tragen können: Halogen, Nitro, Cyano, C₁-C₆-Alkyl;
R²,R³ C₁-C₄-Alkyl, C₁-C₄-Halogenalkyl, C₁-C₄-Alkoxy, C₁-C₄-Halogenalkoxy und/oder C₁-C₄-Alkylthio;
X ein Sauerstoff- oder Schwefelatom;
Z Stickstoff oder die Methingruppe;
R⁴ Wasserstoff, Halogen, Nitro, C₁-C₄-Alkyl, Cyano oder C₁-C₄-Halogenalkyl;
A ein 5-gliedriger Heteroaromat mit ein bis vier Stickstoffatomen oder ein bis drei Stickstoffatomen sowie zusätzlich einem Schwefel- oder Sauerstoffatom im Ring, welcher ein bis drei Halogenatome und/oder einen bis drei der folgenden Reste tragen kann: Nitro, Cyano, C₁-C₄-Alkyl, C₁-C₄-Alkylthio, C₁-C₄-Halogenalkyl oder Phenyl, das unsubstituiert oder durch ein bis drei Halogenatome und/oder ein bis drei Methylgruppen substituiert ist;
ein benzokondensierter 5-gliedriger Heteroaromat, der ein bis drei Stickstoffatome oder ein Stickstoffatom und zusätzlich ein Sauerstoff- oder Schwefelatom enthalten und einen der folgenden Reste tragen kann: C₁-C₄-Alkyl, Halogen, Cyano;
ein Thienyl- oder Furylrest, der ein bis drei Halogenatome und/oder einen bis drei der folgenden Reste tragen kann: C₁-C₄-Alkyl, C₁-C₂-Halogenalkyl, ggf. substituiertes Phenyl oder Nitro;
ein Benzthienyl- oder Benzofurylrest, der ein Halogenatom und/oder einen der folgenden Reste tragen kann: C₁-C₄-Alkyl, C₁-C₂-Halogenalkyl oder Nitro;
ein Pyridylrest, der ein bis drei Halogenatome und/oder einen bis drei der folgenden Reste tragen kann: C₁-C₄-Alkyl, C₁-C₂-Halogenalkyl, ggf. substituiertes Phenyl oder Nitro;
ein Naphthyl- oder Chinolinylrest, welcher jeweils ein bis drei Halogenatome und/oder ein bis drei der folgenden Reste tragen kann: C₁-C₄-Alkyl oder C₁-C₂-Halogenalkyl;
wobei der Ausdruck ggf. substituiert in den oben genannten Fällen jeweils bedeutet, daß die entsprechenden Gruppen einen oder mehrere der folgenden Substituenten tragen können: Halogen, Nitro, Cyano, C₁-C₆-Alkyl, C₁-C₆-Halogenalkyl, C₁-C₆-Alkoxy, C₁-C₆-Alkylthio.

5. Verfahren zur Herstellung der Verbindungen der Formel I gemäß Anspruch 1, dadurch gekennzeichnet, daß man ein entsprechendes Salicylsäure-oder Thiosalicylsäurederivat der Formel II 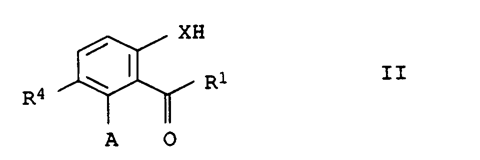 in an sich bekannter Weise mit einem Heteroaromaten der Formel III, 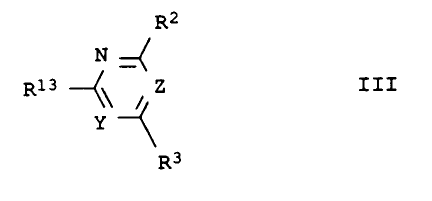 worin R¹³ für eine nucleofuge Abgangsgruppe steht, in Gegenwart einer anorganischen oder organischen Base umsetzt.

6. Verfahren zur Herstellung der Verbindungen der Formel I gemäß Anspruch 1, dadurch gekennzeichnet, daß man die freie Salicylsäure bzw. ihre Schwefelanalogen der Formel I' 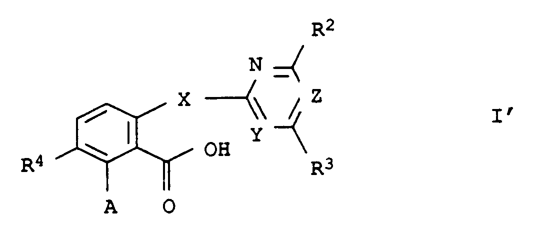 zunächst in an sich bekannter Weise in das Halogenid oder eine andere aktivierte Form der Carbonsäure überführt und diese dann gegebenenfalls in Gegenwart einer anorganischen oder organischen Base mit einem gegebenenfalls substituierten Hydroxylamin, N-Hydroxyazol, Hydroxyalkylphosphonat oder Sulfonamid umsetzt.

7. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Derivates I gemäß Anspruch 1 behandelt.

8. Verfahren zur Regulierung des Pflanzenwachstums, dadurch gekennzeichnet, daß man eine regulativ wirksame Menge eines Salicylsäurederivats der allgemeinen Formel I gemäß Anspruch 1 auf die Samen, die Pflanzen und/oder deren Lebensraum einwirken läßt.

9. Fungizides und/oder nitrifikationshemmendes Mittel und/oder Mittel zur Beeinflussung des Pflanzenwachstums, enthaltend ein Salicylsäurederivat der Formel I gemäß Anspruch 1 und übliche inerte Zusatzstoffe.

## Claims (Claims for the following Contracting State(s): AT, BE, CH, DE, FR, GB, IT, LI, NL, SE)

1. A salicyloyl(thio)ether derivative of the formula I 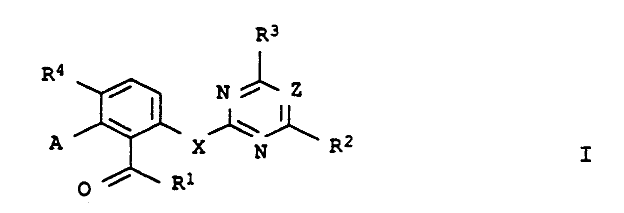 where
R¹ is a radical 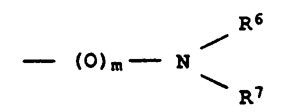 in which m is 0 or 1 and R⁶ and R⁷ are each hydrogen;
C₁-C₆-alkyl, C₃-C₆-alkenyl, C₃-C₆-alkynyl, where these radicals may each carry from one to five halogen atoms and/or one or two of the following groups: C₁-C₆-alkoxy, C₃-C₆-alkenyloxy, C₃-C₆-alkynyloxy, C₁-C₆-alkylthio, C₃-C₆-alkenylthio, C₃-C₆-alkynylthio, C₁-C₆-haloalkoxy, cyano, C₁-C₆-alkylcarbonyl, C₃-C₆-alkenylcarbonyl, C₃-C₆-alkynylcarbonyl, C₁-C₆-alkoxycarbonyl, C₃-C₆-alkenyloxycarbonyl, C₃-C₆-alkynyloxycarbonyl, C₁-C₆-dialkylamino or C₁-C₆-cycloalkyl;
unsubstituted or substituted C₁-C₆-cycloalkyl;
unsubstituted or substituted phenyl;
R⁶ together with R⁷ may be an unsubstituted or substituted C₄-C₇-alkylene chain in which a CH₂ group may be replaced with oxygen, sulfur or -NH;
a group 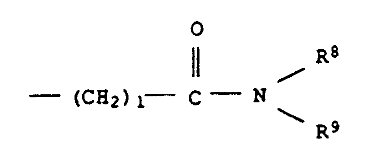 where R⁸ and R⁹ are each hydrogen, C₁-C₆-alkyl, C₃-C₆-alkenyl or C₃-C₆-alkynyl and l is 1, 2, 3 or 4; or a group
―(CH₂)ₗ―S(=O)ₖ-R¹⁰
where R¹⁰ is C₁-C₆-alkyl, C₃-C₆-alkenyl or C₃-C₆-alkynyl, l is 1, 2, 3 or 4 and k is 0, 1 or 2;
a radical OR⁵, where
R⁵ is an unsubstituted or substituted 5-membered aromatic heterocyclic structure bonded via a nitrogen atom and having from one to four nitrogen atoms in the ring or a radical 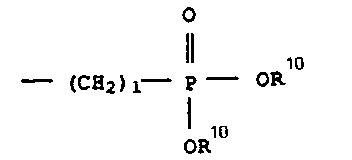 where R¹⁰ and l have the abovementioned meanings, or a radical
-HN-SO₂-R¹²
where R¹² is C₁-C₆-alkyl, C₁-C₆-haloalkyl or phenyl, which in turn may carry from one to four of the following substituents: halogen, nitro, cyano or C₁-C₆-alkyl; R² and R³ are each C₁-C₄-alkyl, C₁-C₄-haloalkyl, C₁-C₄-alkoxy, C₁-C₄-haloalkoxy and/or C₁-C₄-alkylthio; X is oxygen or sulfur;
Z is nitrogen or the methine group;
R⁴ is hydrogen, halogen, nitro, C₁-C₄-alkyl, cyano or C₁-C₄-haloalkyl; and
A is a 5-membered heteroaromatic structure having from one to four nitrogen atoms or from one to three nitrogen atoms and additionally a sulfur or oxygen atom in the ring, which may carry from one to three halogen atoms and/or from one to three of the following radicals: nitro, cyano, C₁-C₄-alkyl, C₁-C₄-alkylthio, C₁-C₄-haloalkyl or phenyl which is unsubstituted or substituted by from one to three halogen atoms and/or from one to three methyl groups;
a benzofused 5-membered heteroaromatic structure which may contain from one to three nitrogen atoms or a nitrogen atom and additionally one oxygen or sulfur atom and may carry one of the following radicals: C₁-C₄-alkyl, halogen or cyano;
a thienyl or furyl radical which may carry from one to three halogen atoms and/or from one to three of the following radicals: C₁-C₄-alkyl, C₁- or C₂-haloalkyl, unsubstituted or substituted phenyl or nitro;
a benzothienyl or benzofuryl radical which may carry one halogen atom and/or one of the following radicals: C₁-C₄-alkyl, C₁- or C₂-haloalkyl or nitro;
pyridyl which may carry from one to three halogen atoms and/or from one to three of the following radicals: C₁-C₄-alkyl, C₁- or C₂-haloalkyl, unsubstituted or substituted phenyl or nitro;
a naphthyl or quinolyl radical, each of which may carry up to three halogen atoms and/or up to three of the following radicals: C₁-C₄-alkyl or C₁- or C₂-haloalkyl, the expression unsubstituted or substituted in the abovementioned cases meaning that the corresponding groups may carry one or more of the following substituents: halogen, nitro, cyano, C₁-C₆-alkyl, C₁-C₆-haloalkyl, C₁-C₆-alkoxy or C₁-C₆-alkylthio.

2. A salicylic acid derivative of the formula I as claimed in claim 1,
where
R¹ is a group 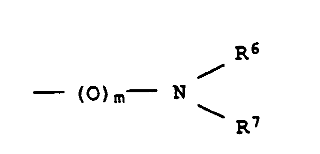 where m is 0 or 1 and R⁶ and R⁷ are each hydrogen or methyl or R⁶ and R⁷ together form a C₄- or C₅-alkylene chain;
R² and R³ are each methoxy, methyl, difluoromethoxy or chlorine;
R⁴ is hydrogen or methyl;
X is oxygen;
Y is nitrogen;
Z is the methine group and
A has the meanings stated in claim 1.

3. A salicylic acid derivative of the formula I as claimed in claim 1, where R¹ is O-N(CH₃)₂, X is oxygen, Y is nitrogen, Z is the methine group and R² and R³ are each methoxy and A has the abovementioned meanings.

4. A novel salicylic acid derivative of the formula II 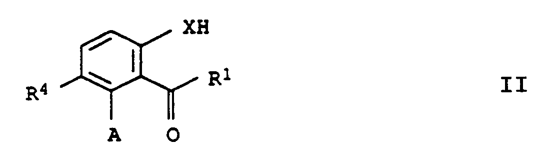 where
R¹ is a radical 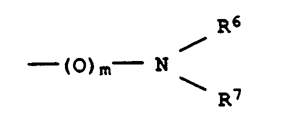 in which m is 0 or 1 and R⁶ and R⁷ are each hydrogen;
C₁-C₆-alkyl, C₃-C₆-alkenyl, C₃-C₆-alkynyl, where these radicals may each carry from one to five halogen atoms and/or one or two of the following groups: C₁-C₆-alkoxy, C₃-C₆-alkenyloxy, C₃-C₆-alkynyloxy, C₁-C₆-alkylthio, C₃-C₆-alkenylthio, C₃-C₆-alkynylthio, C₁-C₆-haloalkoxy, cyano, C₁-C₆-alkylcarbonyl, C₃-C₆-alkenylcarbonyl, C₃-C₆-alkynylcarbonyl, C₁-C₆-alkoxycarbonyl, C₃-C₆-alkenyloxycarbonyl, C₃-C₆-alkynyloxycarbonyl, C₁-C₆-dialkylamino or C₃-C₆-cycloalkyl;
unsubstituted or substituted C₁-C₆-cycloalkyl;
unsubstituted or substituted phenyl;
R⁶ together with R⁷ may be an unsubstituted or substituted C₄-C₇-alkylene chain in which a CH₂ group may be replaced with oxygen, sulfur or -NH;
a group 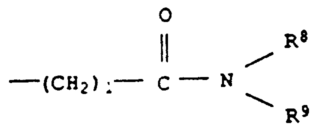 where R⁸ and R⁹ are each hydrogen, C₁-C₆-alkyl, C₃-C₆-alkenyl or C₃-C₆-alkynyl and l is 1, 2, 3 or 4; or a group
―(CH₂)ₗ―S(=O)ₖ-R¹⁰
where R¹⁰ is C₁-C₆-alkyl, C₃-C₆-alyenyl or C₃-C₆-alkynyl, l is 1, 2, 3 or 4 and k is 0, 1 or 2;
a radical OR⁵, where
R⁵ is an unsubstituted or substituted 5-membered aromatic heterocyclic structure bonded via a nitrogen atom and having from one to four nitrogen atoms in the ring or a radical 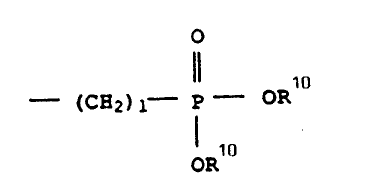 where R¹⁰ and l have the abovementioned meanings, or a radical
-HN-SO₂-R¹²
where R¹² is C₁-C₆-alkyl, C₁-C₆-haloalkyl or phenyl, which in turn may carry from one to four of the following substituents: halogen, nitro, cyano or C₁-C₆-alkyl;
R² and R³ are each C₁-C₄-alkyl, C₁-C₄-haloalkyl, C₁-C₄-alkoxy, C₁-C₄-haloalkoxy and/or C₁-C₄-alkylthio;
X is oxygen or sulfur;
Z is nitrogen or the methine group;
R⁴ is hydrogen, halogen, nitro, C₁-C₄-alkyl, cyano or C₁-C₄-haloalkyl; and
A is a 5-membered heteroaromatic structure having from one to four nitrogen atoms or from one to three nitrogen atoms and additionally a sulfur or oxygen atom in the ring, which may carry from one to three halogen atoms and/or from one to three of the following radicals: nitro, cyano, C₁-C₄-alkyl, C₁-C₄-alkylthio, C₁-C₄-haloalkyl or phenyl which is unsubstituted or substituted by from one to three halogen atoms and/or from one to three methyl groups;
a benzofused 5-membered heteroaromatic structure which may contain from one to three nitrogen atoms or a nitrogen atom and additionally one oxygen or sulfur atom and may carry one of the following radicals: C₁-C₄-alkyl, halogen or cyano;
a thienyl or furyl radical which may carry from one to three halogen atoms and/or from one to three of the following radicals: C₁-C₄-alkyl, C₁- or C₂-haloalkyl, unsubstituted or substituted phenyl or nitro;
a benzothienyl or benzofuryl radical which may carry one halogen atom and/or one of the following radicals: C₁-C₄-alkyl, C₁- or C₂-haloalkyl or nitro;
pyridyl which may carry from one to three halogen atoms and/or from one to three of the following radicals: C₁-C₄-alkyl, C₁- or C₂-haloalkyl, unsubstituted or substituted phenyl or nitro;
a naphthyl or quinolyl radical, each of which may carry up to three halogen atoms and/or up to three of the following radicals: C₁-C₄-alkyl or C₁- or C₂-haloalkyl, the expression unsubstituted or substituted in the abovementioned cases meaning that the corresponding groups may carry one or more of the following substituents: halogen, nitro, cyano, C₁-C₆-alkyl, C₁-C₆-haloalkyl, C₁-C₆-alkoxy or C₁-C₆-alkylthio.

5. A process for the preparation of a compound of the formula I as claimed in claim 1, wherein the corresponding salicylic acid or thiosalicylic acid derivatives of the formula II 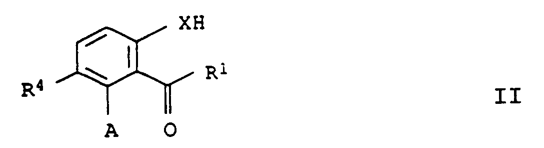 is reacted in a conventional manner with a heteroaromatic structure of the formula III 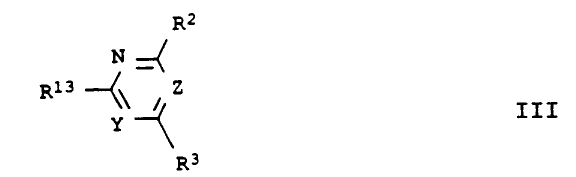 where R¹³ is a nucleofugic leaving group, in the presence of an inorganic or organic base.

6. A process for the preparation of a compound of the formula I as claimed in claim 1, wherein the free salicylic acid or a sulfur analog thereof of the formula I' 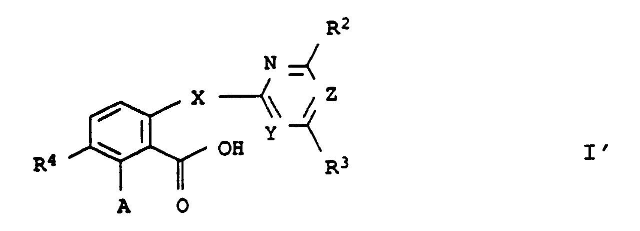 is first converted in a conventional manner into the halide or another activated form of the carboxylic acid and this is then reacted, in the presence or absence of an inorganic or organic base, with an unsubstituted or substituted hydroxylamine, N-hydroxyazole, hydroxyalkyl phosphonate or sulfonamide.

7. A herbicide containing a compound of the formula I as claimed in claim 1 and conventional inert additives.

8. A method for controlling undesirable plant growth, wherein the undesirable plants and/or their habitat is or are treated with a herbicidal amount of a derivative I as claimed in claim 1.

9. A method for regulating plant growth, wherein an amount, having a regulatory effect, of a salicylic acid derivative of the formula I as claimed in claim 1 is allowed to act on the seeds, the plants and/or their habitat.

10. A fungicide and/or nitrification inhibitor and/or agent for influencing plant growth, containing a salicylic acid derivative of the formula I as claimed in claim 1 and conventional inert additives.

## Claims (Claims for the following Contracting State(s): ES)

1. An agent containing, besides conventional inert additives, a salicyloyl(thio)ether derivative of the formula I 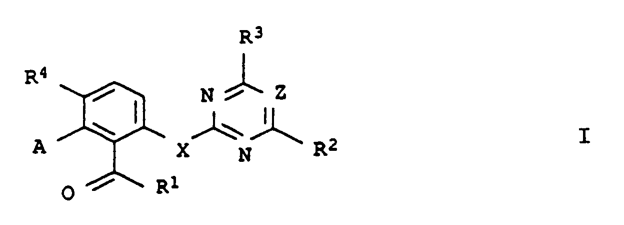 where
R¹ is a radical 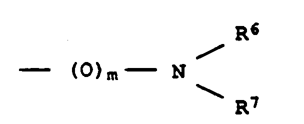 in which m is 0 or 1 and R⁶ and R⁷ are each hydrogen;
C₁-C₆-alkyl, C₃-C₆-alkenyl, C₃-C₆-alkynyl, where these radicals may each carry from one to five halogen atoms and/or one or two of the following groups: C₁-C₆-alkoxy, C₃-C₆-alkenyloxy, C₃-C₆-alkynyloxy, C₁-C₆-alkylthio, C₃-C₆-alkenylthio, C₃-C₆-alkynylthio, C₁-C₆-haloalkoxy, cyano, C₁-C₆-alkylcarbonyl, C₃-C₆-alkenylcarbonyl, C₃-C₆-alkynylcarbonyl, C₁-C₆-alkoxycarbonyl, C₃-C₆-alkenyloxycarbonyl, C₃-C₆-alkynyloxycarbonyl, C₁-C₆-dialkylamino or C₁-C₆-cycloalkyl;
unsubstituted or substituted C₁-C₆-cycloalkyl;
unsubstituted or substituted phenyl;
R⁶ together with R⁷ may be an unsubstituted or substituted C₄-C₇-alkylene chain in which a CH₂ group may be replaced with oxygen, sulfur or -NH;
a group 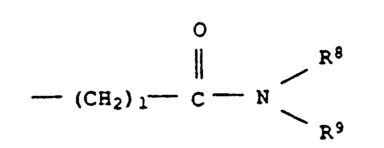 where R⁸ and R⁹ are each hydrogen, C₁-C₆-alkyl, C₃-C₆-alkenyl or C₃-C₆-alkynyl and l is 1, 2, 3 or 4; or a group
―(CH₂)ₗ―S(=O)ₖ-R¹⁰
where R¹⁰ is C₁-C₆-alkyl, C₃-C₆-alkenyl or C₃-C₆-alkynyl, l is 1, 2, 3 or 4 and k is 0, 1 or 2;
a radical OR⁵, where
R⁵ is an unsubstituted or substituted 5-membered aromatic heterocyclic structure bonded via a nitrogen atom and having from one to four nitrogen atoms in the ring or a radical 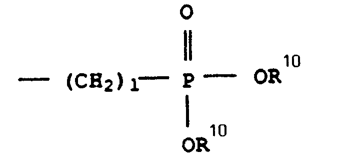 where R¹⁰ and l have the abovementioned meanings, or a radical
-HN-SO₂-R¹²
where R¹² is C₁-C₆-alkyl, C₁-C₆-haloalkyl or phenyl, which in turn may carry from one to four of the following substituents: halogen, nitro, cyano or C₁-C₆-alkyl;
R² and R³ are each C₁-C₄-alkyl, C₁-C₄-haloalkyl, C₁-C₄-alkoxy, C₁-C₄-haloalkoxy and/or C₁-C₄-alkylthio;
X is oxygen or sulfur;
Z is nitrogen or the methine group;
R⁴ is hydrogen, halogen, nitro, C₁-C₄-alkyl, cyano or C₁-C₄-haloalkyl; and
A is a 5-membered heteroaromatic structure having from one to four nitrogen atoms or from one to three nitrogen atoms and additionally a sulfur or oxygen atom in the ring, which may carry from one to three halogen atoms and/or from one to three of the following radicals: nitro, cyano, C₁-C₄-alkyl, C₁-C₄-alkylthio, C₁-C₄-haloalkyl or phenyl which is unsubstituted or substituted by from one to three halogen atoms and/or from one to three methyl groups;
a benzofused 5-membered heteroaromatic structure which may contain from one to three nitrogen atoms or a nitrogen atom and additionally one oxygen or sulfur atom and may carry one of the following radicals: C₁-C₄-alkyl, halogen or cyano;
a thienyl or furyl radical which may carry from one to three halogen atoms and/or from one to three of the following radicals: C₁-C₄-alkyl, C₁- or C₂-haloalkyl, unsubstituted or substituted phenyl or nitro;
a benzothienyl or benzofuryl radical which may carry one halogen atom and/or one of the following radicals: C₁-C₄-alkyl, C₁- or C₂-haloalkyl or nitro;
pyridyl which may carry from one to three halogen atoms and/or from one to three of the following radicals: C₁-C₄-alkyl, C₁- or C₂-haloalkyl, unsubstituted or substituted phenyl or nitro;
a naphthyl or quinolyl radical, each of which may carry up to three halogen atoms and/or up to three of the following radicals: C₁-C₄-alkyl or C₁- or C₂-haloalkyl, the expression unsubstituted or substituted in the abovementioned cases meaning that the corresponding groups may carry one or more of the following substituents: halogen, nitro, cyano, C₁-C₆-alkyl, C₁-C₆-haloalkyl, C₁-C₆-alkoxy or C₁-C₆-alkylthio.

2. An agent containing a salicylic acid derivative of the formula I as claimed in claim 1,
where
R¹ is a group 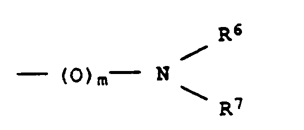 where m is 0 or 1 and R⁶ and R⁷ are each hydrogen or methyl or R⁶ and R⁷ together form a C₄- or C₅-alkylene chain;
R² and R³ are each methoxy, methyl, difluoromethoxy or chlorine;
R⁴ is hydrogen or methyl;
X is oxygen;
Y is nitrogen;
Z is the methine group and
A has the meanings stated in claim 1.

3. An agent containing a salicylic acid derivative of the formula I as claimed in claim 1, where R¹ is O-N(CH₃)₂, X is oxygen, Y is nitrogen, Z is the methine group and R² and R³ are each methoxy and A has the above-mentioned meanings.

4. A novel salicylic acid derivative of the formula II 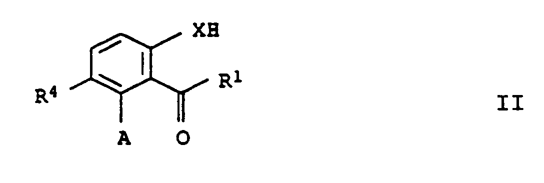 where
R¹ is a radical 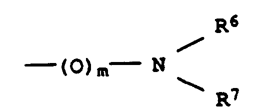 in which m is 0 or 1 and R⁶ and R⁷ are each hydrogen;
C₁-C₆-alkyl, C₃-C₆-alkenyl, C₃-C₆-alkynyl, where these radicals may each carry from one to five halogen atoms and/or one or two of the following groups: C₁-C₆-alkoxy, C₃-C₆-alkenyloxy, C₃-C₆-alkynyloxy, C₁-C₆-alkylthio, C₃-C₆-alkenylthio, C₃-C₆-alkynylthio, C₁-C₆-haloalkoxy, cyano, C₁-C₆-alkylcarbonyl, C₃-C₆-alkenylcarbonyl, C₃-C₆-alkynylcarbonyl, C₁-C₆-alkoxycarbonyl, C₃-C₆-alkenyloxycarbonyl, C₃-C₆-alkynyloxycarbonyl, C₁-C₆-dialkylamino or C₃-C₆-cycloalkyl;
unsubstituted or substituted C₁-C₆-cycloalkyl;
unsubstituted or substituted phenyl;
R⁶ together with R⁷ may be an unsubstituted or substituted C₄-C₇-alkylene chain in which a CH₂ group may be replaced with oxygen, sulfur or -NH;
a group 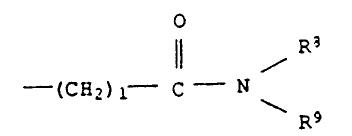 where R⁸ and R⁹ are each hydrogen, C₁-C₆-alkyl, C₃-C₆-alkenyl or C₃-C₆-alkynyl and l is 1, 2, 3 or 4; or a group
―(CH₂)ₗ―S(=O)ₖ-R¹⁰
where R¹⁰ is C₁-C₆-alkyl, C₃-C₆-alkenyl or C₃-C₆-alkynyl, l is 1, 2, 3 or 4 and k is 0, 1 or 2;
a radical OR⁵, where
R⁵ is an unsubstituted or substituted 5-membered aromatic heterocyclic structure bonded via a nitrogen atom and having from one to four nitrogen atoms in the ring or a radical 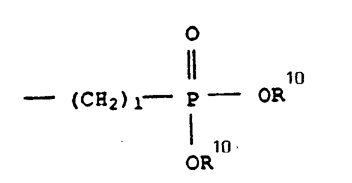 where R¹⁰ and l have the abovementioned meanings, or a radical
-HN-SO₂-R¹²
where R¹² is C₁-C₆-alkyl, C₁-C₆-haloalkyl or phenyl, which in turn may carry from one to four of the following substituents: halogen, nitro, cyano or C₁-C₆-alkyl;
R² and R³ are each C₁-C₄-alkyl, C₁-C₄-haloalkyl, C₁-C₄-alkoxy, C₁-C₄-haloalkoxy and/or C₁-C₄-alkylthio;
X is oxygen or sulfur;
Z is nitrogen or the methine group;
R⁴ is hydrogen, halogen, nitro, C₁-C₄-alkyl, cyano or C₁-C₄-haloalkyl; and
A is a 5-membered heteroaromatic structure having from one to four nitrogen atoms or from one to three nitrogen atoms and additionally a sulfur or oxygen atom in the ring, which may carry from-one to three halogen atoms and/or from one to three of the following radicals: nitro, cyano, C₁-C₄-alkyl, C₁-C₄-alkylthio, C₁-C₄-haloalkyl or phenyl which is unsubstituted or substituted by from one to three halogen atoms and/or from one to three methyl groups;
a benzofused 5-membered heteroaromatic structure which may contain from one to three nitrogen atoms or a nitrogen atom and additionally one oxygen or sulfur atom and may carry one of the following radicals: C₁-C₄-alkyl, halogen or cyano;
a thienyl or furyl radical which may carry from one to three halogen atoms and/or from one to three of the following radicals: C₁-C₄-alkyl, C₁- or C₂-haloalkyl, unsubstituted or substituted phenyl or nitro;
a benzothienyl or benzofuryl radical which may carry one halogen atom and/or one of the following radicals: C₁-C₄-alkyl, C₁- or C₂-haloalkyl or nitro;
pyridyl which may carry from one to three halogen atoms and/or from one to three of the following radicals: C₁-C₄-alkyl, C₁- or C₂-haloalkyl, unsubstituted or substituted phenyl or nitro;
a naphthyl or quinolyl radical, each of which may carry up to three halogen atoms and/or up to three of the following radicals: C₁-C₄-alkyl or C₁- or C₂-haloalkyl, the expression unsubstituted or substituted in the abovementioned cases meaning that the corresponding groups may carry one or more of the following substituents: halogen, nitro, cyano, C₁-C₆-alkyl, C₁-C₆-haloalkyl, C₁-C₆-alkoxy or C₁-C₆-alkylthio.

5. A process for the preparation of a compound of the formula I as claimed in claim 1, wherein the corresponding salicylic acid or thiosalicylic acid derivatives of the formula II 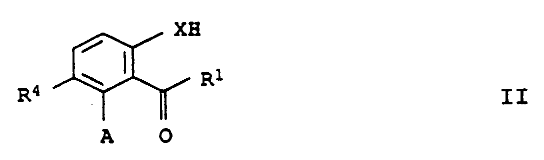 is reacted in a conventional manner with a heteroaromatic structure of the formula III 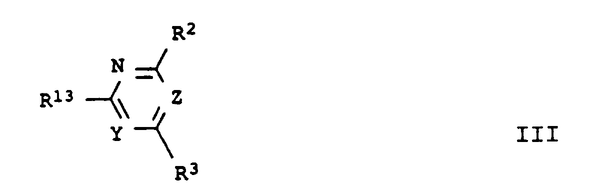 where R¹³ is a nucleofugic leaving group, in the presence of an inorganic or organic base.

6. A process for the preparation of a compound of the formula I as claimed in claim 1, wherein the free salicylic acid or a sulfur analog thereof of the formula I' 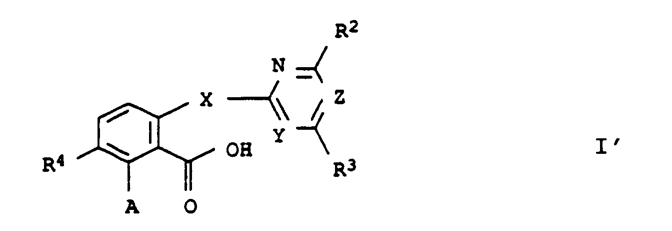 is first converted in a conventional manner into the halide or another activated form of the carboxylic acid and this is then reacted, in the presence or absence of an inorganic or organic base, with an unsubstituted or substituted hydroxylamine, N-hydroxyazole, hydroxyalkyl phosphonate or sulfonamide.

7. A method for controlling undesirable plant growth, wherein the undesirable plants and/or their habitat is or are treated with a herbicidal amount of a derivative I as claimed in claim 1.

8. A method for regulating plant growth, wherein an amount, having a regulatory effect, of a salicylic acid derivative of the formula I as claimed in claim 1 is allowed to act on the seeds, the plants and/or their habitat.

9. A fungicide and/or nitrification inhibitor and/or agent for influencing plant growth, containing a salicylic acid derivative of the formula I as claimed in claim 1 and conventional inert additives.

## Revendications (Revendications pour l'(les) Etat(s) contractant(s) suivant(s): AT, BE, CH, DE, FR, GB, IT, LI, NL, SE)

1. Dérivé salicyl(thio)éther de formule I 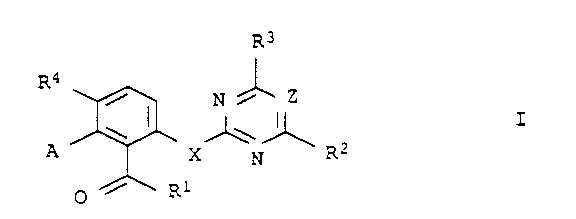 dans laquelle les substituants ont les significations suivantes :
R¹ un reste 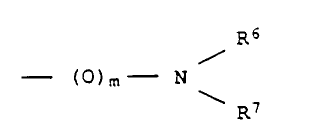 où m vaut 0 ou 1 et R⁶ et R⁷ ont les significations suivantes :
un atome d'hydrogène ;
un reste alkyle en C₁-C₆, alcényle en C₃-C₆, alcinyle en C₃-C₆, ces restes pouvant porter chacun de un à cinq atomes d'halogène et/ou de un à deux des groupes suivants : alcoxy en C₁-C₆, alcényloxy en C₃-C₆, alcinyloxy en C₃-C₆, alkylthio en C₁-C₆, alcénylthio en C₃-C₆, alcinylthio en C₃-C₆, halogénoalcoxy en C₁-C₆, cyano, alkylcarbonyle en C₁-C₆ alcénylcarbonyle en C₃-C₆, alcinylcarbonyle en C₃-C₆, alcoxycarbonyle en C₁-C₆, alcényloxycarbonyle en C₃-C₆, alcinyloxycarbonyle en C₃-C₆, dialkylamino en C₁-C₆, cycloalkyle en C₁-C₆ ;
un reste cycloalkyle en C₁-C₆ éventuellement substitué ;
un reste phényle éventuellement substitué ;
R⁶ et R⁷ forment ensemble une chaîne alkylène en C₄-C₇ éventuellement substituée, dans laquelle un groupe CH₂ peut être remplacé par un atome d'oxygène, de soufre ou un groupe -NH ;
un groupe 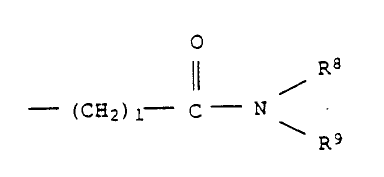 dans lequel R⁸ et R⁹ représentent un atome d'hydrogène, un groupe alkyle en C₁-C₆, alcényle en C₃-C₆ ou alcinyle en C₃-C₆, et l peut valoir 1, 2, 3 ou 4 ;
ou un groupe
―(CH₂)ₗ―S(=O)ₖ-R¹⁰
où R¹⁰ représente un groupe alkyle en C₁-C₆, alcényle en C₃-C₆ ou alcinyle en C₃-C₆, l peut valoir 1, 2, 3 ou 4 et k peut valoir 0, 1 ou 2 ;
un reste OR⁵ dans lequel
R⁵ représente un hétérocycle aromatique à cinq maillons éventuellement substitué, lié par l'intermédiaire d'un atome d'azote, dont le cycle comporte de un à quatre atomes d'azote,
ou un reste 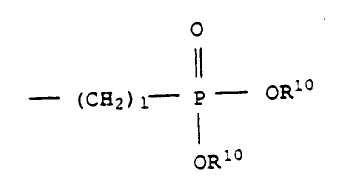 dans lequel R¹⁰ et l ont les significations mentionnées plus haut,
ou un reste
-HN-SO₂-R¹²
ou R¹² représente un reste alkyle en C₁-C₆, halogénoalkyle en C₁-C₆ ou phényle, qui peut porter a son tour de un à quatre des substituants suivants : halogéno, nitro, cyano, alkyle en C₁-C₆ ;
R², R³ un groupe alkyle en C₁-C₄, halogénoalkyle en C₁-C₄, alcoxy en C₁-C₄, halogénoalcoxy en C₁-C₄ et/ou alkylthio en C₁-C₄ ;
X un atome d'oxygène ou un atome de soufre ;
Z un atome d'azote ou le groupe méthine ;
R⁴ un atome d'hydrogène, d'halogène, un groupe nitro, alkyle en C₁-C₄, cyano ou halogénoalkyle en C₁-C₄ ;
A un composé hétéroaromatique à 5 maillons, dont le cycle comporte de un à quatre atomes d'azote ou de un à trois atomes d'azote ainsi qu'un atome de soufre ou d'oxygène en plus, qui peut porter un à trois atomes d'halogène et/ou de un à trois des restes suivants : nitro, cyano, alkyle en C₁-C₄, alkylthio en C₁-C₄, halogénoalkyle en C₁-C₄ ou phényle ne portant pas de substituant ou pouvant porter de un à trois atomes d'halogène et/ou de un à trois groupes méthyle ;
un composé hétéroaromatique à 5 maillons, condensé à un noyau benzénique, qui peut comporter de un à trois atomes d'azote ou un atome d'azote et un atome d'oxygène ou de soufre en plus et qui peut porter un des restes suivants : alkyle en C₁-C₄, halogèno, cyano ;
un reste thiényle ou furyle, qui peut porter de un à trois atomes d'halogène et/ou de un à trois des restes suivants : alkyle en C₁-C₄, halogénoalkyle en C₁-C₂, phényle éventuellement substitué ou nitro ;
un reste benzothiényle ou benzofuryle, qui peut porter un atome d'halogène et/ou un des restes suivants : alkyle en C₁-C₄, halogénoalkyle en C₁-C₂ ou nitro ;
un reste pyridyle, qui peut porter de un à trois atomes d'halogène et/ou de un à trois des restes suivants : alkyle en C₁-C₄, halogénoalkyle en C₁-C₂, phényle éventuellement substitué ou nitro ;
un reste naphtyle ou quinoléinyle, qui peut porter chaque fois de un à trois atomes d'halogène et/ou de un à trois des restes suivants : alkyle en C₁-C₄ ou halogénoalkyle en C₁-C₂,
l'expression éventuellement substitué signifiant, dans les cas mentionnés ci-dessus, que les groupes correspondants peuvent porter un ou plusieurs des substituants suivants : halogéno, nitro, cyano, alkyle en C₁-C₆, halogénoalkyle en C₁-C₆, alcoxy en C₁-C₆, alkylthio en C₁-C₆.

2. Dérivé de l'acide salicylique de formule I selon la revendication 1, dans laquelle
R¹ représente un groupe 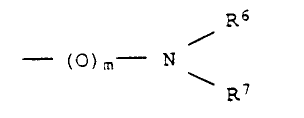 où m vaut 0 ou 1 et R⁶, R⁷ représentent un atome d'hydrogène ou un groupe méthyle, ou R⁶ et R⁷ forment ensemble une chaîne alkylène en C₄-C₅,
R², R³ représentent un groupe méthoxy, méthyle, difluorométhoxy ou chloro ;
R⁴ représente un atome d'hydrogène ou un groupe méthyle ;
X représente un atome d'oxygène ;
Y représente un atome d'azote ;
Z représente le groupe méthine et
A a la signification mentionnée dans la revendication 1.

3. Dérivé de l'acide salicylique de formule I selon la revendication 1, dans laquelle R¹ représente O-N(CH₃)₂, X représente un atome d'oxygène, Y représente un atome d'azote, Z le groupe méthine et R² et R³ représentent un groupe méthoxy et A a la signification mentionnée ci-dessus.

4. Nouveau dérivé de l'acide salicylique, de formule II 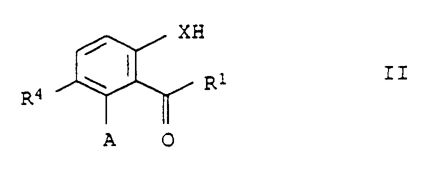 dans laquelle les restes ont les significations suivantes :
R¹ un reste 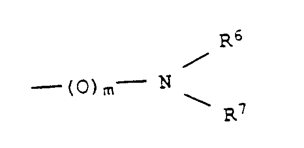 où m vaut 0 ou 1 et R⁶ et R⁷ ont les significations suivantes :
un atome d'hydrogène ;
un reste alkyle en C₁-C₆, alcényle en C₃-C₆, alcinyle en C₃-C₆, ces restes pouvant porter chacun de un à cinq atomes d'halogène et/ou un à deux des groupes suivants : alcoxy en C₁-C₆, alcényloxy en C₃-C₆, alcinyloxy en C₃-C₆, alkylthio en C₁-C₆, alcénylthio en C₃-C₆, alcinylthio en C₃-C₆, halogénoalcoxy en C₁-C₆, cyano, alkylcarbonyle en C₁-C₆, alcénylcarbonyle en C₃-C₆, alcinylcarbonyle en C₃-C₆, alcoxycarbonyle en C₁-C₆, alcényloxycarbonyle en C₃-C₆, alcinyloxycarbonyle en C₃-C₆, dialkylamino en C₁-C₆, cycloalkyle en C₃-C₆ ;
un reste cycloalkyle en C₁-C₆ éventuellement substitué ;
un reste phényle éventuellement substitué ;
R⁶ et R⁷ forment ensemble une chaîne alkylène en C₄-C₇ éventuellement substituée, dans laquelle un groupe CH₂ peut être remplacé par un atome d'oxygène, de soufre ou un groupe -NH ;
un groupe 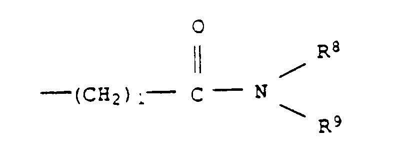 dans lequel R⁸ et R⁹ représentent un atome d'hydrogène, un groupe alkyle en C₁-C₆, alcényle en C₃-C₆ ou alcinyle en C₃-C₆, et l peut valoir 1, 2, 3 ou 4 ;
ou un groupe
―(CH₂)ₗ―S(=O)ₖ-R¹⁰
où R¹⁰ représente un groupe alkyle en C₁-C₆, alcényle en C₃-C₆ ou alcinyle en C₃-C₆, l peut valoir 1, 2, 3 ou 4 et k peut valoir 0, 1 ou 2 ;
un reste OR⁵ dans lequel
R⁵ représente un hétérocycle aromatique à cinq maillons éventuellement substitué, lié par l'intermédiaire d'un atome d'azote, dont le cycle comporte de un à quatre atomes d'azote,
ou un reste 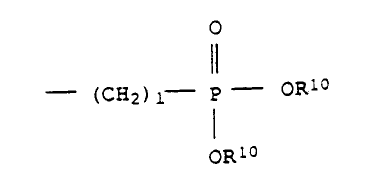 dans lequel R¹¹ et l ont les significations mentionnées plus haut,
ou un reste
-HN-SO₂-R¹²
ou R¹² représente un reste alkyle en C₁-C₆, halogénoalkyle en C₁-C₆ ou phényle, qui peuvent porter à leur tour de un à quatre des substituants suivants : halogéno, nitro, cyano, alkyle en C₁-C₆, ;
R², R³ un groupe alkyle en C₁-C₄, halogénoalkyle en C₁-C₄, alcoxy en C₁-C₄, halogénoalcoxy en C₁-C₄ et/ou alkylthio en C₁-C₄ ;
X un atome d'oxygène ou un atome de soufre ;
Z un atome d'azote ou le groupe méthine ;
R⁴ un atome d'hydrogène, d'halogène, un groupe nitro, alkyle en C₁-C₄, cyano ou halogénoalkyle en C₁-C₄ ;
A un composé hétéroaromatique à 5 maillons, dont le cycle comporte de un à quatre atomes d'azote ou de un à trois atomes d'azote ainsi qu'un atome de soufre ou d'oxygène en plus, qui peut porter un à trois atomes d'halogène et/ou de un à trois des restes suivants : nitro, cyano, alkyle en C₁-C₄, alkylthio en C₁-C₄, halogénoalkyle en C₁-C₄ ou phényle ne portant pas de substituant ou pouvant porter de un à trois atomes d'halogène et/ou un à trois groupes méthyle ;
un composé hétéroaromatique à 5 maillons condensé à un noyau benzénique, qui peut comporter de un à trois atomes d'azote ou un atome d'azote et un atome d'oxygène ou de soufre en plus et qui peut porter un des restes suivants : alkyle en C₁-C₄, halogèno, cyano ;
un reste thiényle ou furyle, qui peut porter de un à trois atomes d'halogène et/ou de un à trois des restes suivants : alkyle en C₁-C₄, halogénoalkyle en C₁-C₂, phényle éventuellement substitué ou nitro ;
un reste benzothiényle ou benzofuryle, qui peut porter un atome d'halogène et/ou un des restes suivants : alkyle en C₁-C₄, halogénoalkyle en C₁-C₂ ou nitro ;
un reste pyridyle, qui peut porter de un à trois atomes d'halogène et/ou de un à trois des restes suivants : alkyle en C₁-C₄, halogénoalkyle en C₁-C₂, phényle éventuellement substitué ou nitro ;
un reste naphtyle ou quinoléinyle, qui peut porter chaque fois de un à trois atomes d'halogène et/ou de un à trois des restes suivants : alkyle en C₁-C₄ ou halogénoalkyle en C₁-C₂,
l'expression éventuellement substitué signifiant, dans les cas mentionnés ci-dessus, que les groupes correspondants peuvent porter un ou plusieurs des substituants suivants : halogéno, nitro, cyano, alkyle en C₁-C₆, halogénoalkyle en C₁-C₆, alcoxy en C₁-C₆, alkylthio en C₁-C₆.

5. Procédé de préparation des composés de formule l selon la revendication 1, caractérisé par le fait que l'on fait réagir un dérivé approprié de l'acide salicylique ou de l'acide thiosalicylique, de formule II 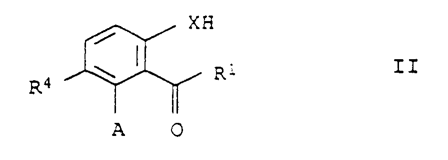 de manière connue en soi, avec un composé hétéroaromatique de formule III 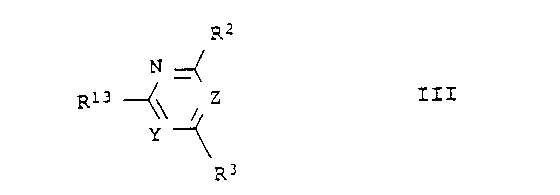 dans laquelle R¹³ représente un groupe partant nucléofuge, en présence d'une base inorganique ou organique.

6. Procédé de préparation des composés de formule I selon la revendication 1 caractérisé par le fait que l'on transforme d'abord l'acide salicylique libre ou son analogue soufré de formule I' 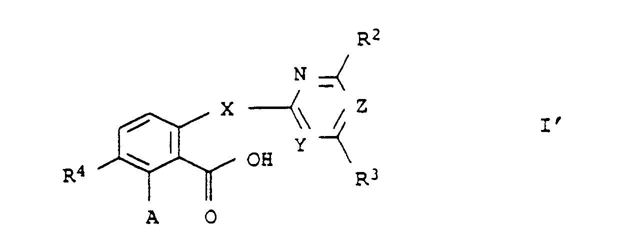 de manière connue en soi, en l'halogénure ou en une autre forme activée de l'acide carboxylique et, ensuite, on fait réagir celle-ci, éventuellement en présence d'une base inorganique ou organique, avec une hydroxylamine, un N-hydroxyazole, un phosphonate d'hydroxyalkyle ou un sulfonamide éventuellement substitué.

7. Agent herbicide, contenant un composé de formule I selon la revendication 1, et des adjuvants inertes usuels.

8. Procédé pour lutter contre des plantes indésirables, caractérisé par le fait que l'on traite les plantes indésirables et/ou leur espace vital avec une quantité ayant une action herbicide d'un dérivé l selon la revendication 1.

9. Procédé de régulation de la croissance des plantes, caractérisé par le fait que l'on fait agir une quantité, produisant une action régulatrice, d'un dérivé de l'acide salicylique, de formule générale l selon la revendication 1, sur les semences, les plantes et/ou leur espace vital.

10. Agent fongicide et/ou inhibant la nitrification, et/ou agent destiné à agir sur la croissance des plantes, contenant un dérivé de l'acide salicylique, de formule I selon la revendication 1, et des adjuvants inertes usuels.

## Revendications (Revendications pour l'(les) Etat(s) contractant(s) suivant(s): ES)

1. Agent contenant, en plus d'adjuvants inertes usuels, un dérivé salicyl(thio)éther de formule I 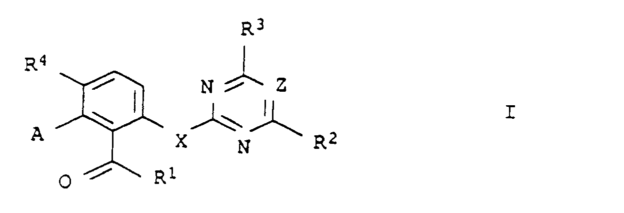 dans laquelle les substituants ont les significations suivantes :
R¹ un reste 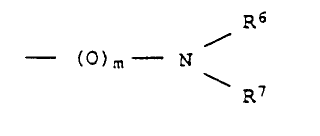 où m vaut 0 ou 1 et R⁶ et R⁷ ont les significations suivantes :
un atome d'hydrogène ;
un reste alkyle en C₁-C₆, alcényle en C₃-C₆, alcinyle en C₃-C₆, ces restes pouvant porter chacun de un à cinq atomes d'halogène et/ou de un à deux des groupes suivants : alcoxy en C₁-C₆, alcényloxy en C₃-C₆, alcinyloxy en C₃-C₆, alkylthio en C₁-C₆, alcénylthio en C₃-C₆, alcinylthio en C₃-C₆, halogénoalcoxy en C₁-C₆, cyano, alkylcarbonyle en C₁-C₆, alcénylcarbonyle en C₃-C₆, alcinylcarbonyle en C₃-C₆, alcoxycarbonyle en C₁-C₆, alcényloxycarbonyle en C₃-C₆, alcinyloxycarbonyle en C₃-C₆, dialkylamino en C₁-C₆, cycloalkyle en C₁-C₆ ;
un reste cycloalkyle en C₁-C₆ éventuellement substitué ;
un reste phényle éventuellement substitué ;
R⁶ et R⁷ forment ensemble une chaîne alkylène en C₄-C₇ éventuellement substituée, dans laquelle un groupe CH₂ peut être remplacé par un atome d'oxygène, de soufre ou un groupe -NH ;
un groupe 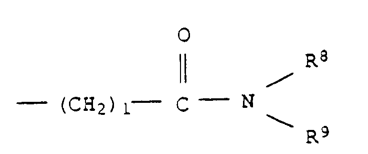 dans lequel R⁸ et R⁹ représentent un atome d'hydrogène, un groupe alkyle en C₁-C₆, alcényle en C₃-C₆ ou alcinyle en C₃-C₆, et l peut valoir 1, 2, 3 ou 4;
ou un groupe
―(CH₂)ₗ―S(=O)ₖ-R¹⁰
où R¹⁰ représente un groupe alkyle en C₁-C₆, alcényle en C₃-C₆ ou alcinyle en C₃-C₆, l peut valoir 1, 2, 3 ou 4 et k peut valoir 0, 1 ou 2 ;
un reste OR⁵ dans lequel
R⁵ représente un hétérocycle aromatique à cinq maillons éventuellement substitué, lié par l'intermédiaire d'un atome d'azote, dont le cycle comporte de un à quatre atomes d'azote,
ou un reste 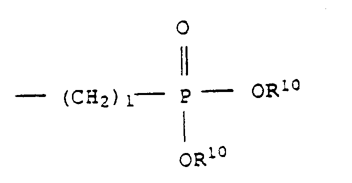 dans lequel R¹⁰ et l ont les significations mentionnées plus haut,
ou un reste
-HN-SO₂-R¹²
ou R¹² représente un reste alkyle en C₁-C₆, halogénoalkyle en C₁-C₆ ou phényle, qui peut porter à son tour de un à quatre des substituants suivants : halogéno, nitro, cyano, alkyle en C₁-C₆ ;
R², R³ un groupe alkyle en C₁-C₄, halogénoalkyle en C₁-C₄, alcoxy en C₁-C₄, halogénoalcoxy en C₁-C₄ et/ou alkylthio en C₁-C₄ ;
X un atome d'oxygène ou un atome de soufre ;
Z un atome d'azote ou le groupe méthine ;
R⁴ un atome d'hydrogène, d'halogène, un groupe nitro, alkyle en C₁-C₄, cyano ou halogénoalkyle en C₁-C₄ ;
A un composé hétéroaromatique à 5 maillons, dont le cycle comporte de un à quatre atomes d'azote ou de un à trois atomes d'azote ainsi qu'un atome de soufre ou d'oxygène en plus, qui peut porter de un à trois atomes d'halogène et/ou de un à trois des restes suivants : nitro, cyano, alkyle en C₁-C₄, alkylthio en C₁-C₄, halogénoalkyle en C₁-C₄ ou phényle ne portant pas de substituant ou pouvant porter de un à trois atomes d'halogène et/ou de un à trois groupes méthyle ;
un composé hétéroaromatique à 5 maillons, condensé à un noyau benzénique, qui peut comporter de un à trois atomes d'azote ou un atome d'azote et un atome d'oxygène ou de soufre en plus et qui peut porter un des restes suivants : alkyle en C₁-C₄, halogèno, cyano ;
un reste thiényle ou furyle, qui peut porter de un à trois atomes d'halogène et/ou de un à trois des restes suivants : alkyle en C₁-C₄, halogénoalkyle en C₁-C₂, phényle éventuellement substitué ou nitro ;
un reste benzothiényle ou benzofuryle, qui peut porter un atome d'halogène et/ou un des restes suivants : alkyle en C₁-C₄, halogénoalkyle en C₁-C₂ ou nitro ;
un reste pyridyle, qui peut porter de un à trois atomes d'halogène et/ou de un à trois des restes suivants : alkyle en C₁-C₄, halogénoalkyle en C₁-C₂, phényle éventuellement substitué ou nitro ;
un reste naphtyle ou quinoléinyle, qui peut porter chaque fois de un à trois atomes d'halogène et/ou de un à trois des restes suivants : alkyle en C₁-C₄ ou halogénoalkyle en C₁-C₂,
l'expression éventuellement substitué signifiant, dans les cas mentionnés ci-dessus, que les groupes correspondants peuvent porter un ou plusieurs des substituants suivants : halogéno, nitro, cyano, alkyle en C₁-C₆, halogénoalkyle en C₁-C₆, alcoxy en C₁-C₆, alkylthio en C₁-C₆.

2. Agent contenant un dérivé de l'acide salicylique, de formule I selon la revendication 1, dans laquelle
R¹ représente un groupe 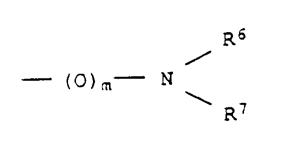 où m vaut 0 ou 1 et R⁶, R⁷ représentent un atome d'hydrogène ou un groupe méthyle, ou R⁶ et R⁷ forment ensemble une chaîne alkylène en C₄-C₅,
R², R³ représentent un groupe méthoxy, méthyle, difluorométhoxy ou chloro ;
R⁴ représente un atome d'hydrogène ou un groupe méthyle ;
X représente un atome d'oxygène ;
Y représente un atome d'azote ;
Z représente le groupe méthine et
A a la signification mentionnée dans la revendication 1.

3. Agent contenant un dérivé de l'acide salicylique, de formule I selon la revendication 1, dans laquelle R¹ représente O-N(CH₃)₂, X représente un atome d'oxygène, Y représente un atome d'azote, Z le groupe méthine et R² et R³ représentent un groupe méthoxy et A a la signification mentionnée ci-dessus.

4. Nouveau dérivé de l'acide salicylique, de formule II 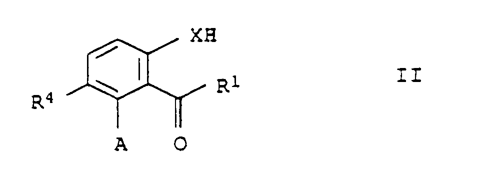 dans laquelle les restes ont les significations suivantes :
R¹ un reste 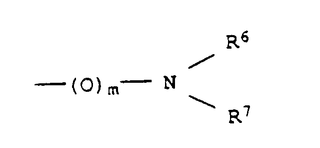 où m vaut 0 ou 1 et R⁶ et R⁷ ont les significations suivantes :
un atome d'hydrogène ;
un reste alkyle en C₁-C₆, alcényle en C₃-C₆, alcinyle en C₃-C₆, ces restes pouvant porter chacun de un à cinq atomes d'halogène et/ou un à deux des groupes suivants : alcoxy en C₁-C₆, alcényloxy en C₃-C₆, alcinyloxy en C₃-C₆, alkylthio en C₁-C₆, alcénylthio en C₃-C₆, alcinylthio en C₃-C₆, halogénoalcoxy en C₁-C₆, cyano, alkylcarbonyle en C₁-C₆, alcénylcarbonyle en C₃-C₆, alcinylcarbonyle en C₃-C₆, alcoxycarbonyle en C₁-C₆, alcényloxycarbonyle en C₃-C₆, alcinyloxycarbonyle en C₃-C₆, dialkylamino en C₁-C₆, cycloalkyle en C₃-C₆ ;
un reste cycloalkyle en C₁-C₆ éventuellement substitué ;
un reste phényle éventuellement substitué ;
R⁶ et R⁷ forment ensemble une chaîne alkylène en C₄-C₇ éventuellement substituée, dans laquelle un groupe CH₂ peut être remplacé par un atome d'oxygène, de soufre ou un groupe -NH ;
un groupe 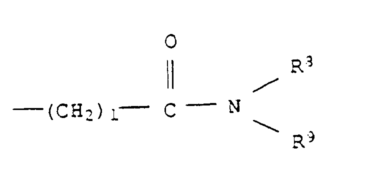 dans lequel R⁸ et R⁹ représentent un atome d'hydrogène, un groupe alkyle en C₁-C₆, alcényle en C₃-C₆ ou alcinyle en C₃-C₆, et l peut valoir 1, 2, 3 ou 4 ;
ou un groupe
―(CH₂)ₗ―S(=O)ₖ-R¹⁰
où R¹⁰ représente un groupe alkyle en C₁-C₆, alcényle en C₃-C₆ ou alcinyle en C₃-C₆, l peut valoir 1, 2, 3 ou 4 et k peut valoir 0, 1 ou 2 ;
un reste OR⁵ dans lequel
R⁵ représente un hétérocycle aromatique à cinq maillons éventuellement substitué, lié par l'intermédiaire d'un atome d'azote, dont le cycle comporte de un à quatre atomes d'azote,
ou un reste 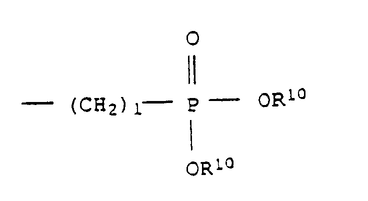 dans lequel R¹⁰ et l ont les significations mentionnées plus haut,
ou un reste
-HN-SO₂-R¹²
ou R¹² représente un reste alkyle en C₁-C₆, halogénoalkyle en C₁-C₆ ou phényle, qui peuvent porter à leur tour de un à quatre des substituants suivants : halogéno, nitro, cyano, alkyle en C₁-C₆, ;
R², R³ un groupe alkyle en C₁-C₄, halogénoalkyle en C₁-C₄, alcoxy en C₁-C₄, halogénoalcoxy en C₁-C₄ et/ou alkylthio en C₁-C₄ ;
X un atome d'oxygène ou un atome de soufre ;
Z un atome d'azote ou le groupe méthine ;
R⁴ un atome d'hydrogène, d'halogène, un groupe nitro, alkyle en C₁-C₄, cyano ou halogénoalkyle en C₁-C₄ ;
A un composé hétéroaromatique à 5 maillons, dont le cycle comporte de un à quatre atomes d'azote ou de un à trois atomes d'azote ainsi qu'un atome de soufre ou d'oxygène en plus, qui peut porter de un à trois atomes d'halogène et/ou de un à trois des restes suivants : nitro, cyano, alkyle en C₁-C₄, alkylthio en C₁-C₄, halogénoalkyle en C₁-C₄ ou phényle ne portant pas de substituant ou pouvant porter de un à trois atomes d'halogène et/ou un à trois groupes méthyle ;
un composé hétéroaromatique à 5 maillons condensé à un noyau benzénique, qui peut comporter de un à trois atomes d'azote ou un atome d'azote et un atome d'oxygène ou de soufre en plus et qui peut porter un des restes suivants : alkyle en C₁-C₄, halogèno, cyano ;
un reste thiényle ou furyle, qui peut porter de un à trois atomes d'halogène et/ou de un à trois des restes suivants : alkyle en C₁-C₄, halogénoalkyle en C₁-C₂, phényle éventuellement substitué ou nitro ;
un reste benzothiényle ou benzofuryle, qui peut porter un atome d'halogène et/ou un des restes suivants : alkyle en C₁-C₄, halogénoalkyle en C₁-C₂ ou nitro ;
un reste pyridyle, qui peut porter de un à trois atomes d'halogène et/ou de un à trois des restes suivants : alkyle en C₁-C₄, halogénoalkyle en C₁-C₂, phényle éventuellement substitué ou nitro ;
un reste naphtyle ou quinoléinyle, qui peut porter chaque fois de un à trois atomes d'halogène et/ou de un à trois des restes suivants : alkyle en C₁-C₄ ou halogénoalkyle en C₁-C₂,
l'expression éventuellement substitué signifiant, dans les cas mentionnés ci-dessus, que les groupes correspondants peuvent porter un ou plusieurs des substituants suivants : halogéno, nitro, cyano, alkyle en C₁-C₆, halogénoalkyle en C₁-C₆, alcoxy en C₁-C₆, alkylthio en C₁-C₆.

5. Procédé de préparation des composés de formule I selon la revendication 1, caractérisé par le fait que l'on fait réagir un dérivé approprié de l'acide salicylique ou de l'acide thiosalicylique, de formule II 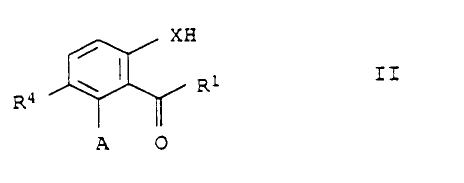 de manière connue en soi, avec un composé hétéroaromatique de formule III 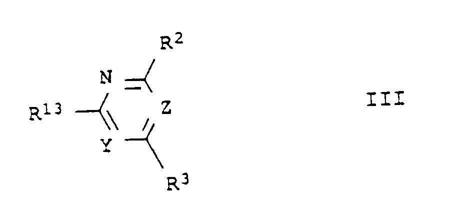 dans laquelle R¹³ représente un groupe partant nucléofuge, en présence d'une base inorganique ou organique.

6. Procédé de préparation des composés de formule I selon la revendication 1, caractérisé par le fait que l'on transforme d'abord l'acide salicylique libre ou son analogue soufré de formule I' 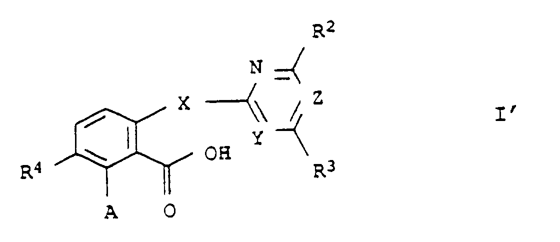 de manière connue en soi, en l'halogénure ou en une autre forme activée de l'acide carboxylique et, ensuite, on fait réagir celle-ci, éventuellement en présence d'une base inorganique ou organique, avec une hydroxylamine, un N-hydroxyazole, un phosphonate d'hydroxyalkyle ou un sulfonamide éventuellement substitué.

7. Procédé pour lutter contre des plantes indésirables, caractérisé par le fait que l'on traite les plantes indésirables et/ou leur espace vital avec une quantité produisant une action herbicide d'un dérivé l selon la revendication 1.

8. Procédé de régulation de la croissance des plantes, caractérisé par le fait que l'on fait agir une quantité, produisant une action régulatrice, d'un dérivé de l'acide salicylique, de formule générale l selon la revendication 1, sur les semences, les plantes et/ou leur espace vital.

9. Agent fongicide et/ou inhibant la nitrification, et/ou agent destiné à agir sur la croissance des plantes, contenant un dérivé de l'acide salicylique, de formule I selon la revendication 1, et des adjuvants inertes usuels.
